# EUROPEAN PATENT APPLICATION

(11) **EP 4 509 139 A1**
(43) Date of publication of application: **19.02.2025**
(21) Application number: 23787842.6
(22) Date of filing: 14.04.2023
(51) Int. Cl.: A61K 47/54, C12N 15/113, A61K 31/7088, A61K 31/713, C07C 237/06, C07C 229/24, A61P 19/06, A61P 35/00, A61P 35/04, A61B 8/08

(54) **CONJUGATE AND COMPOSITION AS WELL AS PREPARATION METHOD THEREFOR AND USE THEREOF**

(30) Priority: 14.04.2022 CN 202210386579; 14.04.2022 CN 202210377179; 14.04.2022 CN 202210401035
(71) Applicant: Suzhou Ribo Life Science Co., Ltd., Suzhou, Jiangsu 215300 (CN)
(72) Inventor: LIANG, Zicai, Suzhou, Jiangsu 215300 (CN); ZHANG, Hongyan, Suzhou, Jiangsu 215300 (CN); GAO, Shan, Suzhou, Jiangsu 215300 (CN)
(74) Representative: SSM Sandmair
(86) International application number: PCT/CN2023/088459
(87) International publication number: WO 2023/198200

(57) **Abstract**

The present disclosure provides a conjugate. The conjugate comprises one or more delivery groups and one or more functional groups. Each of the delivery groups is independently connected to the functional group by means of a covalent bond or by means of a linking group. Each of the fnnctional groups is selected from a small molecule therapeutic group having a therapeutic effect on tumors. The conjugate provided by the present disclosure can be efficiently targeted for delivery to tumor tissues, thereby effectively treating tumors and tumor-related diseases.

## Description

### TECHNICAL FIELD

The present disclosure relates to a conjugate and a pharmaceutical composition comprising an aptamer-based delivery group and a functional group. The present disclosure also relates to methods for preparing the conjugates and pharmaceutical compositions and uses thereof.

### BACKGROUND ART

A tumor refers to a neoplasm formed by the cell proliferation of local tissues under the action of various tumorigenic factors in the body. Among them, tumor cells that metastasize and invade surrounding tissues are called malignant tumors. According to the classification of the source tissue cells of the generated tumors, the tumors could be generally divided into malignant tumors derived from epithelial cells (cancer), malignant tumors derived from mesenchymal tissue cells (sarcoma), malignant tumors derived from blood stem cells (leukemia, etc.), and malignant tumors derived from glial cells (gliomas) and so on. Among them, glioma is the most common primary intracranial malignant tumor, accounting for approximately 40% to 50% of brain tumors, with an annual incidence of 3 to 8 cases per 100,000 persons worldwide. According to the WHO pathological classification standards, gliomas belong to neuroepithelial tumors, which include a variety of pathological types, including but not limited to pilocytic astrocytoma, diffuse astrocytoma, anaplastic astrocytoma, glioblastoma, oligodendroglioma, anaplastic oligodendroglioma, etc.

Currently, one of the important issues in the art of tumor treatment, especially glioma treatment, is how to specifically deliver therapeutic agents to tumor tissues and cells and enable these therapeutic agents to produce corresponding therapeutic effects at the appropriate time and in an appropriate manner.

Aptamers, also known as nucleic acid aptamers, are oligonucleotide molecules that can bind to a variety of target molecules, such as small molecule compounds, proteins, nucleic acids, and even cells, tissues and organs and so on. The aptamers can provide the important property of "recognizing specific molecules", so they are commonly used in biotechnology and therapy similar to antibodies. The aptamers can be designed in test tubes and quickly synthesized using chemical methods; meanwhile, they have the excellent properties of being easy to store and having low or no immunogenicity. Therefore, they have gradually gained attention from researchers in the art in recent years. However, the aptamers suitable for tumor-targeted delivery still need to be further developed and applied in the art.

### SUMMARY OF THE INVENTION

The inventors of the present disclosure unexpectedly discovered a conjugate that could specifically target tumor cells, especially glioma cells. The conjugate shows high specificity to tumor cells, especially glioma cells, so that it could be effectively enriched in tumor cells, especially glioma cells, and be effective in targeted treatment for tumors. Thus, the inventors made the following inventions:
In one aspect, the present disclosure provides a conjugate comprising one or more delivery groups and one or more functional groups; the delivery group is formed by removing one or more hydrogen atoms or one or more functional radicals from an aptamer, the aptamer comprises a segment of continuous nucleotide sequence, the group connecting two adjacent nucleotides is independently a phosphate group or a phosphate group with a modified group, each nucleotide is selected from one of modified or unmodified A, U, C or G, and the continuous nucleotide sequence has a sequence as shown by Formula (1):

5'- T₁-S₁-Nₐ-S₂-N_{b}-S₃-N_{c}-S₄-T₂ -3' Formula (1)

wherein T₁ is a motif consisting of 1-3 nucleotides, T₂ is a motif consisting of 0-15 nucleotides, and T₂ does not contain a motif that is completely reverse complementary to T₁;
S₁ and S₄ are a motif consisting of 3-7 nucleotides, respectively, and S1 and S4 have the same length and are completely reverse complementary;
Nₐ and N_{c} are a motif consisting of 1-4 nucleotides, respectively, and each nucleotide in Nₐ is not complementary to each nucleotide in N_{c}, and the total number of U in Nₐ and N_{c} accounts for more than 50% of the total number of all nucleotides in Nₐ and N_{c};
S₂ and S₃ are a motif consisting of 1-4 nucleotides, respectively, and S₂ and S₃ have the same length and are completely reverse complementary;
N_{b} is a motif consisting of 3-6 nucleotides, and the nucleotides at both ends of N_{b} do not form AU or GC complementation;
each of the delivery groups is independently connected to the functional group via a covalent bond or via a linking group; and each of the functional groups is selected from a small molecule therapeutic agent group having a therapeutic effect on tumors.

In another aspect, the present disclosure also provides a pharmaceutical composition comprising the conjugate according to the present disclosure and a pharmaceutically acceptable carrier.

In yet another aspect, the present disclosure also provides use of the conjugate and/or the pharmaceutical composition according to the present disclosure in the manufacture of a medicament for treating tumors and tumor-related diseases or symptoms.

In yet another aspect, the present disclosure also provides a method for treating tumors and tumor-related diseases or symptoms, comprising administering an effective amount of the conjugate and/or the pharmaceutical composition according to the present disclosure to a subject in need thereof.

In yet another aspect, the present disclosure further provides a kit comprising the conjugate and/or the pharmaceutical composition according to the present disclosure.

### INCORPORATION BY REFERENCE

All publications, patents, and patent applications mentioned in this specification are incorporated by reference into the present disclosure to the same extent as if each individual publication, patent, or patent application is specifically and individually incorporated by reference into the present disclosure.

### BENEFICIAL EFFECTS

The conjugates and pharmaceutical compositions provided by the present disclosure have excellent ability to target tumors, especially glioma tissues and cells, and can significantly treat or alleviate tumors and tumor-related diseases and/or symptoms.

On the one hand, the delivery groups in the conjugates provided by the present disclosure can specifically deliver various small molecule drug groups (such as small molecule toxin groups) to tumor tissue, and exhibit excellent tumor inhibition effects. For example, the conjugates of the present disclosure can effectively deliver MMAE to different tumor tissues, demonstrating tumor targeting ability while reducing the toxicity risk of caused by the distribution of MMAE molecules in other tissues. Moreover, various administration methods can effectively inhibit the increase rate of tumor volume and tumor weight, indicating that the conjugates of the present disclosure can effectively inhibit tumor proliferation. In addition, further increasing the administration dosage of the conjugates can result in almost no increase in tumor volume during the testing period, demonstrating more excellent anti-tumor effects.

Furthermore, the inventors of the present disclosure unexpectedly discovered that the conjugates and/or pharmaceutical compositions of the present disclosure can efficiently cross the blood brain barrier and target gliomas in the brain in the case of systemic administration, and significantly inhibit the increase of tumor volume, or even decreasing to less than 1/10 of the initial volume, or even decreasing to less than 1/100 as compared with the control group, indicating that the conjugates of the present disclosure can effectively penetrate the blood-brain barrier, and efficiently target and enter into gliomas, and have good inhibitory effects on tumor growth, demonstrating good treatment compliance and high druggability of efficiently inhibiting tumors.

The above results indicate that the conjugates of the present disclosure can significantly and effectively inhibit tumor proliferation, and have good application prospects.

### BRIEF DESCRIPTION OF THE DRAWINGS

Figures 1A-1C show the fluorescence imaging results in mice at 1h, 24h, and 48h after administration of different conjugates, respectively. Figure 1D shows the images of the fluorescence signal imaging of tumor tissues and kidneys in each group of mice after the mice were euthanized on D5.
Figures 2A-2C show the images of the fluorescence imaging results in mice at 1h, 24h, and 48h after administration of different conjugates, respectively. Figure 2D shows the images of the fluorescence signal imaging of tumor tissues and kidneys in each group of mice after the mice were euthanized on D6.
Figure 3 is a line graph showing the changes in tumor volume in each group of mice after administration of the conjugates or control compounds provided by the present disclosure.
Figure 4 shows the fluorescence imaging images of brain tissue in U118MG orthotopic tumor model mice established after administration of different conjugates at 24h and 48h after administration.
Figure 5 is a line graph showing the changes of tumor light intensity values in U118MG orthotopic tumor model mice over time after administration of the conjugates or control compounds provided by the present disclosure.
Figure 6 is a line graph showing the changes in tumor volume in U118MG subcutaneous tumor model mice over time after administration of the conjugates or control compounds provided by the present disclosure.
Figure 7 is a line graph showing the changes in tumor volume in U118MG subcutaneous tumor model mice over time after administration of different concentrations of the conjugates or control compounds provided by the present disclosure.
Figure 8 is a line graph showing the changes in tumor volume in U118MG subcutaneous tumor model mice over time after administration of different concentrations of the conjugates or control compounds provided by the present disclosure.
Figure 9 is a line graph showing the changes in tumor volume in A549 subcutaneous tumor model mice over time after administration of different concentrations of the conjugates or control compounds provided by the present disclosure.

### DETAILED DESCRIPTION OF THE INVENTION

The following is the detailed description of the specific embodiments of the present disclosure. It should be understood that the specific embodiments described herein are only used to illustrate and explain the present disclosure and are not intended to limit the present disclosure.

### Definitions

In the present disclosure, unless otherwise specified, A, U, C, G and T refer to an adenine nucleotide, a uracil nucleotide, a cytosine nucleotide, a guanine nucleotide and a thymine nucleotide, respectively, and 2-methylcytosine nucleotide refers to a nucleotide in which the 2' hydrogen on the cytosine base of the cytosine nucleotide is substituted with a methyl group. The structures of these nucleotides are well-known to those skilled in the art. As used herein, a "nucleic acid motif" or a "motif" refers to the fragment of a nucleic acid sequence in an oligonucleotide, consisting of one or more nucleotides. In some embodiments, a motif is the fragment of a nucleic acid sequence having a biological function.

As used herein, "alkyl" refers to straight chain and branched chain saturated alkyl having the specified number of carbon atoms, typically from 1 to 20 carbon atoms, for example from 1 to 10 carbon atoms, such as from 1 to 8 or from 1 to 6 carbon atoms. For example, C₁-C₆ alkyl refers to both straight chain and branched chain alkyl groups encompassing from 1 to 6 carbon atoms. When referring to an alkyl residue with a specific amount of carbon, it is intended to encompass all branched and straight chain forms with that amount of carbon; thus, for example, "butyl" is meant to encompass n-butyl, sec-butyl, isobutyl and tert-butyl; "propyl" includes n-propyl and isopropyl. Alkylene is a subset of alkyl, referring to the same residues as alkyl, but having two attachment points.

As used herein, "alkenyl" refers to an unsaturated branched or straight chain alkyl group having one or more carbon-carbon double bonds obtained by removing one hydrogen molecule from two adjacent carbon atoms of the parent alkyl. The group can be in either cis or trans configuration of the double bond(s). Typical alkenyl groups include, but are not limited to: ethenyl; propenyl, such as prop-1-en-1-yl, prop-1-en-2-yl, prop-2-en-1-yl (allyl), prop-2-en-2-yl; butenyl, such as, but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl; and the like. In certain embodiments, an alkenyl group has from 2 to 20 carbon atoms, and in other embodiments, from 2 to 10, from 2 to 8, or from 2 to 6 carbon atoms. Alkenylene is a subset of alkenyl, referring to the same residues as alkenyl, but having two attachment points.

As used herein, "alkynyl" refers to an unsaturated branched or straight chain alkyl group having one or more carbon-carbon triple bonds obtained by removing two hydrogen molecules from two adjacent carbon atoms of the parent alkyl. Typical alkynyl groups include, but are not limited to, ethynyl; propynyl, such as, prop-1-yn-1-yl, prop-2-yn-1-yl; butynyl, such as, but-1-yn-1-yl, but-1-yn-3-yl, but-3-yn-1-yl; and the like. In certain embodiments, an alkynyl group has from 2 to 20 carbon atoms, and in other embodiments, from 2 to 10, 2 to 8, or 2 to 6 carbon atoms. Alkynylene is a subset of alkynyl, referring to the same residues as alkynyl, but having two attachment points.

As used herein, "heterocyclyl" refers to a stable 3- to 18- membered non-aromatic ring radical that comprises 2 - 12 carbon atoms and 1 - 6 heteroatoms selected from nitrogen, oxygen or sulfur. Unless otherwise stated in the specification, the heteroaryl can be a monocyclic, bicyclic, tricyclic or tetracyclic ring system, and may include fused or bridged ring system(s). The heteroatom(s) in the heteroaryl is optionally oxidized. One or more nitrogen atoms, if present, are optionally quaternized. The heteroaryl is partially saturated or fully saturated. The heteroaryl may be linked to the rest of the molecule through any atom of the ring(s). Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxapiperazinyl, 2-oxapiperidinyl, 2-oxapyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. heterocyclylene is a subset of heterocyclyl, referring to the same residues as heterocyclyl, but having two attachment points.

As used herein, "aryl" refers to a radical derived from an aromatic monocyclic or multicyclic hydrocarbon ring system by removing a hydrogen atom from a ring carbon atom. The aromatic monocyclic or multicyclic hydrocarbon ring system contains only hydrogen and carbon, including from 6 to 18 carbon atoms, wherein one or more rings in the ring system is fully unsaturated, i.e., it contains a cyclic, delocalized (4n+2)π-electron system in accordance with the Hückel theory. Aryl groups include, but are not limited to, groups such as phenyl, fluorenyl, and naphthyl. Arylene is a subset of aryl, referring to the same residues as aryl, but having two attachment points.

"Heteroaryl" refers to a radical derived from a 3- to 18- membered aromatic ring radical that comprises from 2 to 17 carbon atoms and 1 to 6 heteroatoms selected from nitrogen, oxygen or sulfur. As used herein, the heteroaryl may be a monocyclic, bicyclic, tricyclic or tetracyclic ring system, wherein one or more rings in the ring system is fully unsaturated, i.e., it contains a cyclic, delocalized (4n+2)π-electron system in accordance with the Hückel theory. Heteroaryl includes fused or bridged ring system(s). The heteroatom(s) in the heteroaryl is optionally oxidized. One or more nitrogen atoms, if present, are optionally quaternized. The heteroaryl may be linked to the rest of the molecule through any atom of the ring(s). Examples of heteroaryls include, but are not limited to, azepinyl, acridinyl, benzimidazolyl, benzindolyl, 1,3-benzodioxazolyl, benzofuranyl, benzoxazolyl, benzo[d]thiazolyl, benzothiadiazolyl, benzo[b][1,4]dioxepinyl, benzo[b][1,4]oxazinyl, 1,4-benzodioxanyl, benzonaphthofuranyl, benzoxazolyl, benzodioxolyl, benzodioxinyl, benzopyranyl, benzopyranonyl, benzofuranyl, benzofuranonyl, benzothienyl, benzothieno[3,2-d]pyrimidinyl, benzotriazolyl, benzo[4,6]imidazo[1,2-a]pyridinyl, carbazolyl, cinnolinyl, cyclopenta[d]pyrimidinyl, 6,7-dihydro-5H-cyclopenta[4,5]thieno[2,3-d]pyrimidinyl, 5,6-dihydrobenzo[h]quinazolinyl, 5,6-dihydrobenzo[h]cinnolinyl, 6,7-dihydro-5H-benzo[6,7]cyclohepta[1,2-c]pyridazinyl, dibenzofuranyl, dibenzothienyl, furanyl, furanonyl, furo[3,2-c]pyridinyl, 5,6,7,8,9,10-hexahydrocycloocta[d]pyrimidinyl, 5,6,7,8,9,10-hexahydrocyclohepta[d]pyridazinyl, 5,6,7,8,9,10-hexahydrocycloocta[d]pyridinyl, isothiazolyl, imidazolyl, indazolyl, indolyl, isoindolyl, indolinyl, isoindolinyl, isoquinolyl, indolizinyl, isoxazolyl, 5,8-methano-5,6,7,8-tetrahydroquinazolinyl, naphthyridinonyl, 1,6-naphthyridinonyl, oxadiazolyl, 2-oxoazepinyl, oxazolyl, oxiranyl, 5,6,6a,7,8,9,10,10a-octahydrobenzo[h]quinazolinyl, 1-phenyl-1H-pyrrolyl, phenazinyl, phenothiazinyl, phenoxazinyl, phthalazinyl, pteridinyl, purinyl, pyrrolyl, pyrazolyl, pyrazolo[3,4-d]pyrimidinyl, pyridinyl, pyrido[3,2-d]pyrimidinyl, pyrido[3,4-d]pyrimidinyl, pyrazinyl, pyrimidinyl, pyridazinyl, pyrrolyl, quinazolinyl, quinoxalinyl, quinolinyl, tetrahydroquinolinyl, 5,6,7,8-tetrahydroquinazolinyl, 5,6,7,8-tetrahydrobenzo[4,5]thieno[2,3-d]pyrimidinyl, 6,7,8,9-tetrahydro-5H-cyclohepta [4,5]thieno[2,3-d]pyrimidinyl, 5,6,7,8-tetrahydropyrido[4,5-c]pyridazinyl, thiazolyl, thiadiazolyl, triazolyl, tetrazolyl, triazinyl, thieno[2,3-d]pyrimidinyl, thieno[3,2-d]pyrimidinyl, thieno[2,3-c]pridinyl and thiophenyl/thienyl. Heteroarylene is a subset of heteroaryl, referring to the same residues as heteroaryl, but having two attachment points.

### Conjugates provided by the present disclosure

In one aspect, the present disclosure provides a conjugate comprising one or more delivery groups and one or more functional groups; the delivery group is formed by removing one or more hydrogen atoms or one or more functional radicals from the above aptamer; each of the delivery groups is independently connected to the functional group via a covalent bond or via a linking group; each of the functional groups is selected from a small molecule therapeutic agent group having a therapeutic effect on tumors. The conjugates of the present disclosure can deliver the functional group to tumors, whererin the conjugate is formed by connecting the functional group via a covalent bond or via a linking group. In the conjugate provided by the present disclosure, the delivery group is formed by removing one or more hydrogen atoms or one or more functional radicals from an aptamer, the aptamer comprises a segment of contiguous nucleotide sequence, the group connecting two adjacent nucleotides is independently a phosphate group or a phosphate group with a modified group, each nucleotide is selected from one of modified or unmodified A, U, C or G, and the contiguous nucleotide sequence has a sequence as shown by Formula (1):

5'-T₁-S₁-Nₐ-S₂-N_{b}-S₃-N_{c}-S₄-T₂-3' Formula (1)

wherein T₁ is a motif consisting of 1-3 nucleotides. The inventors found that the presence of T₁ may make the conjugates provided by the present disclosure to exhibit efficient tumor targeting effect. In some embodiments, T₁ is consisted of 2 nucleotides. In this case, the conjugates provided by the present disclosure have more excellent tumor targeting ability. In some embodiments, T₁ is consisted of 2 nucleotides and contains at least one C. In some embodiments, in the 5'-3' direction, T₁ is CU, UC or AC.

T₂ is a motif consisting of 0-15 nucleotides. The inventors found that T₂ with these nucleotide numbers and with various nucleotide sequences does not significantly affect the tumor targeting ability of the conjugates provided by the present disclosure. In some embodiments, T₂ is a motif consisting of 0-10 nucleotides. In some embodiments, in the 5'-3' direction, T₂ is consisted of 1-9 nucleotides starting with U. In this case, the aptamer may have more excellent stability.

T₂ does not contain a motif that is completely reverse complementary to T₁. In the context of the present disclosure, "reverse complementary" means that hydrogen bond connection are formed between two segments of nucleotide sequence or motifs according to the law of nucleic acid base pairing, and each nucleotide of a segment of nucleotide sequence or motif in the 5'-3' direction can sequentially form base pairs with each nucleotide of the other end nucleotide sequence or motif in the 3'-5' direction. In some embodiments, "reverse complementation" includes one or more of AU, GC, and UG complementation.

S₁ and S₄ are motifs consisting of 3-7 nucleotides, respectively, and S₁ and S₄ have the same length and are completely reverse complementary. The aptamer with the above S₁ and S₄ motifs has better stability and can target tumor tissues and cells over a long time period. In some embodiments, S₁ and S₄ are consisted of 3-5 nucleotides, respectively, and have the same length. In some embodiments, in the reverse complementation formed by S₁ and S₄, GC complementation accounts for more than 40% of the total number of all complementations. In this case, the conjugates provided by the present disclosure have further more excellent stability and tumor targeting ability. In some embodiments, in the 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

Nₐ and N_{c} are motifs consisting of 1-4 nucleotides, respectively. Each nucleotide in the Nₐ is not complementary to each nucleotide in N_{c}, and the total number of U in Nₐ and N_{c} accounts for more than 50% of the total number of all nucleotides in Nₐ and N_{c}. Aptamer with the above Nₐ and N_{c} motifs shows excellent tumor tissue targeting ability. In some embodiments, the sum of the number of nucleotides in Nₐ and N_{c} is an integer of 2-4. In some embodiments, the sum of the number of nucleotides in Nₐ and N_{c} is 3 or 4, and the sum of the number of U in Nₐ and N_{c} is 2 or 3. In some embodiments, in the 5'-3' direction, Nₐ and/or N_{c} are U, UU, UC or CU.

S₂ and S₃ are motifs consisting of 1-4 nucleotides, respectively, and S₂ and S₃ have the same length and are completely reverse complementary. By comprising S₂ and S₃ motifs, the conjugates provided by the present disclosure show good stability and excellent tumor targeting ability. In some embodiments, S₂ and S₃ are consisted of 2-3 nucleotides, respectively, and have the same length. In some embodiments, the reverse complementation formed by S2 and S3 contains at least one GC complementation. In this case, the reverse complementation has better stability. In some embodiments, in the 5'-3' direction, S₂ is CA and S₃ is UG, or S₂ is AC and S₃ is GU, or S₂ is GCC and S₃ is GGU.

N_{b} is a motif consisting of 3-6 nucleotides, and the nucleotides at both ends of N_{b} are not complementation of AU or GC. Without being bound to theory, the aptamer with the above N_{b} motif can maintain a specific configuration in terms of space, thereby allowing the conjugates provided by the present disclosure to stably and efficiently target tumor tissues and cells. In some embodiments, N_{b} is consisted of 4-5 nucleotides. In some embodiments, in the 5'-3' direction, N_{b} is GACG, GACGU, GACCG, UACU, GUUG or GAUCU.

The inventors of the present disclosure unexpectedly found that a delivery group formed by an aptamer with the sequence as shown by the above Formula (1) can effectively target tumors, especially glioma tissues, thereby allowing the conjugates provided by the present disclosure to specifically enter into tumor cells, and thus more efficiently delivering therapeutic agent group at the cellular level.

In some embodiments, in the conjugates provided by the present disclosure, the contiguous nucleotide sequence has a length of 18-50 nucleotides, or 20-40 nucleotides, or 21-36 nucleotides, or 24-32 nucleotides. The delivery group formed by an aptamer having the length of these contiguous nucleotides and the conjugate provided by the present disclosure comprising the delivery group can more easily target tumors, and have a good balance in terms of synthesis cost and targeting effect.

In some embodiments, in the conjugates provided by the present disclosure, the contiguous nucleotide sequence has the following sequence as shown in SEQ ID NO: 1, SEQ ID NO: 2, or SEQ ID NO: 3:
5'-CUGCUUCAGACGUGUUUAGCUU-3' (SEQ ID NO: 1)
wherein in the 5'-3' direction, T₁ is CU, S₁ is GCU, Nₐ is U, S₂ is CA, N_{b} is GACG, S₃ is UG, N_{c} is UU, S₄ is AGC, T₂ is UU;
5'-CUGAGUUCAGACGUGUUGCUCU-3' (SEQ ID NO: 2)
wherein in the 5'-3' direction, T₁ is CU, S₁ is GAGU, Nₐ is U, S₂ is CA, N_{b} is GACG, S₃ is UG, N_{c} is UU, S₄ is GCUC, T₂ is U;
5'- UCUAUGGCUGCCGAUCUGGUCUCCAUGUACGU -3' (SEQ ID NO: 3),
wherein in the 5'-3' direction, T₁ is CU, S₁ is GAGU, Nₐ is U, S₂ is CA, N_{b} is GACG, S₃ is UG, N_{c} is UU, S₄ is GCUC, T₂ is U.

In some embodiments, the contiguous nucleotide sequence has the nucleotide sequence as shown in SEQ ID NO: 4:
5'- N₆GGAGUUCAN₁N₂N₃N₄UGN₅GCUCN₇ -3' (SEQ ID NO: 4),
wherein, N₁, N₂ and N₃ independently are one of A, U, C and G, N₄ is U, C or G or a motif consisting of two of U, C or G; N₅ is U, CU or UU; N₆ is CU, UC or AC; N₇ is U, UU or UUN₈, and N₈ is a motif consisting of 1-15 nucleotides.

In the above nucleotide sequence, in the 5'-3' direction, T₁ is the motif as shown by N₆, S₁ is the motif represented for GGAGU, Nₐ is U, S₂ is CA, N_{b} is the motif N₁N₂N₃N₄ consisting of N₁, N₂, N₃ and N₄, S₃ is UG, N_{c} is the motif as shown by N₅, S₄ is the motif consisting of GCUC and the first nucleotide in N₇, and T₂ is the motif consisting of the remaining nucleotides in N₇.

The aptamer containing the above nucleotide sequence as shown in SEQ ID NO: 4 can more effectively target tumors, especially gliomas, and can be enriched in tumor tissues.

Experimental verification shows that, in the nucleotide sequence as shown in SEQ ID NO: 4, the above selection of N₁, N₂, N₃ and N₄ does not significantly affect the tumor targeting ability of the conjugates provided by the present disclosure. In some embodiments, the motif N₁N₂N₃N₄ consisting of N₁, N₂, N₃ and N₄ is one of GACG, GACGU, GACCG, UACU, GUUG, or GAUCU, and the aptamer containing these motifs have higher tumor-specific targeting effect.

In some embodiments, in the nucleotide sequence as shown in SEQ ID NO: 4, N₅ is U or UU. In this case, the conjugates provided by the present disclosure all have excellent targeting effect on tumors.

In some embodiments, the aptamer has the nucleotide sequence as shown in any one of SEQ ID NOs: 5-11:
5'- CUGGAGUUCAGACGUGUUGCUCUU -3' (SEQ ID NO: 5)
5'- CUGGAGUUCAGACGUUGUGCUCUU -3' (SEQ ID NO: 6)
5'- CUGGAGUUCAGACCGUGUGCUCUU -3' (SEQ ID NO: 7)
5'- CUGGAGUUCAGACGUGUUGCUCU -3' (SEQ ID NO: 8)
5'- ACGGAGUUCAGACGUGUUGCUCUU -3' (SEQ ID NO: 9)
5'- CUGGAGUUCACUACUGUUGCUCUU -3' (SEQ ID NO: 10)
5'- CUGGAGUUCAGUUGUGUUGCUCUU -3' (SEQ ID NO: 11).

The conjugates provided by the present disclosure with the nucleotide sequences as described above show a highly targeting effect on tumors.

In some embodiments, motif N₈ is consisted of 1-15 nucleotides. In some embodiments, N₈ is consisted of 1-8 nucleotides.

In some embodiments, the presence of motif N₈ makes the conjugates provided by the present disclosure more stable to an *in vivo* exonuclease, thereby allowing them to exert targeting effect on tumors *in vivo* over a longer time period. In some embodiments, T₈ can increase or maintain the targeting effect on tumors of the conjugates provided by the present disclosure. In some embodiments, motif N₈ is consisted of 8 nucleotides, considering the balance of stability, targeting, and synthesis efficiency. In some embodiments, in the 5'-3' direction, the nucleotide sequence of motif N₈ is CCGAUCUC. In some embodiments, the contiguous nucleotide sequence has the nucleotide sequence as shown in any one of SEQ ID NOs: 12-14:
5'-CUGGAGUUCAGACGUGUUGCUCUUCCGAUCUC-3' (SEQ ID NO: 12)
5'-CUGGAGUUCAGACGUUGUGCUCUUCCGAUCUC-3' (SEQ ID NO: 13)
5'-CUGGAGUUCAGACCGUGUGCUCUUCCGAUCUC-3' (SEQ ID NO: 14)

In the contiguous nucleotide sequence, the terminal groups of the ribose 5' end of the 5' terminal nucleotide and the ribose 3' end of the 3' terminal nucleotide are independently hydroxyl or phosphate groups, and the selection of these terminal groups does not change the targeting ability of the conjugates provided by the present disclosure. In some embodiments, in the contiguous nucleotides, the terminal groups of the ribose 5' end of the 5' terminal nucleotide and the ribose 3' end of the 3' terminal nucleotide are both hydroxyl groups.

In the conjugates provided by the present disclosure, each nucleotide may be a modified or unmodified nucleotide. In general, modifications of nucleotides may alter the stability and/or the targeting ability on tumors of the conjugates provided by the present disclosure. In some embodiments, at least one nucleotide in the conjugates provided by the present disclosure is a modified nucleotide. In some embodiments, at least one group connecting two adjacent nucleotides in the conjugates provided by the present disclosure is a phosphate group with a modified group.

The modifications of nucleotides include, but are not limited to, modifications of saccharide, modifications of bases, and/or substitution of nucleotides with nucleotide analogues. In some embodiments, in the conjugates provided by the present disclosure, each of the modified nucleotides is independently one of 2'-halogen modified nucleotide, 2'-alkoxy modified nucleotide, 2'-alkyl modified nucleotide, 2'-substituted alkyl modified nucleotide, 2'-amino modified nucleotide, 2'-substituted amino modified nucleotide, 2'-deoxy nucleotide, base modified nucleotide, and nucleotide analogue.

In the context of the present disclosure, "fluoro modified nucleotide" refers to a nucleotide formed by substituting 2'-hydroxy of the ribose group of the nucleotide with a fluoro atom, which has a structure as shown by the following Formula (7). "Non-fluoro modified nucleotide" refers to a nucleotide formed by substituting the 2'-hydroxy of the ribose group of the nucleotide with a non-fluoro group, or a nucleotide analogue. In some embodiments, each non-fluoro modified nucleotide is independently selected from the group consisting of a nucleotide formed by substituting the 2'-hydroxy of the ribose group of the nucleotide with a non-fluoro group, or a nucleotide analogue.

These nucleotides formed by substituting the 2'-hydroxy of the ribose groups with non-fluoro groups are well-known to those skilled in the art, and can be selected from one of 2'-alkoxy modified nucleotide, 2'-alkyl modified nucleotide, 2'-substituted alkyl modified nucleotide, 2'-amino modified nucleotide, 2'-substituted amino modified nucleotide and 2'-deoxy nucleotide.

In some embodiments, the 2'-alkoxy modified nucleotide is a methoxy modified nucleotide (2'-OMe), as shown by Formula (8). In some embodiments, the 2'-amino modified nucleotide (2'-NH₂) is as shown by Formula (9). In some embodiments, the 2'-deoxy nucleotide (DNA) is as shown by Formula (10):

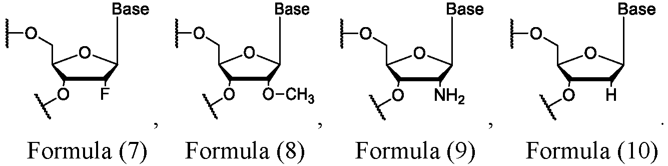

Those skilled in the art know various ways for modifying the bases of nucleotides. In some embodiments, base modification includes, but is not limited to, adding one or more methyl groups to a base. In some embodiments, thymine (T) is considered as one of the base-modified uracil (U). In some embodiments, 2-methylcytosine is considered as one of the base-modified cytosine (C).

A nucleotide analogue refers to a group that can replace a nucleotide in a nucleic acid, while structurally differs from an adenine ribonucleotide, a guanine ribonucleotide, a cytosine ribonucleotide, a uracil ribonucleotide or thymine deoxyribonucleotide. In some embodiments, a nucleotide analogues may be an isonucleotide, a bridged nucleic acid (abbreviated as BNA) or an acyclic nucleotide.

A BNA refers to a constrained or inaccessible nucleotide. BNA can contain a 5-, 6- membered or a 7-membered ring bridged structure with a "fixed" C3'-endo sugar puckering. The bridge is typically incorporated at the 2'- and 4'-position of the ribose to afford a 2', 4'-BNA nucleotide. In some embodiments, BNA may be LNA, ENA, cET BNA and so on, which are as shown by Formulae (12), (13) and (14), respectively.

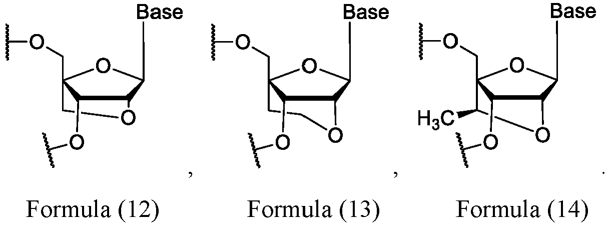

An acyclic nucleotide refers to a class of nucleotides in which the sugar ring is opened. In some embodiments, the acyclic nucleotide may be an unlocked nucleic acid (UNA), a glycerol nucleic acid (GNA) or a peptide nucleic acid (PNA), wherein UNA is as shown by Formula (15) and GNA is as shown by Formula (16):

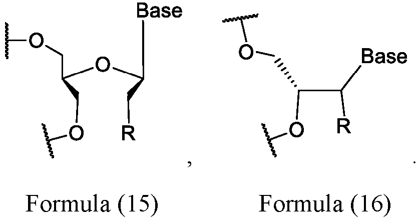

In the above Formulae (15) and (16), R is selected from H, OH, or alkoxy (O-alkyl).

A peptide nucleic acid refers to a class of nucleotide analogues formed by replacing the glycoside-phosphate backbone with the polypeptide backbone. In some embodiments, the peptide nucleic acid may be, for example, a nucleotide analogue formed by replacing the glycoside-phosphate unit with the 2-aminoethyl glycine bond.

An isonucleotide is a compound formed by changing the position of the base on the ribose ring in the nucleotide. In some embodiments, the isonucleotide may be a compound formed by transposing the base from 1'-position to 2'-position or 3'-position on the ribose ring, as shown by Formula (17) or (18), respectively.

In the above compounds of Formulae (217)-(218), "Base" represents a base of a nucleic acid, such as A, U, G, C, or T; R is selected from H, OH, F, or the above non-fluoro group.

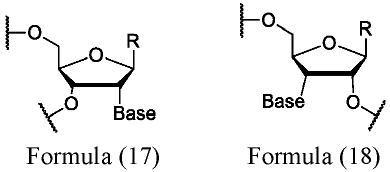

In some embodiments, a nucleotide analogue is selected from the group consisting of an isonucleotide, LNA, ENA, cET, UNA, and GNA. In some embodiments, each non-fluoro modified nucleotide is a methoxy modified nucleotide. In the context of the present disclosure, the methoxy modified nucleotide refers to a nucleotide formed by substituting the 2'-hydroxy of the ribose group with a methoxy group.

In the context of the present disclosure, a "fluoro modified nucleotide", a "2'-fluoro modified nucleotide", a "nucleotide in which 2'-hydroxy of the ribose group is substituted with a fluoro group" and a "nucleotide with 2'-fluororibosyl" have the same meaning, referring to a compound in which 2'-hydroxy of the nucleotide is substituted with a flurorin atom, which has a structure as shown by Formula (7). A "methoxy modified nucleotide", a "2'-methoxy modified nucleotide", a "nucleotide in which 2'-hydroxy of a ribose group is substituted with a methoxy" and a "nucleotide with 2'-methoxyribosyl" have the same meaning, referring to a compound in which 2'-hydroxy of the ribose group in the nucleotide is substituted with a methoxy, and has a structure as shown by Formula (8).

In some embodiments, each cytosine nucleotide of the contiguous nucleotide sequence in the conjugate provided by the present disclosure is a fluoro-modified cytosine nucleotide, and/or each uracil nucleotide of the contiguous nucleotide sequence in the conjugate provided by the present disclosure is a fluoro-modified uracil nucleotide. In some embodiments, each nucleotide of the contiguous nucleotide sequence in the conjugate provided by the present disclosure is a 2'-methoxy modified nucleotide. In some embodiments, one or more uracil nucleotides in the conjugate provided by the present disclosure have modified bases. In some embodiments, a thymine base (T) is considered as a uracil base (U) with methyl modification.

The group connecting two adjacent nucleotides may be a phosphate group or a modified phosphate group. For example, the modified phosphate group is a phosphorothioate group or a phosphorodithioate group formed by replacing at least one non-bridged oxygen atom in the phosphate group with a sulfur atom. In some embodiments, at least one group connecting two adjacent nucleotides in the conjugate provided by the present disclosure is phosphorothioate groups. In some embodiments, at least one group of the three groups connecting two adjacent nucleotides between the first four nucleotides in the 5' terminal of the contiguous nucleotide sequence is a phosphorothioate group. In some embodiments, at least two groups of the three groups connecting two adjacent nucleotides between the first four nucleotides in the 5' terminal of the contiguous nucleotide sequence are phosphorothioate groups. In some embodiments, at least one group connecting two adjacent nucleotides between the first four nucleotides in the 3' terminal of the contiguous nucleotide sequence is a phosphorothioate group. In some embodiments, at least two groups of the three groups connecting two adjacent nucleotides between the first four nucleotides in the 3' terminal of the contiguous nucleotide sequence are phosphorothioate groups. In some embodiments, each group connecting two adjacent nucleotides in the contiguous nucleotide sequence is a phosphorothioate group.

The conjugates provided by the present disclosure with the above modification is not only low-cost, but also can make the endogenous ribonuclease less likely to cleave the linking group, thereby increasing the stability of the conjugates provided by the present disclosure and having more resistant to nuclease hydrolysis. Meanwhile, the conjugates provided by the present disclosure comprising delivery groups with the above modification have higher activity to target tumor tissues and/or cells.

In some embodiments, the contiguous nucleotide sequence has the nucleotide sequence as shown in any one of SEQ ID NOs: 15-39:
5'- CfUfGGAGUfUfCfAGACfGUfGUfUfGCfUfCfUfUfCfCfGAUfCfUfCf -3' (SEQ ID NO: 15)
5'- CfUfGGAGUfUfCfAGACfGUfGUfUfGCfUfCfUfUf -3' (SEQ ID NO: 16)
5'- CfUfGGAGUfUfCfAGACfGUfUfGUfGCfUfCfUfUfCfCfGAUfCfUfCf -3' (SEQ ID NO: 17)
5'- CfUfGGAGUfUfCfAGACfGUfUfGUfGCfUfCfUfUf -3' (SEQ ID NO: 18)
5'- CfUfGGAGUfUfCfAGACfCfGUfGUfGCfUfCfUfUfCfCfGAUfCfUfCf -3' (SEQ ID NO: 19)
5'- CfUfGGAGUfUfCfAGACfCfGUfGUfGCfUfCfUfUf -3' (SEQ ID NO: 20) 
5'-
CmUmGmGmAmGmUmUmCmAmGmAmCmGmUmGmUmUmGmCmUmCmUmUmCmC
mGmAmUmCmUmCm -3' (SEQ ID NO: 21)
 5'-CmUmGmGmAmGmUmUmCmAmGmAmCmGmUmGmUmUmGmCmUmCmUmUmCmC mGmAmUmCmUmCm -3' (SEQ ID NO: 21) 
5'-
CmUmGmGmAmGmUmUmCmAmGmAmCmGmUmUmGmUmGmCmUmCmUmUmCmC
mGmAmUmCmUmCm -3' (SEQ ID NO: 23)
5'- CmUmGmGmAmGmUmUmCmAmGmAmCmGmUmUmGmUmGmCmUmCmUmUm -3' (SEQ ID NO: 24) 
5'-
CmUmGmGmAmGmUmUmCmAmGmAmCmCmGmUmGmUmGmCmUmCmUmUmCmC
mGmAmUmCmUmCm -3' (SEQ ID NO: 25)
5'- CmUmGmGmAmGmUmUmCmAmGmAmCmCmGmUmGmUmGmCmUmCmUmUm -3' (SEQ ID NO: 26)
5'- CmUmGmGmAmGmUmUmCmAmGmAmCmGmUmGmUmUmGmCmUmCmUm -3' (SEQ ID NO: 27)
5'- CmUmGmAmGmUmUmCmAmGmAmCmGmUmGmUmUmGmCmUmCmUm -3' (SEQ ID NO: 28)
5'- CmUmGmCmUmUmCmAmGmAmCmGmUmGmUmUmAmGmCmUmUm -3' (SEQ ID NO: 29)
5'- AmCmGmGmAmGmUmUmCmAmGmAmCmGmUmGmUmUmGmCmUmCmUmUm -3' (SEQ ID NO: 30)
5'- CmUmGmGmAmGmUmUmCmAmCmUmAmCmUmGmUmUmGmCmUmCmUmUm -3' (SEQ ID NO: 31) 
5'-      CmUmGmGmAmGmUmUmCmAmGmUmUmGmUmGmUmUmGmCmUmCmUmUm
-3' (SEQ ID NO: 32)
 
5'-
UmCmUmAmUmGmGmCmUmGmCmCmGmAmUmCmUmGmGmUmCmUmCmCmAmU
mGmUmAmCmGmUm -3' (SEQ ID NO: 33)
 
5'-
CmsUmsGmGmAmGmUmUmCmAmGmAmCmGmUmGmUmUmGmCmUmCmUmUmCm
CmGmAmUmCmsUmsCm -3' (SEQ ID NO: 34)
 
5'-
CmsUmsGmGmAmGmUmUmCmAmGmAmCmGmUmGmUmUmGmCmUmCmsUmsUm
-3' (SEQ ID NO: 35)
 
5'-
CmsUmsGmGmAmGmUmUmCmAmGmAmCmGmUmUmGmUmGmCmUmCmUmUmCm
CmGmAmUmCmsUmsCm -3' (SEQ ID NO: 36)
 
5'-
CmsUmsGmGmAmGmUmUmCmAmGmAmCmGmUmUmGmUmGmCmUmCmsUmsUm
-3' (SEQ ID NO: 37)
 
5'-
CmsUmsGmGmAmGmUmUmCmAmGmAmCmCmGmUmGmUmGmCmUmCmUmUmCm
CmGmAmUmCmsUmsCm -3' (SEQ ID NO: 38)
 
5'-
CmsUmsGmGmAmGmUmUmCmAmGmAmCmCmGmUmGmUmGmCmUmCmsUmsUm
-3' (SEQ ID NO: 39)
wherein C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; s represents the two nucleotides adjacent to both sides of the letter s are linked by a thiophosphate linkage

In some embodiments, the conjugate provided by the present disclosure has a structure as shown by Formula (101): 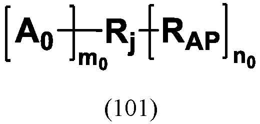
wherein each R_{AP} group independently is a group having a structure as shown by Formula (2): 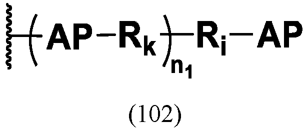
where each AP group is the same or different, and independently represents one of the delivery groups; each A₀ group is the same or different, and independently represents one of the functional groups; Rⱼ, each Rₖ or each Rᵢ is the same or different, and independently represents a covalent bond or a linking group, and Rᵢ and Rₖ are not both a covalent bond at the same time; m₀ is an integer of 1-6; n₀ is an integer of 1-6, each n₁ independently represents an integer of 0-4; 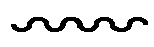 represents the site where the group is covalently linked.

In some embodiments, m₀ is an integer of 1-6, that is, the conjugate represented by Formula (101) contains 1 to 6 of the functional groups of A₀. In some embodiments, considering delivery efficiency and synthesis cost, m₀ is an integer of 1-4, that is, the conjugate represented by Formula (101) contains 1 to 4 of the functional groups A₀. In some embodiments, m₀ is 1, that is, the conjugate represented by Formula (101) contains 1 of the functional group A₀.

In some embodiments, n₀ is an integer of 1-6, that is, the conjugate represented by Formula (101) contains 1 to 6 of R_{AP} groups. In some embodiments, considering delivery efficiency and cost, n₀ is an integer of 1-3, that is, the conjugate represented by Formula (101) contains 1 to 3 of R_{AP}. In some embodiments, n₀ is 1, that is, the conjugate represented by Formula (101) contains 1 of R_{AP}.

In some embodiments, each n₁ independently represents an integer of 0-4, and Rᵢ and Rₖ are not both a covalent bond at the same time, so that each R_{AP} contains 1 to 5 of the delivery group AP. In some embodiments, each n₁ independently represents an integer of 0-1, so that each R_{AP} contains 1-2 of the delivery group AP. In some embodiments, n₀ is 1 and n₁ is 0, that is, the conjugate represented by Formula (101) contains 1 of the functional group AP.

In the R_{AP} group, the role of Rₖ and Rᵢ is to covalently connect the delivery group AP to the Rⱼ group, and connect to the functional group A₀ via the Rⱼ group. Therefore, as long as the above-mentioned linkage can be achieved, and any Rₖ or Rᵢ that has no negative effect on the role of the delivery group AP and the functional group A₀ can be used in the present invention. In some embodiments, each of the Rₖ or each of the Rᵢ is independently a linear alkylene group of 1-70 carbon atoms in length, or one or more carbon atoms in the linear alkylene group are replaced with one or more groups selected from the group consisting of: C(O), NH, O, S, CH=N, S(O)₂, OP(O)₂, OP(O)(S), C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene optionally may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl) (C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl). In some embodiments, considering the cost and difficulty of synthesis and the tumor targeting effect of the conjugates, each n₁ is 0, and each Rᵢ is independently a covalent bond, or any one linking group or a connection combination of more linking groups selected from the group consisting of: C₁-C₂₀ alkylene, phosphate bond, phosphorothioate bond, amide bond, ester bond, ether bond, thioether bond, disulfide bond, 1,2,3-triazolylidene group, a subunit of polyethylene glycol, pyrrolidinylidene group, 2-oxopyrrolidinylidene group, phenylene, cyclohexylene, 2-succinimidoylidene group, 2-thiosuccinimidoylidene group, a subunit of amino acid, a subunit of nucleotide.

In some embodiments, the linking group Rⱼ contains linking groups that are well-known to those skilled in the art to be useful for antibody-conjugated drugs. The linking group Rⱼ may be cleavable or non-cleavable. In some embodiments, the linking group Rⱼ may be cleavable. In the context of the present disclosure, "cleavable" refers that after the conjugate of the present disclosure targeting to a tumor, covalent bond cleavage of the linking group Rⱼ occurs in the tumor internal environment and/or within tumor cells, releasing a individual therapeutic agent group to produce a therapeutic effect. In some embodiments, the linking group Rⱼ contains one or more of an activating enzyme linking group, a sulfatase-cleavable linking group, a galactose cleavable linking group, a lysosomal protease-sensitive linking group, a peptidyl linking group, a glucuronide linking group, an acid-sensitive cleavable linking group, or a glutathione-sensitive disulfide linking group. In some embodiments, the linking group Rⱼ contains a peptidyl linking group. In some embodiments, the peptidyl linking group is selected from one or more of a valine-citrulline dipeptide linker (Val-Cit), an alanine-alanine dipeptide linker (Ala-Ala), a valine-alanine dipeptide linker (Val-Ala), a tetrapeptide linker of glycine-glycine-phenylalanine-glycine (Gly-Gly-Phc-Gly). In some embodiments, the linking group Rⱼ is selected from one of N-succinimidyl 4-(2-pyridyldithio) butanoate (SPDB), N-succinimido-4-(2-thiopyridinylene) pentanoate (SPP), (S)-2-((S)-2-amino-3-methylbutanamide)-5-ureidopentanoic acid (Val-Cit-PAB-OH), N-succinimidyl-4-(N-maleimidomethyl)cyclohexane-1-carboxylate (SMCC) or 2-(phosphate-(CH₂)₆-S-)-maleimidohexanoyl-valine-citrulline-p-aminobenzyl subunit. In some embodiments, the linking group Rⱼ contains the linking groups listed in Mckertish CM, Kayser V. Advances and Limitations of Antibody Drug Conjugates for Cancer. Biomedicines. 2021 Jul 23; 9 (8): 872., which are incorporated herein by reference in their entirety.

In some embodiments, the linking group Rⱼ contains one or more of a valine-citrulline dipeptide linker (Val-Cit), a subunit of polyethylene glycol, an iminohexylidene group, an N-succinimidyl group and a GAU trinucleotide linking group. In some embodiments, each Rᵢ is independently a covalent bond, a disulfide bond, a dodecanediyl group, a valine-citrulline dipeptide linker (Val-Cit), a subunit of polyethylene glycol, an iminohexylidene group, an N-succinimidyl group or a subunit of GAU trinucleotide, or a connection combination of two of a covalent bond, a disulfide bond, a dodecanediyl group, a valine-citrulline dipeptide linker (Val-Cit), a subunit of polyethylene glycol, an iminohexylidene group, an N-succinimidyl group or a subunit of GAU trinucleotide.

The role of the Rⱼ group is to connect the R_{AP} group with the functional group A₀, thereby the functional group A₀ is specifically delivered to tumor tissues and/or cells through the tumor targeting effect of the delivery group AP in the R_{AP} group. Therefore, any Rⱼ group that is capable of achieving the above-mentioned linkage without affecting the tumor targeting function of the delivery group AP and the effect of the functional group A₀ will achieve the purpose of the present invention and solve the technical problem to be solved by the present invention. In some embodiments, after the conjugate represented by Formula (1) reaches tumor tissue and/or enters into tumor cells, the cleavage of Rⱼ takes place, releasing a separate pharmaceutically active molecule corresponding to the functional group A₀. In some embodiments, no cleavage of Rⱼ takes place *in vivo.* In this case, the presence of the Rⱼ group and the R_{AP} in the conjugate have no effect on the functional group A₀ to play a therapeutic role.

In some embodiments, Rⱼ is a covalent bond, m₀ is 1. In this case, the conjugate represented by Formula (101) contains one functional group A₀ and one R_{AP} group, and each R_{AP} group is directly connected to the functional group A₀. In some embodiments, each R_{AP} group is connected to a same atom on the functional group A₀. In some embodiments, each R_{AP} group is connected to different atoms on the functional group A₀.

In some embodiments, Rⱼ is a linking group, which comprises a main chain moiety, a side chain moiety and a conjugated linking moiety.

The main chain moiety is respectively connected to the conjugated linking moiety and the side chain moiety. In some embodiments, the main chain moiety is a linear alkylene group of 1-70 carbon atoms in length, or one or more carbon atoms in the linear alkylene group are replaced with one or more groups selected from the group consisting of C(O), NH, O, S, CH=N, S(O)₂, OP(O)₂, C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl) (C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl).

The side chain moiety is respectively connected to the main chain moiety and the R_{AP} group. In some embodiments, each side chain moiety is independently a covalent bond or a linear alkylene group of 1 to 70 carbon atoms in length, or one or more carbon atoms in the linear alkylene group are replaced with one or more groups selected from the group consisting of C(O), NH, O, S, CH=N, S(O)₂, OP(O)₂, C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene optionally may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl) (C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl), and -NHSO₂(C₁-C₁₀ haloalkyl).

The conjugated linking moiety is respectively connected to the main chain moiety and the functional group A₀. In some embodiments, each conjugated linking moiety is independently a covalent bond, or any one linking structure or a connection combination of more linking structures selected from the group consisting of: C₁-C₁₀ linear alkylene, phosphate bond, phosphorothioate bond, amide bond, ester bond, ether bond, disulfide bond, 1,2,3-triazolylidene group, a subunit of polyethylene glycol, pyrrolidinylidene group, 2-oxopyrrolidinylidene group, phenylene, cyclohexylene, 2-succinimidoylidene group, 2-thiosuccinimidoylidene group, a subunit of amino acid, a subunit of nucleotide.

In some embodiments, each of the conjugated linking moieties in the linking group Rⱼ is respectively connected to the main chain moiety and one of the functional groups A₀; the number of the side chain moieties is n₀, and each of the side chain moieties is respectively connected to the main chain moiety and one of the R_{AP} groups. Thus, each functional group A₀ and the R_{AP} group are each independently connected to the linking group Rⱼ. In some embodiments, all side chain moieties are connected to a same atom on the main chain moiety; or, each side chain moiety is connected to a different atom in the main chain moiety.

In some embodiments, m₀ is 1, and the linking group Rⱼ comprises the structure as shown by Formula (301): 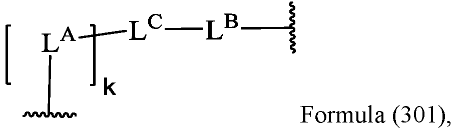 wherein k is an integer of 1-3; L^{C} is the main chain moiety, L^{A} is the side chain moiety, L^{B} is the conjugation linking moiety, and 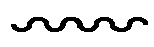 represents the site where the group is covalently linked.

The main chain moiety L^{C} is a covalent bond or a 2- to 4-valence, linear or branched C₁-C₂₅ saturated hydrocarbyl group, or one or more carbon atoms in the saturated hydrocarbyl group are replaced with one or more groups selected from the group consisting of: C(O), NH, O, S, CH=N, S(O)₂, OP(O)₂, C₅-C₈ glycosylene, C₂-C₅ alkenylene, C₂-C₅ alkynylene, C₆-C₁₀ arylene, C₃-C₈ heterocyclylene, and C₅-C₁₀ heteroarylene; and wherein the saturated hydrocarbyl group may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O)(phenyl), -C(O)C₁-C₅ alkyl, -C(O)C₁-C₅ alkylphenyl, -OC(O)C₁-C₅ alkyl, -SO₂(C₁-C₅ alkyl), -SO₂(phenyl), -SO₂NH₂, -SO₂NH(C₁-C₅ alkyl), -SO₂NH(phenyl), -NHSO₂(C₁-C₅ alkyl), and -NHSO₂(phenyl). In some embodiments, L^{C} is a 2- to 4-valence C₅-C₂₀ saturated hydrocarbyl group, or one or more carbon atoms in the saturated hydrocarbyl group are replaced with one or more groups selected from the group consisting of C(O), NH, O, S, CH=N, S(O)₂, OP(O)₂, C₅-C₈ glycosylene, C₂-C₅ alkenylene, C₂-C₅ alkynylene, C₆-C₁₀ arylene, C₃-C₈ heterocyclylene, and C₅-C₁₀ heteroarylene; and wherein the saturated hydrocarbyl group may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, nitro, and -CONH₂. In some embodiments, the length of L^{C} is 5-30 atoms, wherein the length of the L^{C} refers to the number of chained atoms in the longest chain of atoms formed by the atom directly connected with L^{A} to the atom directly connected with L^{B} in L^{C}. To simplify the structure, in some embodiments, the length of L^{C} is 8-25 atoms.

The side chain moiety L^{A} is a covalent bond, or C₁-C₂₀ alkylene group, or one or more carbon atoms in the alkylene group are replaced with one or more groups selected from the group consisting of: C(O), NH, O, S, CH=N, S(O)₂, OP(O)₂, C₅-C₈ glycosylene, C₂-C₅ alkenylene, C₂-C₅ alkynylene, C₆-C₁₀ arylene, C₃-C₈ heterocyclylene, and C₅-C₁₀ heteroarylene; and wherein the alkylene group may have any one or more substituents selected from the group consisting of: C₁-C₅ alkyl, C₆-C₁₀ aryl, C₅-C₁₀ heteroaryl, -O-C₁-C₅ alkyl, -OC₁-C₅ alkylphenyl, -C₁-C₅ alkyl-OH, -SC₁-C₅ alkyl, -SC₁-C₁₀ alkylphenyl, -C₁-C₅ alkyl-SH, -OH, -SH, -NH₂, -C₁-C₅ alkyl-NH₂, -N(C₁-C₅ alkyl)(C₁-C₅ alkyl), -NH(C₁-C₅ alkyl), -N(C₁-C₅ alkyl) (C₁-C₅ alkylphenyl), -NH(C₁-C₅ alkylphenyl), nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O)(phenyl), -C(O)C₁-C₅ alkyl, -C(O)C₁-C₅ alkylphenyl, -OC(O)C₁-C₅ alkyl, -SO₂(C₁-C₅ alkyl), -SO₂(phenyl), -SO₂NH₂, -SO₂NH(C₁-C₅ alkyl), -SO₂NH(phenyl), -NHSO₂(C₁-C₅ alkyl), and -NHSO₂(phenyl).

The conjugated linking moiety L^{B} is a connection combination of one or more of 1-5 linkages: phosphoester bond, phosphorothioate bond, amide bond, ester bond, ether bond, disulfide bond.

In some embodiments, k is an integer of 1-3; L^{C} contains any one of the groups represented by the formulae (L1) - (L3), and is connected with L^{A} moeity through an ether bond in the group represented by the formulae (L1) - (L3): 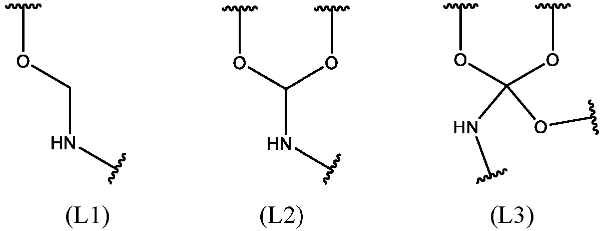 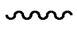 represents the site where the group is linked to the rest of the molecule;

In some embodiments, k = 1, L^{C} contains the group represented by Formula (L1), and the oxygen atom in the group (L1) is directly connected to L^{A}. In some embodiments, k = 2, L^{C} contains the group represented by Formula (L2), and each of two oxygen atoms in the group (L1) is directly connected to one of L^{A}. In some embodiments, k = 4, L^{C} contains the group represented by Formula (L3), and each of three oxygen atoms in the group (L3) is directly connected to one of L^{A}.
L^{B} is a phosphoester bond or a disulfide bond;
each L^{A} is a covalent bond, or each L^{A} is selected from the group consisting of the groups of (L4)-(L23) and the linking combinations thereof:

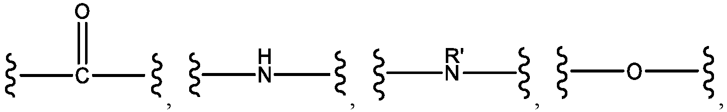

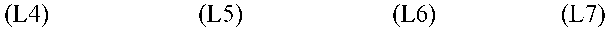

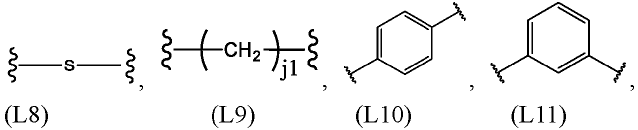

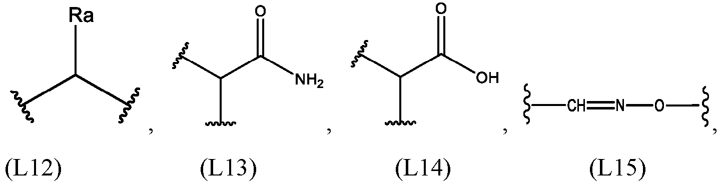

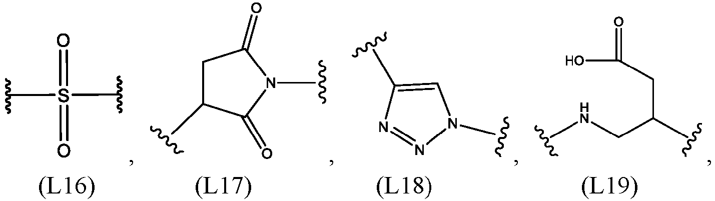

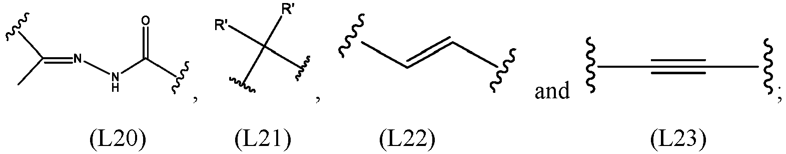

In the formula, each j1 is an integer of 1-10;
each R' is C₁-C₁₀ alkyl;
each Ra is a hydrogen atom, C₁-C₁₀ alkyl, or selected from the group consisting of the groups of (L24)-(L37):

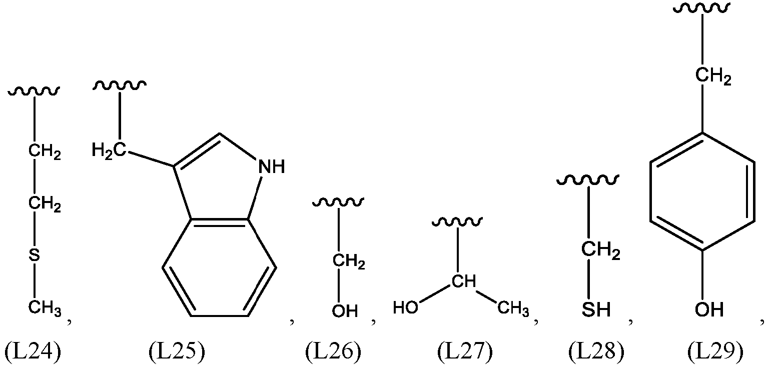

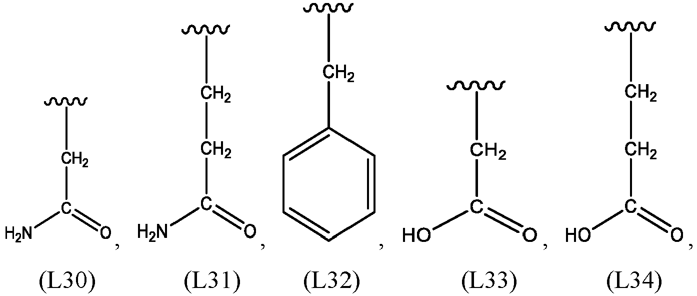

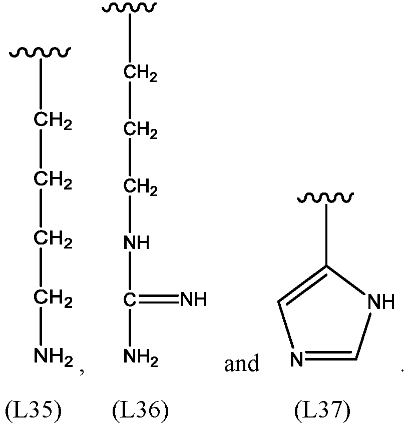

In some embodiments, the length of L^{A} is 3-35 atoms, wherein the length of the L^{A} refers to the number of chained atoms in the longest chain of atoms formed by the atom directly connected with L^{C} to the atom directly connected with R_{AP} in L^{A}. In some embodiments, each L^{A} is a connection combination of at least two of the groups of (L4) to (L9), (L13), (L14), and (L18). In some embodiments, each L^{A} is a connection combination of at least two of the groups of (L4), (L5), (L7), (L9), (L13), (L14) and (L18).

In some embodiments, L^{A} has the structure containing an amide bond as shown by Formula (302); L^{B} has the structure containing a N-acylpyrrolidine, carbonyl groups and oxygen atoms as shown by Formula (303); L^{C} is a linking group based on (hydroxymethyl)aminomethane, bis(hydroxymethyl)aminomethane, or tris(hydroxymethyl)aminomethane: 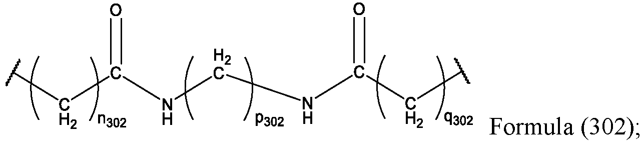 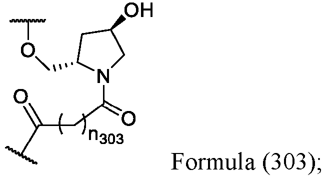 wherein n₃₀₂, q₃₀₂, and p₃₀₂ are each independently an integer of 2-6, optionally, n₃₀₂, q₃₀₂, and p₃₀₂ are each independently 2 or 3; n₃₀₃ is an integer of 4-16, optionally, n₃₀₃ is an integer of 8-12, and 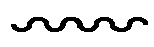 represents the site where a group is covalently linked.

In some embodiments, each of the side chain moiety L^{A} is connected to one R_{AP} group via a phosphate bond, an ether bond, or an ester bond, and is conencted to the main chain moiety L^{C} by forming an ether bond via the oxygen atom of a hydroxyl group in the main chain moiety L^{C}; the conjugated linking moiety L^{B} is connected to the nitrogen atom of the amino group in the main chain moiety L^{C} by forming an amide bond via a carbonyl group in Formula (303), and is connected to the functional group A₀ by forming a phosphate bond, an ether bond, or an ester bond via an oxygen atom in Formula (303). In some embodiments, the main chain moiety L^{C} is a linking group based on (hydroxymethyl)aminomethane, bis(hydroxymethyl)aminomethane, or tris(hydroxymethyl)aminomethane, which is connected to each of the side chain moiety L^{A} through an ether bond via the oxygen atom of a hydroxyl group, and is connected to the conjugated linking moiety L^{B} through an amide bond via the nitrogen atom of an amino group. Therefore, 1-3 side chains in the linking group Rⱼ are connected to the carbon atom of the same aminomethyl group, and are connected to R_{AP} groups containing delivery group via the conjugated linking moiety L^{B}.

In some embodiments, the conjugate has a structure as shown by Formula (305):

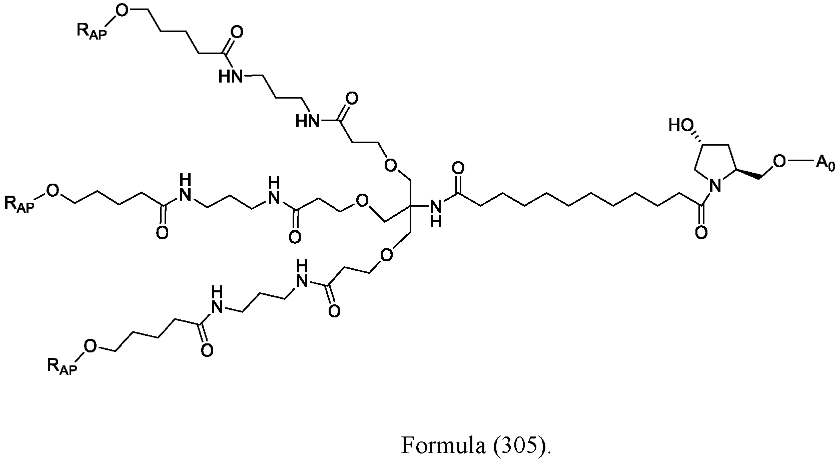

In some embodiments, the linking group Rⱼ comprises a structure as shown by Formula (306): 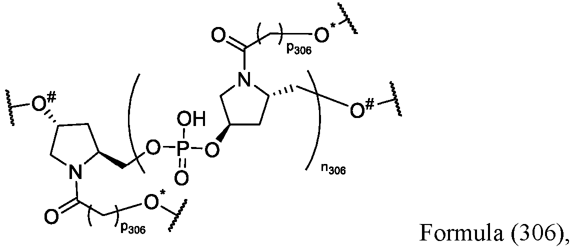 wherein n₃₀₆ is an integer of 0-3, each p₃₀₆ is independently an integer of 1-6, and 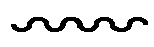 represents the site where a group is covalently linked; the connection combination formed from all pyrrolidinylene groups and any possible phosphodiester group constitutes the main chain moiety, the atomic chain between the carbonyl group attached to the nitrogen atom on a pyrrolidinylene group and an oxygen atom marked with * constitutes each side chain moiety, and the side chain moiety is connected to an R_{AP} group by forming an ether bond via the oxygen atom marked with *; at least one of the oxygen atoms marked with # is a conjugated linking moiety and is connected to a functional group A₀ by forming an ether bond, an ester bond, or a phosphate bond, and the other oxygen atoms marked with # are connected to hydrogen atoms to form hydroxyl groups, or are connected to C₁-C₃ alkyl groups to form C₁-C₃ alkoxy groups. Therefore, 1-3 side chain moieties in the linking group Rⱼ are connected to different carbon atoms in the main chain moiety, and are connected to R_{AP} groups containing delivery group via oxygen atoms.

In some embodiments, the conjugate of the present disclosure has a structure as shown by Formula (307a), (307b), or (307c):

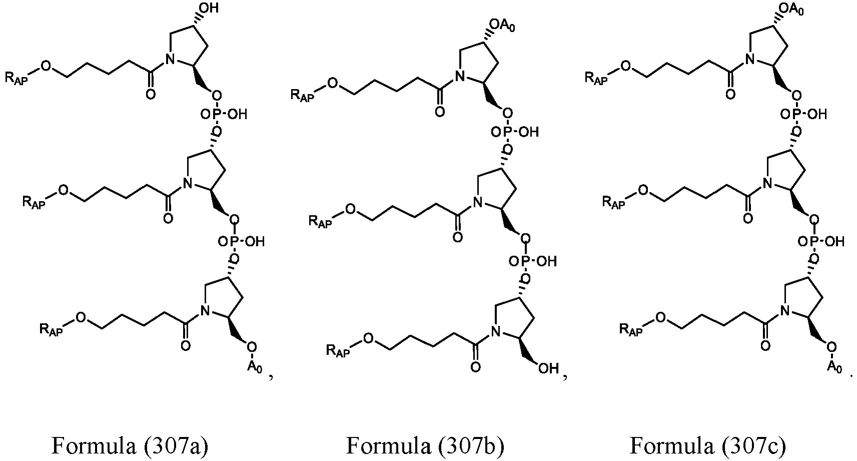

In some embodiments, the conjugate of the present disclosure has a structure as shown by Formula (308): 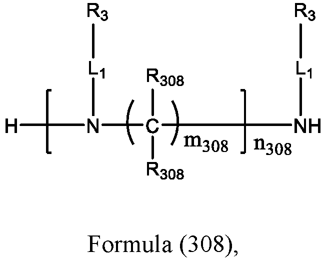 wherein n₃₀₈ may be an integer of 1-10; in some embodiments, in view of various factors such as synthesis convenience, structure/process costs, and tumor cell specificity, n₃₀₈ is an integer of 2-6. In some embodiments, n₃₀₈ is 3 or 4.

Each R₃ is independently a functional group A₀, or a R_{AP} group containing delivery group AP. In some embodiments, at least one R₃ is a functional group A₀, and at least one R₃ is R_{AP}. In some embodiments, one R₃ is a functional group A₀, and the other R₃ are R_{AP} groups.

In some embodiments, when each m₃₀₈ is independently an integer of 2-10, it is considered that in the conjugate, the space and position between multiple delivery group APs may be more suitable for the interaction with corresponding receptors on the surface of tumor cells. In order to make the compound as shown by Formula (308) have simpler structure, easy to synthesize, and/or have reduced costs, according to some embodiments of the present disclosure, each m₃₀₈ is independently an integer of 2-5; in some embodiments, each m₃₀₈ is equal.

Those skilled in the art could understand that when each R₃₀₈ is independently selected from H, C₁-C₁₀ alkyl, C₁-C₁₀ haloalkyl, and C₁-C₁₀ alkoxy, they would not change the properties of the conjugate as shown by Formula (308) and could all achieve the purpose of the present disclosure. In some embodiments, each R₃₀₈ is independently selected from H, methyl, and ethyl. In some embodiments, each R₃₀₈ is H.

Each L₁ connected to the functional group A₀ represents the conjugated linking moiety, and each L₁ connected to the R_{AP} represents the side chain moiety. In some embodiments, one R₃ is the functional group A₀, and the other R₃ are the R_{AP} groups. In some embodiments, one or more L₁, as the side chain moieties, connect R_{AP} groups to N atoms on a skeleton containing nitrogen; and the other one or more L₁, as the conjugated linking moieties, connect functional group A₀ to N atoms on the skeleton containing nitrogen. The skeleton containing nitrogen together form the main chain moiety of the linking group Rⱼ. In the context of the present disclosure, a "skeleton containing nitrogen" refers to the chain structure in the structure as shown by Formula (308), wherein the carbon atoms connected to R₃₀₈ are coadjacently connected to N atoms.

In some embodiments, considering the delivery efficiency and synthesis costs, each L₁ independently has a length of 3-25 atoms. In some embodiments, each L₁ independently has a length of 4-15 atoms. Those skilled in the art would understand that, though L₁ is defined as a linear alkylene for convenience, it may not be a linear group or may be named differently, such as an amine or alkenyl produced by the above replacement and/or substitution. For the purpose of the present disclosure, the length of L₁ is the number of atoms in the chain linking the two attachment points. For this purpose, according to the minimum number of atoms between attachment points on a ring (e.g., a heterocyclylene or heteroarylene) obtained by replacing a carbon atom in the linear alkylene, the length of the corresponding part of the ring in the chain is calculated.

In some embodiments, L₁ is selected from the group consisting of the groups as shown by the above Formulae L4-L23 and any connection combination thereof. In some embodiments, each L₁ is independently selected from the group consisting of the connection combinations of at least two of groups of L4-L9, L13, L14, and L18. In some embodiments, each L₁ is independently a connection combination of at least two of groups of L4, L5, L7, L9, L13, L14, and L18.

In some embodiments, in the conjugate as shown by Formula (308), each L₁ contains both an attachment site attached to the N atom of the nitrogen-containing skeleton and an attachment site attached to the functional group A₀ or the R_{AP} group, and the site attached to the N atom of the nitrogen-containing skeleton forms an amide bond with the N atom. In some embodiments, one or more L₁ is selected from B5, B6, B5' or B6': 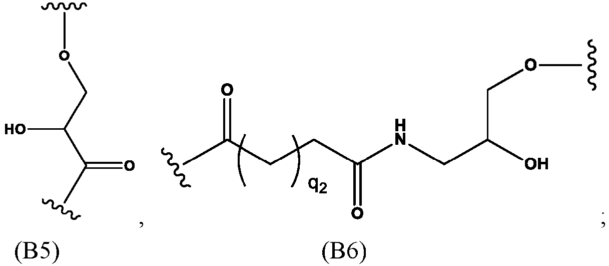 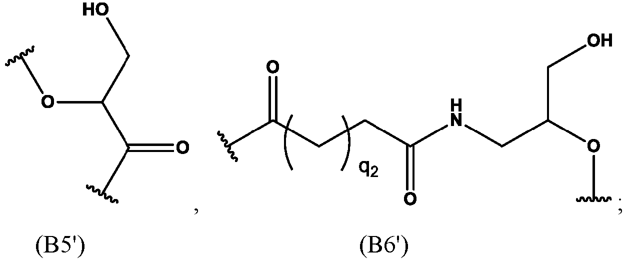 wherein 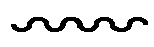 represents the site where a group is covalently linked, and q₂ is an integer of 1-10. In some embodiments, q₂ is an integer of 1-5.

In some embodiments, each R_{AP} group comprises one or more delivery groups. In some embodiments, the compound as shown by Formula (308) comprises multiple functional groups. In some embodiments, each functional group in the compound as shown by Formula (308) is the same functional group. In some embodiments, each functional group in the compound as shown by Formula (308) is a functional group for the same purpose and function. In some embodiments, the compound as shown by Formula (308) comprises various functional groups for different purposes and functions.

In some embodiments, the compound as shown by Formula (308) has a structure as shown by Formula (403), (404), (405), (406), (407), (408), (409), (410), (411), (412), (413), (414), (415), (416), (417), (418), (419), (420), (421), (422), (423), (424), (425), (426), or (427):

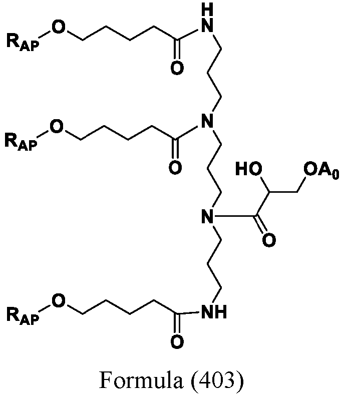

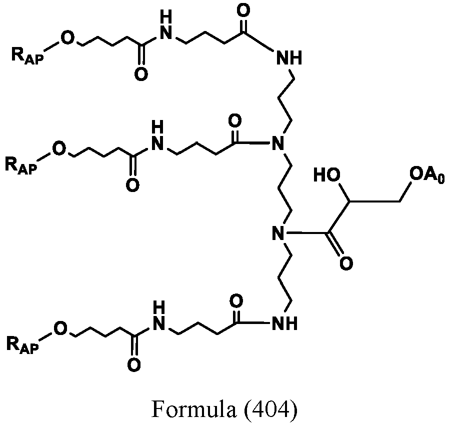

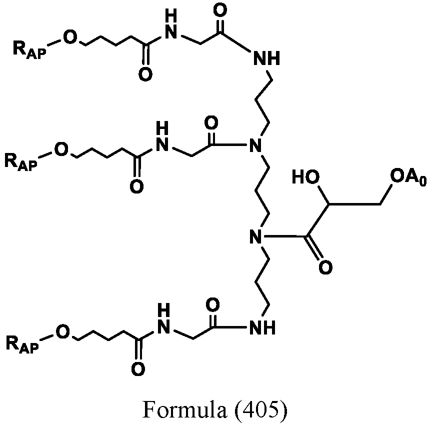

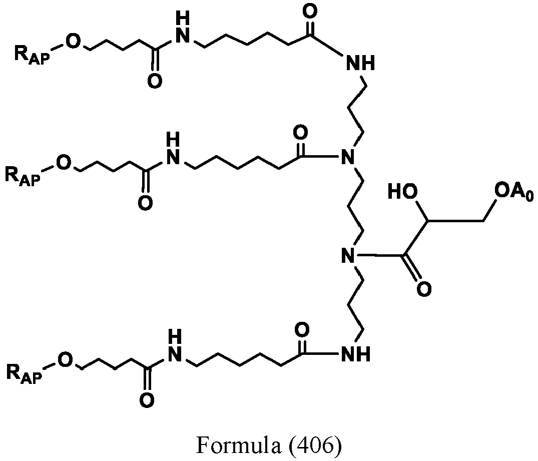

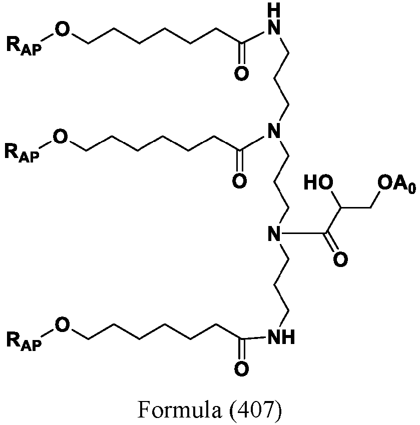

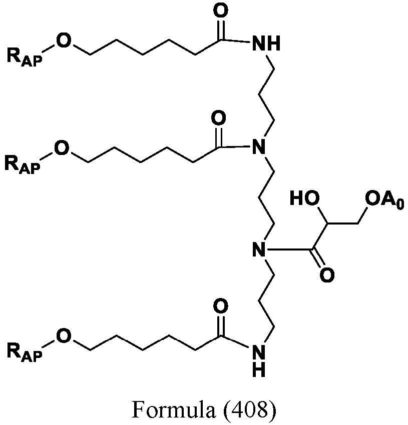

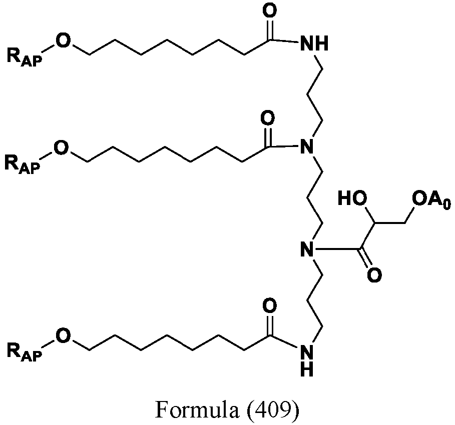

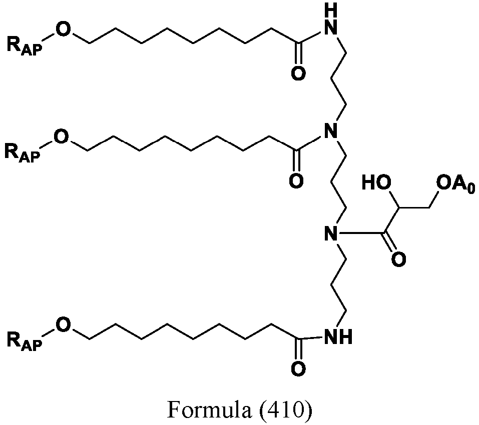

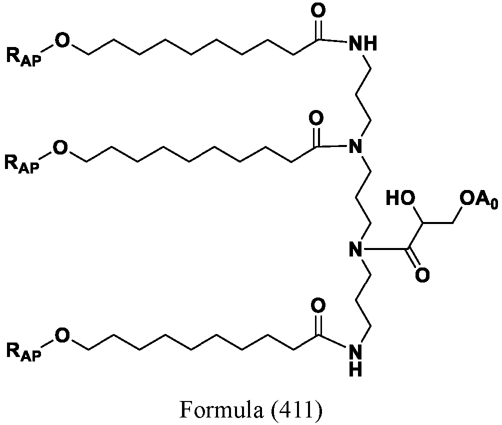

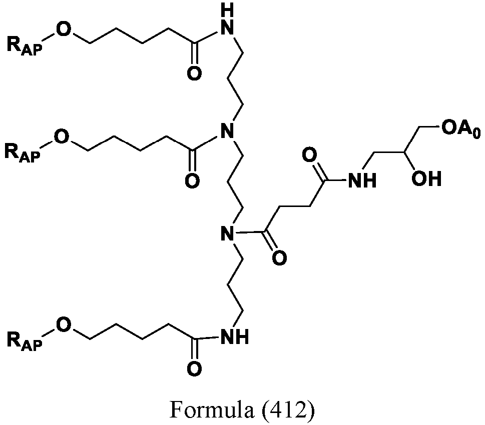

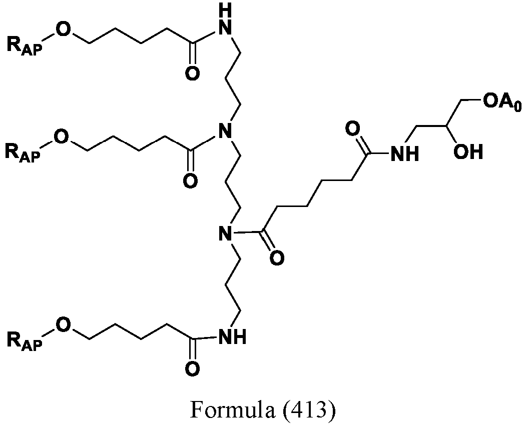

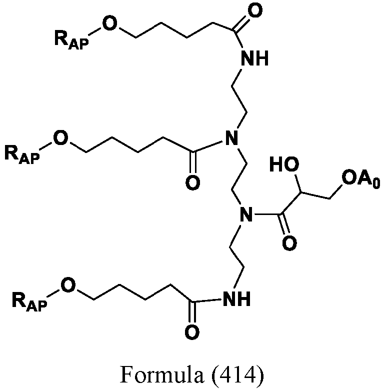

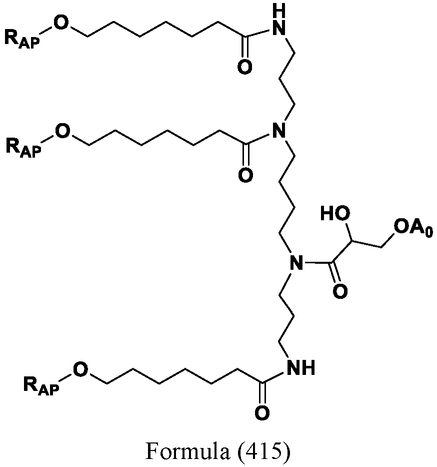

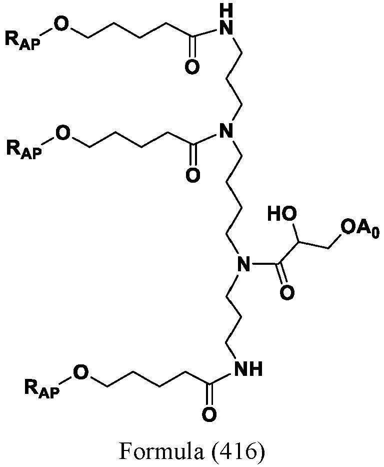

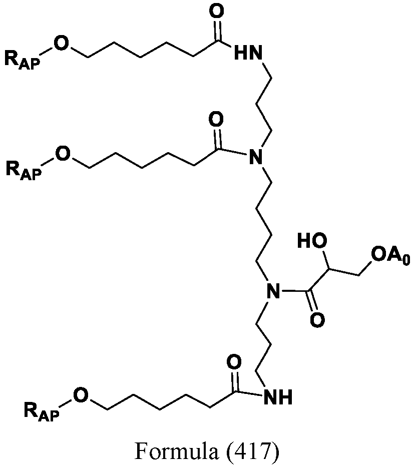

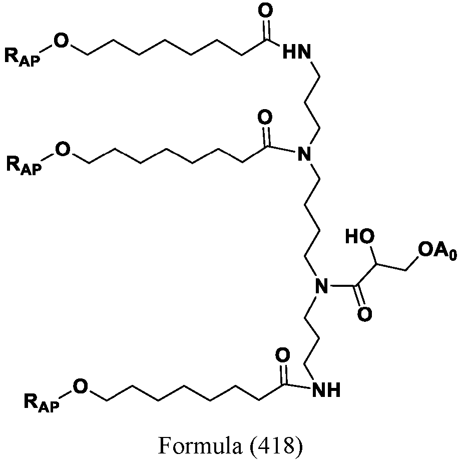

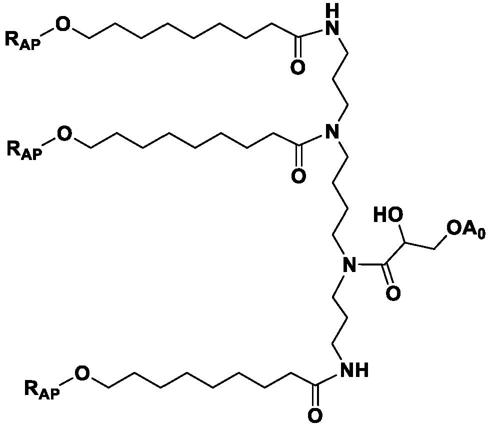

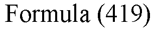

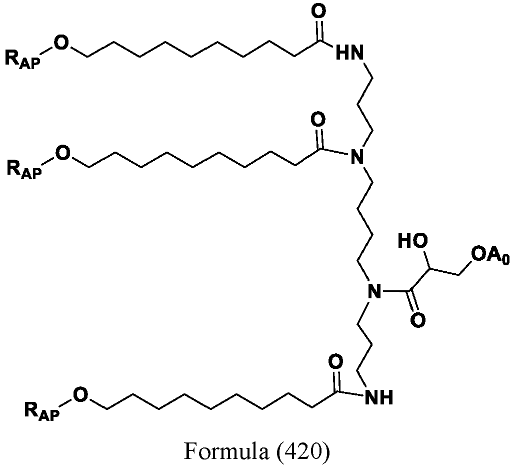

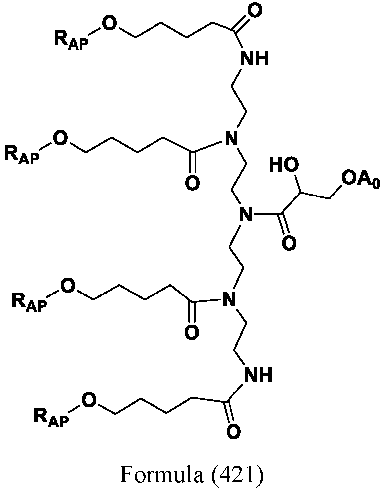

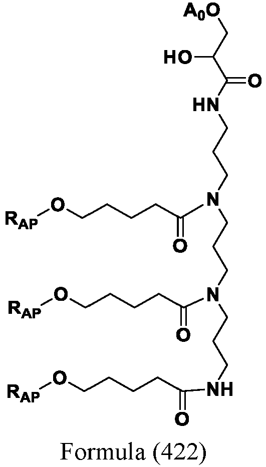

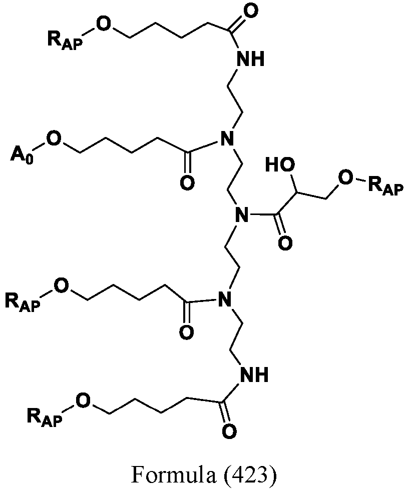

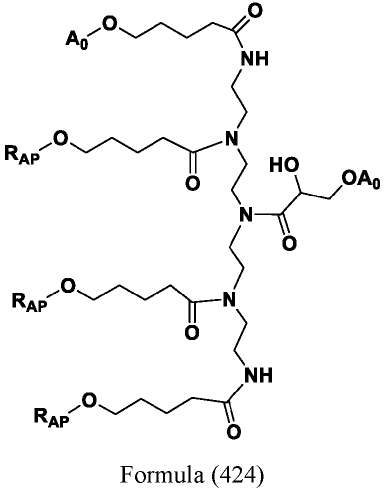

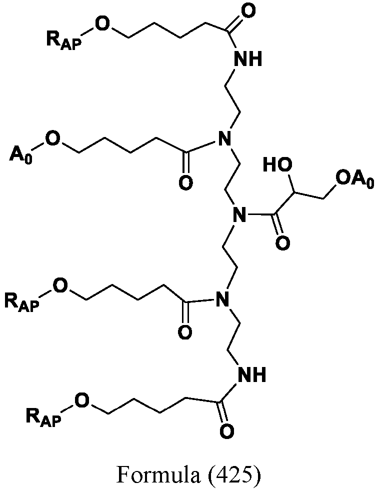

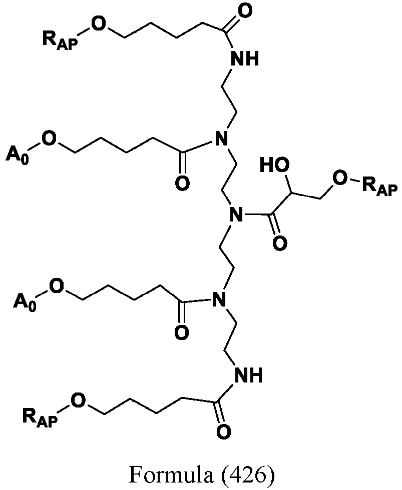

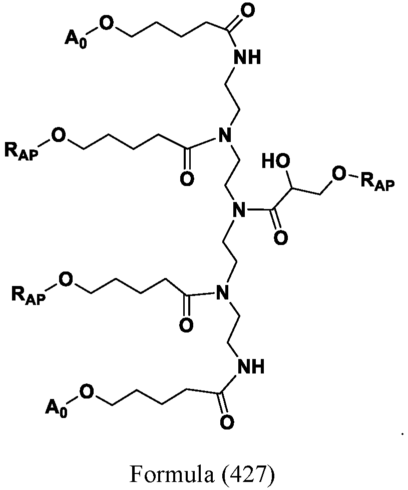

In some embodiments, the linking group Rⱼ comprises a nucleotide sequence I and a nucleotide sequence II, each of the nucleotide sequence I and the nucleotide sequence II comprises 5-25 modified or unmodified nucleotides, and the nucleotide sequence I and the nucleotide sequence II are at least partially reverse complementary, the delivery group is connected to the nucleotide sequence I, the functional group is connected to the nucleotide sequence II, and the nucleotide sequence I and the nucleotide sequence II do not elicit an immune response or toxic response in a subject. In some embodiments, the nucleotide sequence I and the nucleotide sequence II are substantially reverse complementary or completely reverse complementary; or the nucleotide sequence I and the nucleotide sequence II have the same length and are both 10-20 modified or unmodified nucleotides; or the nucleotide sequence I and the nucleotide sequence II are both consisted of 17 nucleotides and are completely reverse complementary. In some embodiments, the 3' terminus of the delivery group is connected to the 5' position of the ribose of the 5' terminal nucleotide of the nucleotide sequence I via a phosphate bond, and the functional group is connected to the 5' position of the ribose of the 5' terminal nucleotide of the nucleotide sequence II; or the functional group comprises a segment of nucleotide sequence, and the 3' terminal of the nucleotide sequence is connected to the 5' position of the ribose of the 5' terminal nucleotide of the nucleotide sequence I via a phosphate bond. In some embodiments, the nucleotide sequence I and the nucleotide sequence II have sequences as shown by SEQ ID NO: 40 and SEQ ID NO: 41, respectively:
5'-GUACAUUCUAGAUAGCC-3' (SEQ ID NO: 40)
5'-GGCUAUCUAGAAUGUAC-3' (SEQ ID NO: 41).

In some embodiments, the nucleotide sequence I and the nucleotide sequence II have sequences as shown by SEQ ID NO: 42 and SEQ ID NO: 43, respectively:
5'-GmUfAmCfAmUfUfCfUfAmGmAmUfAmGmCfCf-3' (SEQ ID NO: 42)
5'-GmGmCfUfAmUfCfUfAmGmAmAmUfGmUfAmCf-3' (SEQ ID NO: 43).

In some embodiments, the functional group is a small molecule therapeutic agent group having a therapeutic effect on tumors, and each small molecule therapeutic agent group is independently selected from a cytotoxin group, an antibiotic group, or an angiogenesis inhibitor. By comprising a small molecule therapeutic agent group, the conjugate of the present disclosure can specifically deliver the small molecule therapeutic agent group to tumors, thereby through the action of the small molecule therapeutic agent group, treating and/or alleviating disease progression or symptoms of tumors. For example, a cytotoxin group is specifically delivered to tumors by the conjugate of the present disclosure to specifically kill cancer cells in tumors, thus reducing side effects caused by low targeting ability of the cytotoxin *per se* while significantly reducing the number of cancer cells in tumors, thereby leading to treatment of tumors.

In some embodiments, the small molecule therapeutic agent group is formed by removing one or more hydrogen atoms or one or more functional radicals from a small molecule therapeutic agent below: the small molecule therapeutic agent is selected from methotrexate, doxorubicin, vinca alkaloids, auristatin (including MMAE and MMAF), calicheamicin, maytansine, and camptothecin. In some embodiments, the small molecule therapeutic agent group is a group formed by removing one or more hydrogen atoms or one or more functional radicals from monomethyl auristatin E (MMAE).

The functional group may be contained in the conjugate of the present disclosure in any suitable manner. For example, the functional group A₀ may be connected to the main chain moiety via the above conjugated linking moiety.

In some embodiments, the conjugate provided by the present disclosure further comprises one or more delivery aid groups selected from one or more of C₁₀-C₃₀ hydrocarbyl, cholesteryl, and phospholipid groups. By comprising the delivery aid group, the conjugate provided by the present disclosure can be more compatible with the internal environment in the central nervous system, may have better bioavailability, and/or is more effectively delivered to tumors. In some embodiments, the delivery aid group is connected to the delivery group or the linking group via a covalent bond or a linking group. In some embodiments, the delivery aid group is connected to the functional group.

Those skilled in the art may prepare the conjugate provided by the present disclosure using any appropriate synthetic route.

In some embodiments, the synthesis method of the conjugate provided by the present disclosure includes contacting a protected conjugate with a deprotection reagent under deprotection reaction conditions in a solvent, and isolating and obtaining the conjugate provided by the present disclosure. The protected conjugate is a compound formed by protecting all active functional groups in the conjugate provided by the present disclosure with protecting groups. In some embodiments, the active functional groups include, but are not limited to, hydroxyl, amino, and/or phosphate groups, and the protecting groups are correspondingly hydroxyl protecting groups, amino protecting groups, and/or phosphate hydroxyl protecting groups (e.g., cyanoethyl protecting groups). The solvent, deprotection reaction conditions, and deprotection reagent to be used are selected and determined according to different protecting groups. In some embodiments, the deprotection reaction conditions, solvent, and deprotection reagent are the deprotection reaction conditions, solvent, and reagent used in solid phase synthesis of nucleic acids. In some embodiments, the method includes adding the protected conjugate to a mixed solution of aqueous methylamine and aqueous ammonia, and the deprotection reaction conditions include reacting for 1-5 h under normal temperature and pressure. In some embodiments, the aqueous methylamine and saturated concentrated aqueous ammonia are mixed in equal volumes to obtain the mixed solution, the amount of the solution is 0.1-10 mL/µmol relative to the protected conjugate. In some embodiments, the isolation includes purifying through column chromatography separation, collecting product eluate, and removing the solvent. Purification conditions may be, for example, eluting with gradient eluent of aqueous sodium chloride and aqueous sodium phosphate using preparative ion chromatography purification column. In some embodiments, gradient elution is performed with: eluent A: 20 mM sodium phosphate (pH 8.1), solvent is water/acetonitrile in 9:1 (v/v); eluent B: 1.5 M sodium chloride, 20 mM sodium phosphate (pH 8.1), solvent is water/acetonitrile in 9:1 (v/v); the ratio of elution gradient: eluent A:eluent B = 100:0-50:50.

In some embodiments, the conjugate provided by the present disclosure has a structure as shown by Formula (101). The synthesis method of the protected conjugate includes contacting a compound containing an active group Rₓ₁ and a delivery group with a compound containing an active group Rₓ₂ and a functional group in an organic solvent under coupling reaction conditions, and reacting to obtain the protected conjugate, wherein the delivery group is formed by removing one or more hydrogen atoms or one or more functional radicals from the above aptamer, and each of the functional groups is independently a small molecule therapeutic agent group having a therapeutic effect on tumors, wherein any active group in the delivery group and functional group is protected by a protecting group, and the active group Rₓ₁ and the active group Rₓ₂ are groups capable of reacting to form a covalent bond or linking group Rⱼ. In some embodiments, the molar ratio of the delivery group linked with an active group Rₓ₁ to the functional group linked with an active group Rₓ₂ is m₀:n₀. In some embodiments, active groups in the delivery group and functional group include, but are not limited to, one or more of hydroxyl, amino, and phosphate groups, and the protecting groups are correspondingly one or more of hydroxyl protecting groups, amino protecting groups, and phosphate hydroxyl protecting groups (e.g., cyanoethyl protecting groups).

Those skilled in the art could obtain the compound containing an active group Rₓ₁ and a delivery group by various methods. In some embodiments, the compound containing an active group Rₓ₁ and a delivery group may be obtained by nucleic acid synthesis methods well-known to those skilled in the art, for example, phosphoramidite solid phase synthesis or phosphodiester method/phosphotriester method liquid phase synthesis. In some embodiments, the compound containing an active group Rₓ₁ and a delivery group is obtained using phosphoramidite solid phase synthesis method. The method includes sequentially linking nucleoside monomers according to the order of the nucleotides in an oligonucleotide single strand under phosphoramidite solid phase synthesis conditions, wherein at least one nucleoside monomer is a nucleoside monomer having an active group Rₓ₁; or after linking all nucleoside monomers, linking a phosphoramidite monomer or protected phosphoramidite monomer having an active group Rₓ₁ according to the phosphoramidite solid phase synthesis method, and then removing the protecting group to form the active group Rₓ₁. The phosphoramidite solid phase synthesis method is well-known to those skilled in the art, whose process and conditions are disclosed in detail in Methods in Molecular Biology, vol. 288: Oligonucleotide Synthesis: Methods and Applications, P17-P31, which is incorporated herein by reference in its entirety.

In some embodiments, the coupling reaction conditions are condensation reaction conditions or thiol-disulfide exchange reaction conditions.

In some embodiments, the coupling reaction conditions are condensation reaction conditions, which are acylation condensation reaction conditions, dehydration condensation reaction conditions, or click chemistry reaction conditions, and the active group Rₓ₁ and active group Rₓ₂ are groups capable of undergoing the above condensation reaction. In some embodiments, the condensation reaction conditions are acylation condensation reaction conditions, and the active groups Rₓ₁ and Rₓ₂ are groups capable of undergoing acylation condensation reaction to form R₁. In some embodiments, the condensation reaction conditions are dehydration condensation reaction conditions, and one of the active groups Rₓ₁ and Rₓ₂ is a group containing an acyl halide or carboxyl group, and the other is a group containing an amino or hydroxyl group. In some embodiments, the condensation reaction conditions are click chemistry reaction conditions, and one of the active groups Rₓ₁ and Rₓ₂ is a group containing an alkynyl group, and the other is a group containing an azide group. In some embodiments, the condensation reaction conditions are Michael addition reaction conditions, and one of the active groups Rₓ₁ and Rₓ₂ is a group containing a sulfhydryl group, and the other is a group containing a succinimido group. In some embodiments, the condensation reaction conditions are N-hydroxysuccinimide-carbodiimide (NHS-EDC) coupling reaction conditions, and one of the active groups Rₓ₁ and Rₓ₂ is a group containing N-hydroxysuccinimide (NHS), and the other is a group containing a carbodiimide group (EDC).

In some embodiments, the compound containing an active group Rₓ₁ and a delivery group is prepared by contacting an aptamer having an active group Rₓ₀ with a cross-linking agent under coupling reaction conditions, and the cross-linking agent contains a click chemistry active group and an acylation group. The active group Rₓ₀ and the acylation group are covalently linked through coupling reaction, allowing the click chemistry active group link to the aptamer of the present disclosure.

In some embodiments, an active group Rₓ₁ is an active group having 1-3 click chemistry active groups at its end, which contain a terminal alkynyl group. In some embodiments, the acylation group is an active ester group, which may be, for example, one of an NHS ester group, imidate group, and pentafluorophenyl ester group. Those skilled in the art could obtain the cross-linking agent by various methods. For example, when the acylation group is a pentafluorophenyl ester group and the click chemistry group contains a terminal alkynyl group, the cross-linking agent may be prepared according to the method as described in Scheme 1a (A) in Østergaard, Michael E., et al. "Efficient synthesis and biological evaluation of 5'-GalNAc conjugated antisense oligonucleotides." Bioconjugate chemistry 26.8 (2015): 1451-1455, which is incorporated herein by reference in its entirety. In some embodiments, the active group Rₓ₀ is an amino group. In some embodiments, the coupling conditions are basic conditions. In some embodiments, the basic conditions are conditions wherein an aqueous weak base is present, for example conditions wherein aqueous sodium bicarbonate is present.

Those skilled in the art could obtain the aptamer having an active group Rₓ₀ by various methods. In some embodiments, the aptamer having an active group Rₓ₀ is prepared by using at the corresponding position a phosphoramidite monomer having an active group during aptamer synthesis. Those skilled in the art could obtain a phosphoramidite monomer having an active group by various methods. In some embodiments, the active group Rₓ₀ is an amino group, and a phosphoramidite monomer having an Rₓ₀ can be commercially available or prepared by methods well-known to those skilled in the art. For example, the phosphoramidite monomer having an Rₓ₀ can be a readily commercially available 6-(trifluoroacetylamino)-hexyl (2-cyanoethyl)-(*N,N*-diisopropyl)-phosphoramidite monomer, wherein the active group Rₓ₀ is an amino group. The active group Rₓ₀ may be obtained by linking the phosphoramidite monomer to an oligonucleotide single strand by the phosphoramidite solid phase synthesis method, and then removing the trifluoroacetyl protecting group through deprotection reaction readily realized by those skilled in the art (e.g., concentrated aqueous ammonia ammonolysis).

In some embodiments, the coupling reaction conditions are one of thiol-disulfide exchange reaction conditions, and one of the active groups Rₓ₁ and Rₓ₂ is a group containing a sulfhydryl group, and the other contains a leaving group linked by a disulfide bond. In some embodiments, in order to avoid side reactions, Rₓ₁ in the above phosphoramidite monomer having an active group Rₓ₁ is present in a protected form of Rₓ₁', and the preparation method further includes a step of isolating and obtaining the compound containing an active group Rₓ₁ and a delivery group through contacting the compound prepared containing the protected active group Rₓ₁' and a delivery group with a deprotection reagent under deprotection reaction conditions. In some embodiments, the Rₓ₁' contains a disulfide bond leaving group, the deprotection reaction conditions are thiol-disulfide exchange reaction conditions, and the deprotection reagent is a disulfide bond activator. In some embodiments, the disulfide bond activator is dithiodipyridine. Those skilled in the art could obtain the above phosphoramidite monomer having an active group Rₓ₁ or Rₓ₁' by various methods. In some embodiments, the phosphoramidite monomer having an active group Rₓ₁ or Rₓ₁' can be commercially available. For example, a phosphoramidite monomer as shown by Formula (105) is commercially available. 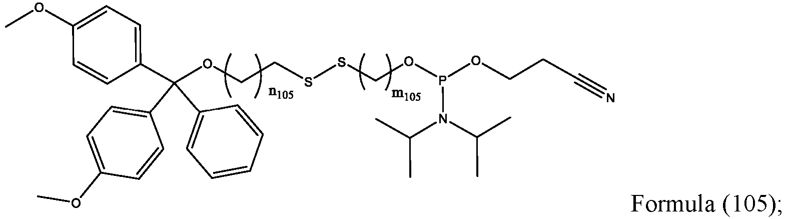 wherein n₁₀₅ and m₁₀₅ are each independently an integer of 1-10.

Those skilled in the art could obtain the compound containing an active group Rₓ₂ and a functional group by various methods. In some embodiments, the coupling reaction conditions are thiol-disulfide exchange reaction conditions, the active group Rₓ₂ contains a sulfhydryl group, and the compound containing an active group Rₓ₂ and a functional group may be obtained by those skilled in the art via various known methods. For example, it may be prepared by the phosphoramidite solid phase synthesis method using a phosphoramidite monomer containing a sulfhydryl group, or may be commercially available. In some embodiments, the coupling reaction conditions are phosphoramidite solid phase synthesis reaction conditions, the active group Rₓ₂ is a phosphoramidite group, and the compound containing an active group Rₓ₂ and a functional group may be, for example, a readily commercially available compound containing a phosphoramidite group and a small molecule therapeutic agent group. In some embodiments, the coupling reaction conditions are Michael addition reaction conditions, the active group Rₓ₂ is a *N*-succinimido group, and the compound containing an active group Rₓ₂ and a functional group may be, for example, a readily commercially available compound containing a *N*-succinimido group and a small molecule therapeutic agent group. In some embodiments, the active group Rₓ₁ and active group Rₓ₂ are respectively a nucleotide sequence I and a nucleotide sequence II, each of the nucleotide sequence I and the nucleotide sequence II comprises 5-25 modified or unmodified nucleotides, and the nucleotide sequence I and the nucleotide sequence II are at least partly reverse complementary. The delivery group is linked to the nucleotide sequence I, the functional group is connected to the nucleotide sequence II, and the nucleotide sequence I and the nucleotide sequence II do not elicit immune response or toxic reaction in a subject. The coupling reaction conditions are reaction conditions for annealing to form a nucleic acid double strand. In some embodiments, the nucleotide sequence I and the nucleotide sequence II are both consisted of 17 nucleotides and are completely reverse complementary. In some embodiments, the nucleotide sequence I and the nucleotide sequence II have sequences as shown by SEQ ID NO: 40 and SEQ ID NO: 41, respectively. In some embodiments, the nucleotide sequence I and the nucleotide sequence II have sequences as shown by SEQ ID NO: 42 and SEQ ID NO: 43, respectively.

A delivery group is formed by removing one or more hydrogen atoms or functional groups from an aptamer. In some embodiments, the group at position 5' of the ribose in the 5'-terminal nucleotide of the continuous nucleotide sequence and the group at position 3' of the ribose in the 3'-terminal nucleotide of the continuous nucleotide sequence are both hydroxyl groups, and a delivery group is formed by removing one hydrogen atom from the 5'-hydroxyl group of the 5'-terminal nucleotide of the aptamer; in some embodiments, a delivery group is formed by removing one hydrogen atom from the 3'-hydroxyl group of the 3'-terminal nucleotide of the aptamer; in some embodiments, a delivery group is formed by removing the 5'-hydroxyl group from the 5'-terminal nucleotide of the aptamer; in some embodiments, a delivery group is formed by removing the 3'-hydroxyl group from the 3'-terminal nucleotide of the aptamer. In some embodiments, a delivery group is formed by removing the 2'-hydroxyl group from the ribose in a nucleotide contained in an aptamer. The aptamer may be obtained by conventional aptamer preparation methods in the art (e.g., methods of solid phase synthesis and liquid phase synthesis of nucleic acids). Therein, there has been commercial customized service of solid phase synthesis of nucleic acids. A modified nucleotide group can be introduced into the conjugate provided by the present disclosure by using a nucleoside monomer with a corresponding modification. The methods for preparing a nucleoside monomer with a corresponding modification and the methods for introducing a modified nucleotide group into an aptamer are also well-known to those skilled in the art. All nucleoside monomers with modifications can be commercially available or prepared by known methods.

In some embodiments, the conjugate may also be used in the present disclosure in the form of a pharmaceutically acceptable salt or precursor compound thereof. In the context of the present disclosure, a "pharmaceutically acceptable salt" refers to a corresponding salt formed from a drug to increase the stability, solubility, and/or bioavailability of the drug without pharmaceutically causing additional side effects on the human body, such as a potassium salt, sodium salt, or carboxylate. A "precursor compound" refers to a compound that, although not identical as such in structure and function to the conjugate, can react and form the conjugate of the present disclosure after entering the body or in the body fluid environment, thereby exerting its effect and achieving the purpose of the present disclosure. Under certain circumstances, these precursor compounds can increase the stability, extend release time, increase bioavailability, etc. of the drug. In some embodiments, the precursor compound contains precursor groups capable of reacting in the human body to form all functional group A₀ in a conjugate. In some embodiments, the precursor compound includes a compound formed by substituting all active hydroxyl groups in a conjugate with acetoxy groups. In some embodiments, the precursor compound contains a drug precursor group, which is a residue formed from a precursor compound of the therapeutic agent corresponding to the functional group in a conjugate. In some embodiments, the drug precursor group may be, for example, a group formed by substituting active hydrogen in a hydroxyl or amino functional group in the functional group with an acyl, alkyl, or phosphoryl group. Those skilled in the art could understand that use of these pharmaceutically acceptable salts and precursor compounds are also within the scope of the present disclosure.

### Pharmaceutical composition

In one aspect, the present disclosure further provides a pharmaceutical composition comprising the conjugate provided by the present disclosure and a pharmaceutically acceptable carrier.

The pharmaceutically acceptable carrier may be a carrier conventionally used in the art, for example, but not limited to, one or more of water, normal saline, magnetic nanoparticles (such as Fe₃O₄-based or Fe₂O₃-based nanoparticles), carbon nanotubes, mesoporous silicon, calcium phosphate nanoparticles, polyethylenimine (PEI), polyamidoamine (PAMAM) dendrimer, poly(L-lysine) (PLL), chitosan, 1,2-dioleoyl-3-trimethylammonium-propane (DOTAP), poly(D&L-lactic/glycolic acid) copolymer (PLGA), poly(2-aminoethyl ethylene phosphate) (PPEEA), poly(2-dimethylaminoethyl methacrylate) (PDMAEMA), and derivatives thereof.

In some embodiments, the pharmaceutically acceptable carrier contains a physiologically acceptable compound, which exerts the function of, for example, stabilizing the pharmaceutical composition or increasing or reducing the absorption of the conjugate and/or pharmaceutical composition. The physiologically acceptable compound is selected from one or more of the following compounds: carbohydrates, such as glucose, sucrose, and/or glucan; antioxidants, such as ascorbic acid and/or glutathione; chelating agents; low molecular weight proteins; compositions that reduce the clearance or hydrolysis of any co-administered substance; excipients; stabilizers and buffers. A detergent may also be used for stabilizing the composition or increasing or reducing the absorption of the pharmaceutical composition. The physiologically acceptable compound may further include one or more of wetting agents, emulsifiers, dispersants, and preservatives used specifically for preventing microbial growth or action. The physiologically acceptable compound is known to those skilled in the art, which is not described herein in detail. Those skilled in the art could easily understand that the choices of the pharmaceutically acceptable carrier and physiologically acceptable compound depend on, for example, administration routes and specific physiochemical characteristics of any co-administered substance.

In some embodiments, the pharmaceutically acceptable carrier is sterile and usually does not contain an undesirable substance. The pharmaceutical composition provided by the present disclosure may further comprise medicinal auxiliary substances according to the actual need to approach physiological conditions, such as pH regulators and buffers and toxicity regulators, for example, sodium acetate, sodium chloride, potassium chloride, calcium chloride, and sodium lactate. The concentration of the conjugate provided by the present disclosure in the pharmaceutical composition may vary within a wide range, and is selected mainly depending upon the fluid volume, viscosity, body weight, etc. according to the specific administration method.

In some embodiments, in the pharmaceutical composition, there are no special requirements for the contents of the conjugate and the pharmaceutically acceptable carrier. In some embodiments, the weight ratio of the conjugate to the pharmaceutically acceptable carrier may be 1:(1-500), and in some embodiments, the above weight ratio is 1:(1-50).

In some embodiments, the pharmaceutical composition may also comprise other pharmaceutically acceptable excipients, which may be one or more of various formulations and compounds conventionally employed in the art. For example, the other pharmaceutically acceptable excipients may comprise at least one of a pH buffer, a protective agent, and an osmotic pressure regulator.

The pH buffer may be a tris(hydroxymethyl)aminomethane hydrochloride buffer solution with a pH of 7.5-8.5, and/or a phosphate buffer solution with a pH of 5.5-8.5, such as a phosphate buffer solution with a pH of 5.5-8.5.

The protective agent may be at least one of inositol, sorbitol, sucrose, trehalose, mannose, maltose, lactose, and glucose. The content of the protective agent may be from 0.01 wt% to 30 wt% based on the total weight of the pharmaceutical composition.

The osmotic pressure regulator may be sodium chloride and/or potassium chloride. The content of the osmotic pressure regulator allows the osmotic pressure of the pharmaceutical composition to be 200-700 mOsm/kg. Depending on the desired osmotic pressure, those skilled in the art can readily determine the content of the osmotic pressure regulator. In some embodiments, the dose of the formulation prepared from the pharmaceutical composition will be adjusted due to different administration manners during administration.

In some embodiments, the pharmaceutical composition may be a liquid formulation, for example, an injection solution; or a lyophilized powder for injection, which will be mixed with a liquid excipient to form a liquid formulation upon administration. The liquid formulation may be used for, but not limited to, administration by subcutaneous, intramuscular, or intravenous injection, and the pharmaceutical composition may also be delivered by, but not limited to, puncture injection, oropharyngeal inhalation, nasal administration, or other routes. In some embodiments, the pharmaceutical composition is used for administration by subcutaneous, intramuscular, intravenous, or intrathecal injection.

In some embodiments, the pharmaceutical composition may be in the form of a liposome formulation. In some embodiments, the pharmaceutically acceptable carrier used in the liposome formulation comprises an amine-containing transfection compound (hereinafter also referred to as an organic amine), a helper lipid, and/or a PEGylated lipid. Therein, the organic amine, the helper lipid, and the PEGylated lipid may be respectively selected from one or more of the amine-containing transfection compounds or the pharmaceutically acceptable salts or derivatives thereof, the helper lipids, and the PEGylated lipids as described in the Chinese patent application CN103380113A, which is incorporated herein by reference in its entirety.

In some embodiments, the organic amine may be a compound as shown by Formula (201) or a pharmaceutically acceptable salt thereof as described in the Chinese patent application CN103380113A: 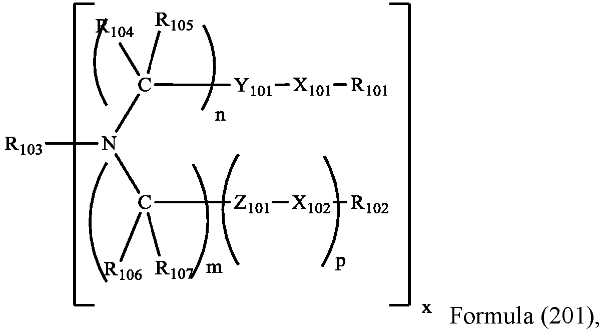 wherein:
X₁₀₁ and X₁₀₂ are each independently O, S, N-A or C-A, wherein A is hydrogen or a C₁-C₂₀ hydrocarbon chain;
Y₁₀₁ and Z₁₀₁ are each independently C=O, C=S, S=O, CH-OH or SO₂;
R₁₀₁, R₁₀₂, R₁₀₃, R₁₀₄, R₁₀₅, R₁₀₆, and R₁₀₇ are each independently hydrogen; a cyclic or an acyclic, substituted or unsubstituted, branched or linear aliphatic group; a cyclic or an acyclic, substituted or unsubstituted, branched or linear heteroaliphatic group; a substituted or unsubstituted, branched or linear acyl group; a substituted or unsubstituted, branched or linear aryl group; or a substituted or unsubstituted, branched or linear heteroaryl group;
x is an integer of 1-10;
n is an integer of 1-3, m is an integer of 0-20, and p is 0 or 1, wherein if m = p = 0, then R₁₀₂ is hydrogen; and
if at least one of n and m is 2, then R₁₀₃ and the nitrogen in Formula (201) form a structure as shown by Formula (202) or (203): 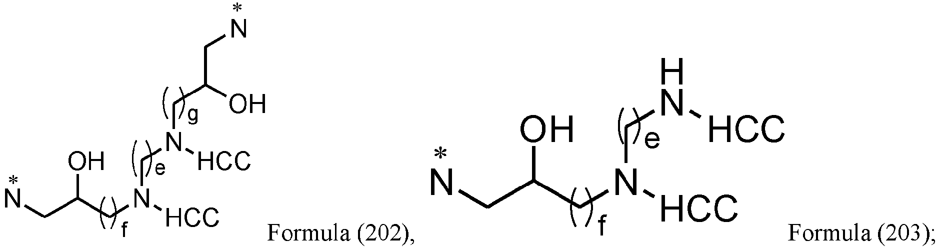
wherein g, e, and f are each independently an integer of 1-6, "HCC" represents a hydrocarbon chain, and each *N represents a nitrogen atom in Formula (201).

In some embodiments, R₁₀₃ is a polyamine. In other embodiments, R₁₀₃ is a ketal. In some embodiments, R₁₀₁ and R₁₀₂ in Formula (201) are each independently any substituted or unsubstituted, branched or linear alkyl or alkenyl which has 3 to about 20 carbon atoms (such as 8 to about 18 carbon atoms) and 0-4 double bonds (such as 0-2 double bonds).

In some embodiments, if n and m are each independently 1 or 3, R₁₀₃ may be any of the following Formulae (204)-(213): 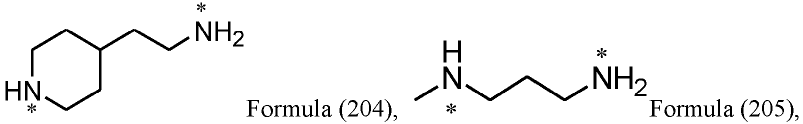 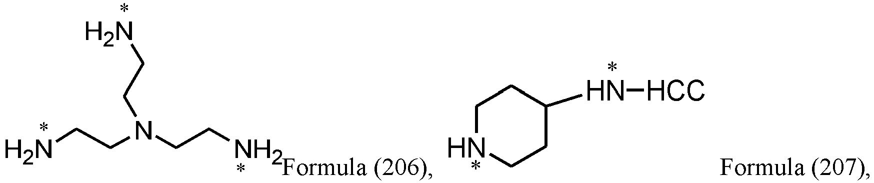 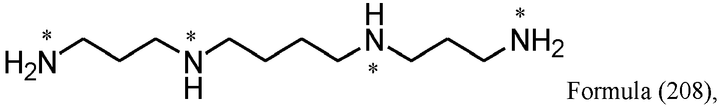 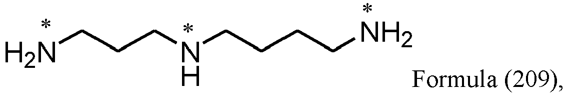 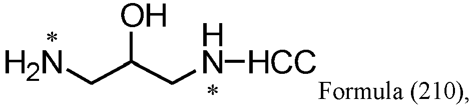 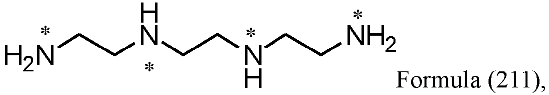 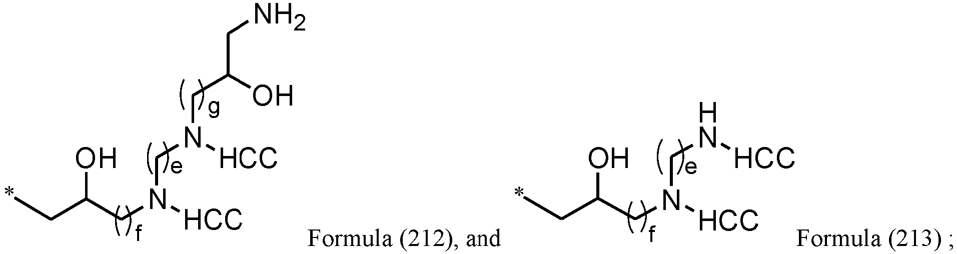 wherein, in Formulae (204)-(213), g, e, and f are each independently an integer of 1-6, each "HCC" represents a hydrocarbon chain, and each * represents a potential attachment point of R₁₀₃ to the nitrogen atom in Formula (201), wherein each H at any * position may be replaced to achieve the attachment to the nitrogen atom in Formula (201).

Those skilled in the art could obtain the compound as shown by Formula (201) by any appropriate method. In some embodiments, the compound as shown by Formula (201) may be prepared according to the description of the Chinese patent application CN103380113A.

In some embodiments, the organic amine is an organic amine as shown by Formula (214) and/or an organic amine as shown by Formula (215): 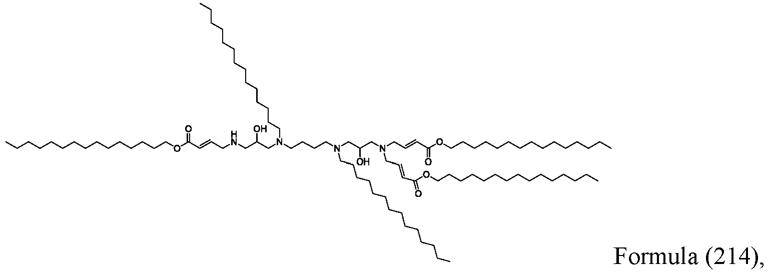 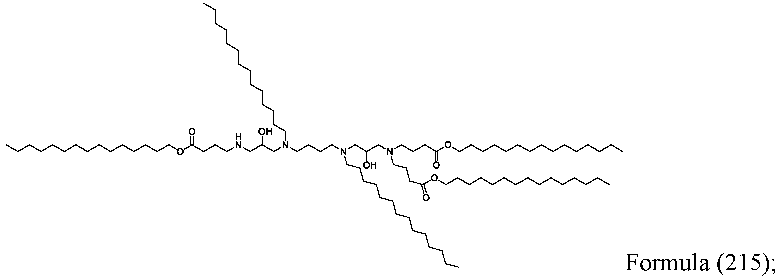
the helper lipid is cholesterol, a cholesterol analogue, and/or a cholesterol derivative; and
the PEGylated lipid is 1,2-dipalmitoyl-sn-glycero-3-phosphoethanolamine-*N*-[methoxy(polyethylene glycol)-2000].

In some embodiments, the molar ratio between the organic amine, the helper lipid, and the PEGylated lipid in the pharmaceutical composition is (19.7-80):(19.7-80):(0.3-50); for example, the molar ratio may be (50-70):(20-40):(3-20).

In some embodiments, the pharmaceutical composition particles formed from the conjugate provided by the present disclosure and the above amine-containing transfection reagents have an average diameter of about 30 nm to about 200 nm, typically about 40 nm to about 135 nm, and more typically, the average diameter of the liposome particles is about 50 nm to about 120 nm, about 50 nm to about 100 nm, about 60 nm to about 90 nm, or about 70 nm to about 90 nm; for example, the average diameter of the liposome particles is about 30, 40, 50, 60, 70, 75, 80, 85, 90, 100, 110, 120, 130, 140, 150, or 160 nm.

In some embodiments, in the pharmaceutical composition formed from the conjugate provided by the present disclosure and the above amine-containing transfection reagents, the weight ratio (weight/weight ratio) of the conjugate to total lipids (e.g., the organic amines, the helper lipids, and/or the PEGylated lipids), ranges from about 1:1 to about 1:50, from about 1:1 to about 1:30, from about 1:3 to about 1:20, from about 1:4 to about 1:18, from about 1:5 to about 1:17, from about 1:5 to about 1:15, from about 1:5 to about 1:12, from about 1:6 to about 1:12, or from about 1:6 to about 1:10. For example, the weight ratio of the conjugate provided by the present disclosure to total lipids is about 1:5, 1:6, 1:7, 1:8, 1:9, 1:10, 1:11, 1:12, 1:13, 1:14, 1:15, 1:16, 1:17, or 1:18.

In some embodiments, the pharmaceutical composition may be marketed with each component being separate, and used in the form of a liquid formulation. In some embodiments, the pharmaceutical composition formed from the conjugate provided by the present disclosure and the above pharmaceutically acceptable carrier may be prepared by various known processes, through merely replacing the existing aptamer or conjugate with the conjugate provided by the present disclosure. In some embodiments, the pharmaceutical composition may be prepared according to the following process:
The organic amines, helper lipids, and PEGylated lipids are suspended in alcohol at a molar ratio as described above and mixed homogeneously to obtain a lipid solution; the alcohol is used in an amount such that the resulting lipid solution is present at a total mass concentration of 2 to 25 mg/mL (e.g., 8 to 18 mg/mL). The alcohol is a pharmaceutically acceptable alcohol, such as an alcohol that is in liquid form at about room temperature, for example, one or more of ethanol, propylene glycol, benzyl alcohol, glycerol, polyethylene glycol 200, polyethylene glycol 300, and polyethylene glycol 400, such as ethanol.

The conjugate provided by the present disclosure is dissolved in a buffer salt solution to obtain an aqueous solution of the conjugate. The buffer salt solution has a concentration of 0.05 to 0.5 M, such as 0.1 to 0.2 M. The pH of the buffer salt solution is adjusted to 4.0 to 5.5, such as 5.0 to 5.2. The buffer salt solution is used in an amount such that the conjugate is present at a concentration of no more than 0.6 mg/mL, such as 0.2 to 0.4 mg/mL. The buffer salt may be one or more selected from the group consisting of soluble acetates and soluble citrates, such as sodium acetate and/or potassium acetate.

The lipid solution and the aqueous solution of the conjugate are mixed. The product obtained by mixing is incubated at a temperature of 40 to 60°C for at least 2 minutes (e.g., 5 to 30 minutes) to produce an incubated liposome formulation. The volume ratio of the lipid solution to the aqueous solution of the conjugate is 1:(2-5) (such as 1:4).

The incubated liposome formulation is concentrated or diluted, and then subjected to impurity removal and sterilization to obtain the pharmaceutical composition provided by the present disclosure, which has the following physicochemical parameters: a pH of 6.5 to 8, an encapsulation efficiency of not lower than 80%, a particle size of 40 to 200 nm, a polydispersity index of no greater than 0.30, and an osmotic pressure of 250 to 400 mOsm/kg. For example, the physicochemical parameters may be as follows: a pH of 7.2 to 7.6, an encapsulation efficiency of not lower than 90%, a particle size of 60 to 100 nm, a polydispersity index of no greater than 0.20, and an osmotic pressure of 300 to 400 mOsm/kg.

Therein, the concentration or dilution step may be performed before, after, or simultaneously with removal of the impurities. The method for removing impurities may be any of various existing methods, for example, ultrafiltration is performed using a tangential flow system or a hollow fiber column under the condition of 100 kDa, with a phosphate buffer (PBS) at pH 7.4 as ultrafiltration exchange solution. The method for sterilization may be any of various existing methods, such as filtration sterilization on a 0.22 µm filter.

### Use of the conjugate of the present disclosure

In another aspect, the present disclosure further provides the use of the conjugate and/or the pharmaceutical composition provided by the present disclosure in the manufacture of a medicament for treating tumors and tumor-related diseases or symptoms.

In another aspect, the present disclosure further provides a method for treating tumors and tumor-related diseases or symptoms, comprising administering the conjugate and/or the pharmaceutical composition of the present disclosure to a subject in need thereof.

By administering the conjugate and/or the pharmaceutical composition of the present disclosure, the method of the present disclosure can effectively treat tumors and tumor-related diseases or symptoms; and with the highly specific targeting effect of the conjugate provided by the present disclosure, the therapeutic agent distributed in other undesired organs/tissues of the body can be reduced, thereby reducing potential side effects, which is of great importance and value especially for radiotherapy and/or chemotherapy drugs commonly used in the field of tumor treatment and known for significant side effects.

As used herein, the term "administration/administer" refers to placing the conjugate and/or the pharmaceutical composition into a subject by a method or route where the conjugate and/or the pharmaceutical composition is at least in part located at a desired site to achieve a desired effect. The administration routes suitable for the method of the present disclosure include topical administration and systemic administration. In general, topical administration results in the delivery of more of the conjugate and/or the pharmaceutical composition to a particular site as compared with the whole body of the subject; whereas systemic administration results in the delivery of the conjugate and/or the pharmaceutical composition to substantially the whole body of the subject.

Furthermore, the inventors of the present disclosure surprisingly found that the conjugate and/or the pharmaceutical composition of the present disclosure can pass through the blood-brain barrier efficiently and target tumors in the brain under systemic administration, thus further improving the delivery efficiency of the functional group and reducing costs and undesired side effects.

The administration to a subject can be achieved by any suitable route known in the art, including but not limited to, oral or parenteral routes, such as intravenous administration, intramuscular administration, subcutaneous administration, transdermal administration, intratracheal administration (aerosol), pulmonary administration, nasal administration, rectal administration, and topical administration (including buccal administration and sublingual administration). The administration frequency may be once or more times daily, weekly, biweekly, triweekly, monthly, or yearly.

The dose of the conjugate and/or the pharmaceutical composition of the present disclosure can be a conventional dose in the art, which may be determined according to various parameters, especially age, weight, and gender of a subject. Toxicity and efficacy may be determined in cell cultures or experimental animals by standard pharmaceutical procedures, for example, by determining LD50 (the dose that causes 50% population death) and ED50 (the dose that can cause 50% of the maximum response intensity in a quantitative response, or that causes 50% of the experimental subjects to have a positive response in a qualitative response). The dose range for human use may be derived based on the data obtained from cell culture analysis and animal studies.

When the conjugate and/or the pharmaceutical composition of the present disclosure is administered, for example, to male or female C57BL/6J or C3H/HeNCrlVr mice with an age of 6 to 12 weeks and a body weight of 18 to 25 g, calculated based on the amount of the conjugate and/or the conjugate in the pharmaceutical composition: the dosage of the conjugate may be 0.001 to 100 mg/kg body weight, in some embodiments is 0.01 to 50 mg/kg body weight, in further embodiments is 0.05 to 20 mg/kg body weight, in even further embodiments is 0.1 to 15 mg/kg body weight, and in still further embodiments is 0.1 to 10 mg/kg body weight. When the conjugate and/or the pharmaceutical composition of the present disclosure is administered, the above dosages may be preferred.

### Kit

The present disclosure provides a kit comprising the conjugate and/or the pharmaceutical composition of the present disclosure.

In some embodiments, the kit of the present disclosure may provide the conjugate and/or the pharmaceutical composition in one container. In some embodiments, the kit of the present disclosure may comprise a container providing pharmaceutically acceptable excipients. In some embodiments, the kit may further comprise additional ingredients, such as stabilizers or preservatives. In some embodiments, the kit of the present disclosure may comprise at least one additional therapeutic agent in a container other than the container providing the conjugate and/or the pharmaceutical composition of the present disclosure. In some embodiments, the kit may comprise instructions for mixing the conjugate and/or the pharmaceutical composition with pharmaceutically acceptable carriers and/or adjuvants or other ingredients (if any).

In the kit of the present disclosure, the conjugate and pharmaceutically acceptable carriers and/or adjuvants as well as the pharmaceutical composition and/or pharmaceutically acceptable adjuvants may be provided in any form, e.g., in a liquid form, a dry form, or a lyophilized form. In some embodiments, the conjugate and pharmaceutically acceptable carriers and/or adjuvants as well as the pharmaceutical composition and optional pharmaceutically acceptable adjuvants are substantially pure and/or sterile. In some embodiments, sterile water may be provided in the kit of the present disclosure.

Hereinafter, the present disclosure will be further illustrated with reference to the examples, but is not limited thereto in any respect.

### Example

Unless otherwise specified, the reagents and culture media used in the following examples are all commercially available, and the procedures used such as nucleic acid electrophoresis, real-time PCR, and the like are all performed according to the method described in Molecular Cloning (Cold Spring Harbor LBboratory Press (1989)).

### Preparation examples 1-8 and 18 Synthesis of the conjugates AP1-AP8 and AP18

The conjugates numbered AP1-AP8 and AP18 in Table 1 were synthesized by solid phase synthesis method, respectively, and after sequentially linking all nucleoside monomers in 3' to 5' direction according to the nucleotide sequences corresponding to AP1-AP8 and AP18 in Table 1, the Cy5 phosphoramidite monomer was then linked according to the solid phase synthesis method for linking nucleoside phosphoramidite monomers (purchased from Suzhou GenePharma Inc., Cat. No. CY5P21H1B). Subsequently, the nucleotide sequence was added into the mixed solution of aqueous methylamine solution and aqueous ammonia at the euqal volume, the dosage of the solution relative to oligonucleotide was 0.5 ml/µmol, reacted at 25°C for 2h, the solids was removed by filtration, and the supernatant was concentrated in vacuum to dryness.

Purification of the prepared conjugates was completed by using a preparative ion chromatography purification column (Source 15Q) with a gradient elution of NaCl aqueous solution. Specifically, eluent A was 20 mM sodium phosphate (pH 8.1), solvent was water/acetonitrile in 9:1 (v/v); eluent B was 1.5 M sodium chloride, 20 mM sodium phosphate (pH 8.1), solvent was water/acetonitrile in 9:1 (v/v); elution gradient: the ratio of eluent A: eluent B = 100:0-50:50. The product eluate was collected, combined and desalted by using a reverse phase chromatography purification column. The specific conditions include: using a Sephadex column for desalination (filler: Sephadex G25) and eluting with deionized water. The obtained eluent was concentrated to remove the solvent and lyophilized to obtain conjugates AP1-AP8 and AP18, respectively, in which the ribose 5' position of 5' terminal nucleotide was linked to Cy5 fluorescent group by phosphate ester linking group.

After the synthesis of the aforementioned conjugates AP1-AP8 and AP18, the obtained conjugates were diluted to a concentration of 0.2 mg/mL by using ultra-pure water (homemade by Milli-Q ultra-pure water instrument, with resistivity of 18.2 MΩ* cm (25°C)). The molecular weight was determined by a LC-MS instrument (LC-MS, liquid chromatography-mass spectrometry, purchased from Waters Crop., model: LCT Premier). As a result, the measured value was in conformity with the calculated value, indicating that the target conjugates have been obtained.

The molecure weights and MS values of the conjugates were shown in Table 1a below.

**Table 1a Mass spectrum characterization results of the conjugates**

| Number | Calculated MS value | Measured MS value |
|---|---|---|
| AP1 | 11113.51 | Molecular ion peak: 1850.9 (6-valent); 1586.3 (7-valent) |
| AP2 | 11113.51 | Molecular ion peak: 1850.9 (6-valent); 1586.3 (7-valent) |
| AP3 | 8173.61 | 8171.39 |
| AP4 | 7814.38 | 7812.23 |
| AP5 | 7455.14 | 7452.96 |
| AP6 | 8516.85 | 8514.56 |
| AP7 | 8414.74 | Molecular ion peak: 2101.9 (4-valent); 1681.4 (5-valent) |
| AP8 | 8471.75 | 8469.53 |
| AP18 | 10704.67 | 10703.27 |

### Comparative preparation examples 9-17 and 19 Synthesis of the comparative conjugates comparative AP9- comparative AP17, comparative AP19

The conjugates numbered comparative AP9-comparative AP17, comparative AP19 in Table 1 were synthesized, respectively,, and confirmed by determining molecular weight according to the method of preparation example 1, the only difference was that sequentially linking nucleoside monomers respectively according to the sequences corresponding to comparative AP9-comparative AP173, comparative AP19 in Table 1. Therefore, the comparative conjugates comparative AP9-comparative AP17, comparative AP19 were obtained, respectively, in which the ribose 5' position of 5' terminal nucleotide was linked to Cy5 fluorescent group by phosphate ester linking group.

**Table 1 Nucleotide sequences of the conjugates**

| Preparation example | Conjugate | Nucleotide sequence 5'-3' | SE Q ID NO: |
|---|---|---|---|
| Preparation example 1 | AP1 | CY5-UmCmUmAmUmGmGmCmUmGmCmCmGmAmUmCmUmGmGmUmCmUmCmCmAmUmGmUm AmCmGmUm | 33 |
| Preparation example 2 | AP2 | CY5-CmUmGmGmAmGmUmUmCmAmGmAmCmGmUmGmUmUmGmCmUmCmUmUmCmCmGmAm UmCmUmCm | 21 |
| Preparation example 3 | AP3 | CY5-CmUmGmGmAmGmUmUmCmAmGmAmCmGmUmGmUmUmGmCmUmCmUm | 27 |
| Preparation example 4 | AP4 | CY5-CmUmGmAmGmUmUmCmAmGmAmCmGmUmGmUmUmGmCmUmCmUm | 28 |
| Preparation example 5 | AP5 | CY5-CmUmGmCmUmUmCmAmGmAmCmGmUmGmUmUmAmGmCmUmUm | 29 |
| Preparation example 6 | AP6 | CY5-AmCmGmGmAmGmUmUmCmAmGmAmCmGmUmGmUmUmGmCmUmCmUmUm | 30 |
| Preparation example 7 | AP7 | CY5-CmUmGmGmAmGmUmUmCmAmCmUmAmCmUmGmUmUmGmCmUmCmUmUm | 31 |
| Preparation example 8 | AP8 | CY5-CmUmGmGmAmGmUmUmCmAmGmUmUmGmUmGmUmUmGmCmUmCmUmUm | 32 |
| Comparative preparation example 9 | Comparative AP9 | CY5-GmGmAmCmAmUmGmGmAmUmUmCmUmUmGmUmCmUmGmUmGmUmCmCm | 45 |
| Comparative preparation example 10 | Comparative AP10 | CY5-GmAmUmCmUmGmUmGmUmCmUmUmAmGmAmCmGmUmGmUmUmGmCmCm | 46 |
| Comparative preparation example 11 | Comparative AP11 | CY5-GmAmUmCmGmUmGmAmCmGmAmCmUmCmUmUmGmUmCmUmGmCmUmGmUmCmCmUm GmAmUmCm | 47 |
| Comparative | Comparative | CY5-CmGmUmCmUmCmCmAmGmCmAmUmGmUmGmUmAmGmGmUmCmUmCmUmGmUmCmUm | 48 |
| preparation example 12 | AP12 | CmUmGmAm | |
| Comparative preparation example 13 | Comparative AP13 | CY5 GmGmAmGmUmUmCmAmGmAmCmGmUmGmUmUmGmCmUmCmUm | 49 |
| Comparative preparation example 14 | Comparative AP14 | CY5 CmUmGmGmAmGmUmUmCmGmUmUmGmCmUmCmUmUmCmCmGmAmUmCmUmCm | 50 |
| Comparative preparation example 15 | Comparative AP15 | CY5 CmUmGmGmAmCmAmGmAmCmGmUmGmUmCmUmUmCmCmGmAmUmCmUmCm | 51 |
| Comparative preparation example 16 | Comparative AP16 | CY5 CmUmGmGmAmGmUmUmCmAmGmAmUmCmAmGmAmCmGmUmGmUmGmUmGmUmUmGm CmUmCmUm | 52 |
| Comparative preparation example 17 | Comparative AP17 | CY5 CmUmGmGmAmGmUmUmCmAmGmAmCmGmUmGmUmAmGmCmUmCmGmUmGmUmGmAm UmCmUmCm | 53 |
| Preparation example18 | AP18 | CY5-CfUfGGAGUfUfCfAGACfGUfGUfUfGCfUfCfUfUfCfCfGAUfCfUfCf | 54 |
| Comparative preparation example 19 | Comparative AP19 | CY5-UfCfUfAUfGGCfUfGCfCfG AUfCfUfGGUfCfUfCfCfAUfGUfACfGUf | 44 |

In Table 1, C, G, U, and A represent the base composition of a nucleotide; m represents that the nucleotide adjacent to the left side of the letter m is a methoxy modified nucleotide; f represents that the nucleotide adjacent to the left side of the letter f is a fluoro modified nucleotide; and CY5 represents the linking site of fluorescent dye group Cy5 (Cyanine 5) group on the aptamer.

### Preparation example 20 Synthesis of conjugate 20

**Table 2 Nucleotide sequences of the conjugates**

| Preparation example | Conjugate | Seuquence | Nucleotide sequence 5'-3' | SEQ ID NO: |
|---|---|---|---|---|
| Preparation example 20 | Conjugate 20 | Oligonucleotide single strand | MMAE-CmUmGmGmAmGmUmUmCmAmGmAmCmGmUmGmUmUmGmC mUmCmUmUmCmCmGmAmUmCmUmCm | 21 |
| Preparation example 21 | Conjugate 21 | Oligonucleotide single strand | MMAE-CmUmGmGmAmGmUmUmCmAmGmAmCmGmUmUmGmUmGmC mUmCmUmUm | 24 |
| Preparation example 22 | Conjugate 22 | Oligonucleotide single strand | MMAE-CmUmGmGmAmGmUmUmCmAmGmAmCmGmUmGmUmUmGmC mUmCmUmUm | 22 |
| Preparation example 23 | Conjugate 23 | Oligonucleotide single strand | MMAE-CmUmGmGmAmGmUmUmCmAmGmAmCmCmGmUmGmUmGmC mUmCmUmUm | 26 |
| Preparation | Comparative | Oligonucleotide | MMAE-GmGmAmCmAmUmGmGmAmUmUmCmUmUmGmUmCmUmGmU | 45 |
| example 24 | conjugate 24 | single strand | mGmUmCmCm | |
| Preparation example 25 | Comparative conjugate 25 | Oligonucleotide single strand | MMAE-CmGmUmCmUmCmCmAmGmCmAmUmGmUmGmUmAmGmGmU mCmUmCmUmGmUmCmUmCmUmGmAm | 48 |
| Preparation example 26 | Conjugate 26 | Oligonucleotide single strand | MMAE-CmUmGmCmUmUmCmAmGmAmCmGmUmGmUmUmAmGmCmU mUm | 29 |

In Table 2, C, G, U, and A represent the base composition of a nucleotide; m represents that the nucleotide adjacent to the left side of the letter m is a methoxy modified nucleotide; and MMAE represents the a linking site of small molecule drug group MMAE (Monomethyl auristatin E) group on the aptamer.

In this preparation example, conjugate 20 was prepared according to the following steps. The conjugate 20 was obtained by replacing the dye group in the conjugate AP2 with the small molecule drug group MMAE, wherein the linking group was 2-(phosphate ester group-(CH₂)₆-S-)-maleimide caproyl -valine-citrulline-p-aminobenzylidene.

### (20-1) Preparation of oligonucleotide S1

The oligonucleotide sequence of conjugate 20 in Table 2 was synthesized by solid phase synthesis method, the only difference was that sequentially linking nucleoside monomers according to the oligonucleotide sequence corresponding to conjugate 20 in Table 2; in the process of solid phase synthesis, after linking the last nucleoside monomer at 5' terminal, the phosphoramidite monomer containing HO-(CH₂)₆-S-S-(CH₂)₆-group (purchased from Hongene Biotech Co. Ltd.) is additionally linked according to the method for linking the nucleoside phosphoramidite monomer, oligonucleotide single strand was cleaved from the solid phase support to obtain oligonucleotide single strand S1 (70.00mg, 6.42 µmol) as shown by Formula (20-a):

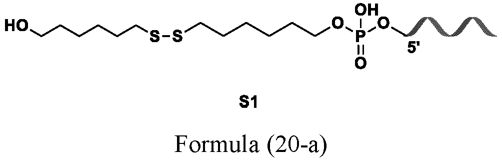

In the Formula, 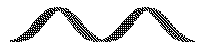 represents the oligonucleotide sequence corresponding to conjugate 20.

### (20-2) Synthesis of oligonucleotide S2

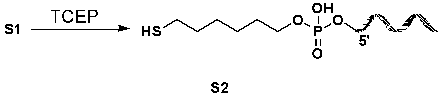

After dissolving 70.0 mg S1 obtained in step (20-1) (6.42 µmol) with 10.0 ml of purified water, TCEP aqueous solution obtained by dissolving 105 mg TCEP (tri(2-chloroethyl) phosphate ester, 0.37 mmol, purchased from Bide Pharmatech Ltd., Cat. No. BD155793) in 10.0 ml of purified water was added to the resulting solution. The reaction solution was mixed and reacted at room temperature for 2h, then diluted with 10 mL of purified water and filtered to obtain 28 mL of reaction solution. The resulting reaction solution was transferred to 3K size of ultrafiltration tube and centrifuged at 3900 rpm for 30min. Then the steps of ultrafiltration and centrifugation were repeated two times to collect the products in filter membrane, to obtain oligonucleotide S2 (67.0 mg, yield: 95.7%).

### (20-3) Synthesis of oligonucleotide 20

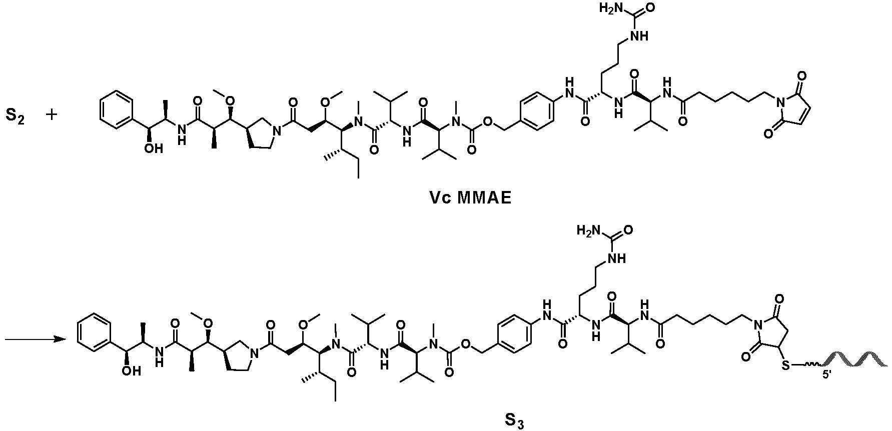

24 mg Vc MMAE (18.56 µmol, 5eq, purchased from CSN Company, Cat. No. CSN16143-005) was dissolved in 6.0 ml of DMF, and 60 µl of triethylamine was added, to obtain Vc MMAE solution. After dissolving 40.0 mg of the oligonucleotide S2 prepared in step (20-2) (3.71 µmol, 1eq) with 6.0 ml of purified water, the above Vc MMAE solution was added to the resulting solution, reacted at room temperature for 2h, to obtain conjugate 20 crude product (represented as S3 in process diagram).

The obtained conjugated 20 crude product was diluted with 0.5 ml of purified water and filtered with 0.45 µm of filter membrane. The filtrate was purified by Agilent semi-prepared reversed-phase column chromatography, the chromatographic column used was Kromasil 100-10-C18, 100Å, 10um, 21.2*250mm; Gradient elution was performed with 100mM triethylamine acetate buffer (TEAA, pH=7.0-7.3): acetonitrile = 95:5 - 35:65 as a mobile phase. The eluent of product peak was collected, evaporated to remove solvent, to obtain conjugate 20 (55 mg, yield 56.7%). The molecular weight was determined by a LC-MS, calculated value: 12092.73, measured value: 12091.39, the measured value was in conformity with the calculated value, indicating that conjugate 20 has the structure as shown by S3, wherein the conjugate 20 was obtained by replacing the dye group in the conjugate AP2 with the small molecule drug group MMAE, and the linking group was 2-(phosphate ester group-(CH₂)₆-S-)-maleimide caproyl -valine-citrulline-p-aminobenzylidene (2-(phosphate ester group-(CH₂)₆-S-)-MC-Val-Cit-PAB).

### Preparation example 21-26 Synthesis of conjugates 21-23, 26 and comparative conjugates 24-25

The conjugates numbered Conjugate 21, Conjugate 22, Conjugate 23, Compartive conjugate 24, Compartive conjugate 25 and Conjugate 26 in Table 2 were synthesized, respectively, and confirmed by determining molecular weight according to the method of preparation example 20, the only difference was that sequentially linking nucleoside monomers according to the sequences corresponding to Conjugate 21, Conjugate 22, Conjugate 23, Compartive conjugate 24, Compartive conjugate 25 and Conjugate 26 in Table 2.

The molecure weights and MS values of the conjugates were shown in Table 2a below:

**Table 2a: Mass spectrum characterization results of the conjugates**

| Number | Calculated MS value | Measured MS value |
|---|---|---|
| Conjugate 20 | 12092.73 | 12091.39 |
| Conjugate 21 | 9473.03 | 9472.07 |
| Conjugate 22 | 9473.03 | 9471.76 |
| Conjugate 23 | 9472.05 | 9472.2 |
| Compartive conjugate 24 | 9473.03 | 9471.41 |
| Compartive conjugate 25 | 12092.73 | 12091.48 |
| Conjugate 26 | 8434.37 | 8434.09 |

### Experimental Example 1 In vivo targeting activity of the conjugates in mice

In this experimental example, the *in vivo* targeting activity of the resulting conjugates AP2, AP3-8 and comparative AP9- comparative AP12 in mice was investigated.

U118MG human glioma cells (purchased from Guangzhou Jennio Biotech Co., Ltd) were cultured in DMEM complete medium (MACGENE company, Cat No. CM15019) supplemented with 10% fetal bovine serum (FBS, RMBIO company) at 37°C in an incubator containing 5% CO₂/95% air.

U118MG human glioma cells that grow logarithmically were selected and digested with (0.25% pancreatic enzyme), the cells were collected, centrifuged to remove the supernatant, then resuspended in DMEM medium supplemented with 10% FBS to formulate into cell culture medium with a concentration of 1×10⁸ cells/mL.

Experimental animals were 24 NOD-SCID mice, male, 12 weeks old (purchased from SPF (Beijing) Biotechnology Co., Ltd). The above cell culture medium was inoculated on subcutaneous site of the right forelimb of NOD-SCID mice with an inoculation volume of 100µL per mouse, i.e., each mouse was inoculated with 1×10⁷ cells. The mice were kept for 20 days after injection, to obtain the mice inoculated with U118MG subcutaneous tumor.

The above resulting aptamers AP2, AP3-8 and comparative AP9-comparative AP12 were formulated into 0.3mg/mLof solution with DMEM medium, respectively.

The administration was initiated 14 days after inoculation of U118MG cells, and the day of administration was designated as D1. The administration method of tail intravenous injection was adopted in this experiment; administered once a day, three times in total.

24 mice inoculated with U118MG subcutaneous tumors were randomly divided into 12 groups with 2 mice per group.

For 7 groups of mice, each mouse in each group was administrated with AP2, AP3, AP4, AP5, AP6, AP7 or AP8 respectively with a single administration volume of 10 µL/g mouse body weight. It was calculated that the single administration dosage was 3 mg/kg, and designated as test groups 1A-1G, respectively.

For other 4 groups of mice, each mouse in each group was administrated with comparative AP9, comparative AP10, comparative AP11 or comparative AP12 respectively with a single administration volume of 10 µL/g mouse body weight. It was calculated that the single administration dosage was 3 mg/kg, and designated as control groups 1H-1K, respectively.

For 2 mice of the last group, each mouse was administrated with DMEM medium with an administration volume of 10 µL/g mouse body weight, and designated as blank control group 1Y.

1h, 24h and 48h after first administration, small animals living optical imaging system IVIS Lumina Series III was used for living imaging of each mouse. On D5, mice of each group were killed; and the tumor tissues and kidneys were taken for fluorescence imaging.

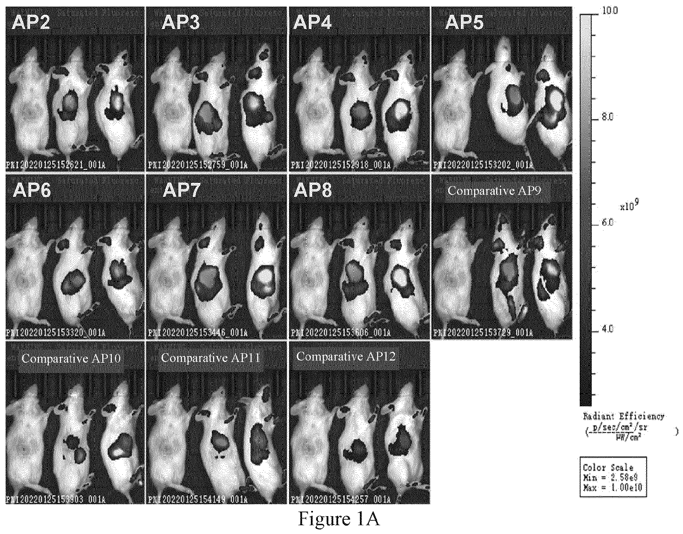

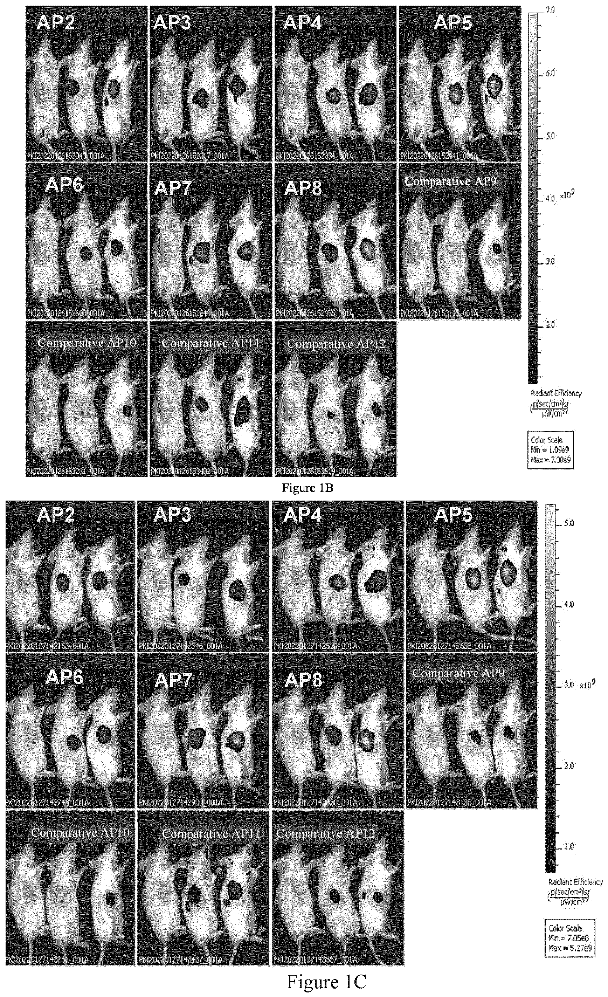

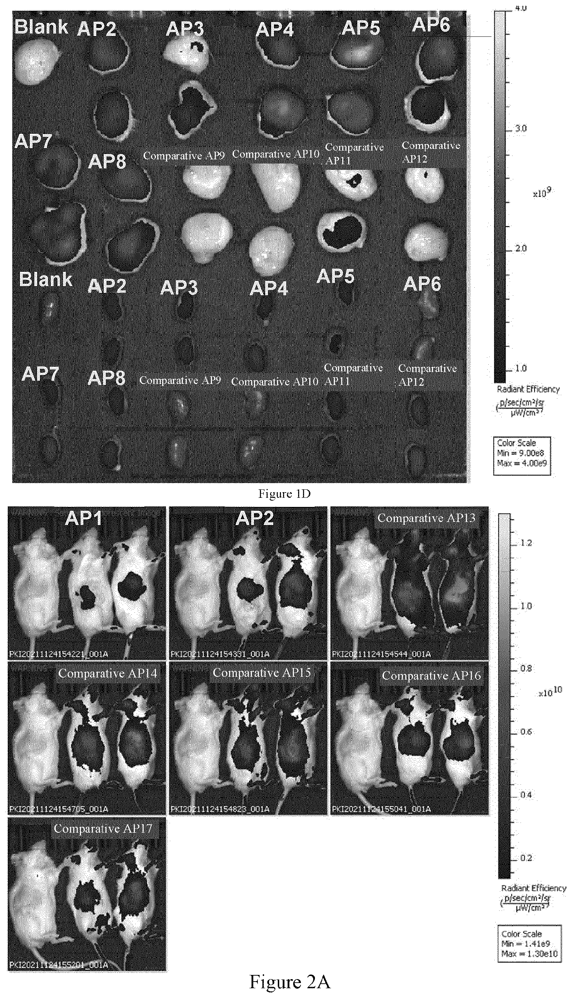

Figures 1A-1C showed the fluorescence imaging pictures in mice at 1h, 24h and 48h after administrating different conjugates, respectively, in which leftmost mouse of the three mice in each small figure was a mouse in blank control group 1Y. As can be seen from Figure 1A, the blank control group showed no fluorescence signal; in contrast, 1h after administration, the mice in all test groups and control groups all showed fluorescence signals at subcutaneous tumors; as can be seen from Figures 1B and 1C, 24h and 48h after administration, the mice in test groups 1A-1G only showed strong fluorescence signals at subcutaneous tumors , while the mice in control groups 1H-1K almost showed no fluorescence signals or only weak fluorescence signals. Furthermore, Figure 1D showed the fluorescence signal imaging picture of tumor tissues and kidneys of mice in each group after the mice were killed on D5, wherein Blank represented blank control group 15Y. As can be seen from Figure 1D, the mice in blank control group 1Y and control groups 1H-1K almost showed no fluorescence signals or only weak fluorescence signals at tumor tissues; in contrast, the mice in test groups 1A-1G administered with the conjugates of the present disclosure showed strong fluorescence signals at tumor tissues, while only showed weak fluorescence signals at the metabolic organ kidney, indicating that the conjugates provided by the present disclosure containing the delivery group can more stably and efficiently target the tumor tissues as compared with the control conjugates.

### Experimental Example 2 In vivo targeting activity of the conjugates in mice

In this experimental example, the *in vivo* targeting activity of the resulting conjugates AP2, AP1 and comparative AP13- comparative AP17 in mice were investigated.

U118MG human glioma cells (purchased from Guangzhou Jennio Biotech Co., Ltd) were cultured in DMEM complete medium (MACGENE company, Cat No. CM15019) supplemented with 10% fetal bovine serum (FBS, RMBIO company) at 37°C in an incubator containing 5% CO₂/95% air.

U118MG human glioma cells that grow logarithmically were selected and digested with (0.25% pancreatic enzyme), the cells were collected, centrifuged to remove the supernatant, then resuspended in DMEM medium supplemented with 10% FBS to formulate into cell culture medium with a concentration of 1×10⁸ cells/mL.

Experimental animals were 16 NOD-SCID mice, male, 12 weeks old (purchased from SPF (Beijing) Biotechnology Co., Ltd). The above cell culture medium was inoculated on subcutaneous site of the right forelimb of NOD-SCID mice with an inoculation volume of 100µL per mouse, i.e., each mouse was inoculated with 1×10⁷ cells. The mice were kept for 20 days after injection, to obtain the mice inoculated with U118MG subcutaneous tumor.

The above resulting aptamers AP2, AP1 and comparative AP13-comparative AP17 were formulated into 0.3mg/mLof solution with DMEM medium, respectively.

The administration was initiated 21 days after inoculation of U118MG cells, and the day of administration was designated as D1. The administration method of tail intravenous injection was adopted in this experiment; administered once a day, three times in total.

16 mice inoculated with U118MG subcutaneous tumors were randomly divided into 8 groups with 2 mice per group.

For 2 groups of mice, each mouse in each group was administrated with AP2 or AP1 respectively with a single administration volume of 10 µL/g mouse body weight. It was calculated that the single administration dosage was 3 mg/kg, and designated as test groups 2A-2B, respectively.

For other 5 groups of mice, each mouse in each group was administrated comparative AP13, comparative AP14, comparative AP15, comparative AP16 or comparative AP17 respectively with a single dose volume of 10 µL/g mouse body weight. It was calculated that the single administration dosage was 3 mg/kg, and designated as control groups 2C-2G, respectively.

For 2 mice of the last group, each mouse was administrated with DMEM medium with an administration volume of 10 µL/g mouse body weight, and designated as blank control group 2Y.

1h, 24h and 48h after first administration, small animals living optical imaging system IVIS Lumina Series III was used for living imaging of each mouse. On D6, mice of each group were killed; and the tumor tissues and kidneys were taken for fluorescence imaging.

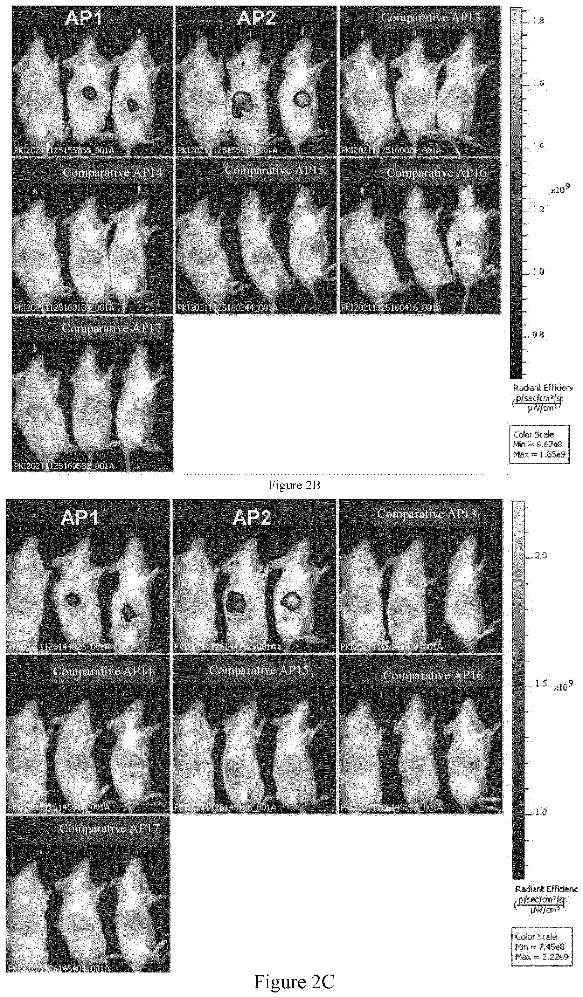

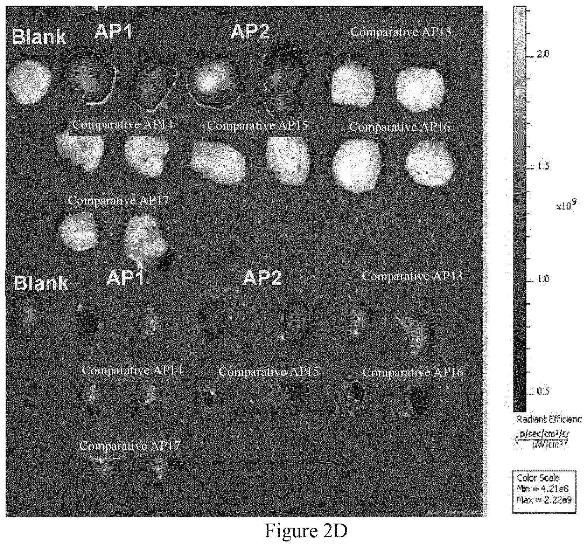

Figures 2A-2C showed the fluorescence imaging pictures in mice at 1h, 24h and 48h after administrating different conjugates, respectively, in which leftmost mouse of the three mice in each small figure was a mouse in blank control group 2Y. As can be seen from Figure 2A, the blank control group showed no fluorescence signal; in contrast, 1h after administration, the mice in all test groups and control groups all showed fluorescence signals at subcutaneous tumors; as can be seen from Figures 2B and 2C, 24h and 48h after administration, the mice in test groups 2A and 2B only showed strong fluorescence signals at subcutaneous tumors, while the mice in blank control group 2Y and control groups 2C-2G showed no fluorescence signals at all. Furthermore, Figure 2D showed the fluorescence signal imaging picture of tumor tissues and kidneys of mice in each group after the mice were killed on D6, wherein Blank represented blank control group 2Y. As can be seen from Figure 2D, the mice in blank control group 2Y and control groups 2C-2G showed no fluorescence signals at all at tumor tissues; in contrast, the mice in test group 2A or 2B administered with the conjugates of the present disclosure showed strong fluorescence signals at tumor tissues, while only showed weak fluorescence signals at the metabolic organ kidney, indicating that the aptamers containing the seuqnece as shown by Formula (1) can stably and efficiently target the tumor tissues as compared with the control aptamers, further indicating that the conjugates provided by the present disclosure containing the delivery group formed by these aptamers can efficiently reach the tumor tissues.

### Experimental Example 3 In vivo activity of the conjugates of the present disclosure in mice

In this experimental example, the *in vivo* anti-tumor activity of the resulting conjugate 20 in mice was investigated.

The mice in this experiment were purchased from SPF Co., Ltd, the germline was NOD-SCID, the grade was SPF, male, 6-8 weeks old; U118MG glioma cells were purchased from Jennio.

U118MG cells that grow logarithmically were selected, digested and resuspended in DMEM complete medium (MACGENE company, Cat No. CM15019) supplemented with 10% fetal bovine serum (FBS, GIBCO company), and cultured until the cell density reached 1×10⁸ cells/mL, to obtain the culture medium containing U118MG cells. The above culture medium containing U118MG cells was inoculated on subcutaneous site of the right forelimb of mice, and the injection volume was 100µL. Each mouse was inoculated with 1×10⁷ U118MG glioma cells.

The above resulting conjugate AP2 was formulated into 1.94 mg/mL of solution with PBS. Conjugat 20 was formulated into 0.625 mg/mL, 1.25 mg/mL and 2.06 mg/mL of solution with PBS, respectively (all calculated based on the amount of oligonucleotide). MMAE (purchased from Shanghai Macklin Inc., Lot No. C12886583) was dissolved into 0.038 mg/mL of solution with a mixed solution of 10%DMSO+90%PBS (volume ratio).

The day of cell inoculation was designated as D1, administered once on D8, D12, D16 and D20, respectively.

36 mice were randomly divided into 6 groups with 6 mice per group:
For blank control group 3a, PBS was administered by tail intravenous injection with a single administration volume of 10 µL/g.

For control group 3b, the above conjugate AP2 solution was administered by tail intravenous injection with a single administration volume of 10 µL/g, a single administration dosage of 15.5 mg/kg.

For control group 3c, the above MMAE solution was administered by tail intravenous injection with a single administration volume of 10 µL/g, a single administration dosage of 0.3 mg/kg.

For test group 3d, the above conjugate 20 solution with a concentration of 0.625 mg/mL was administered by tail intravenous injection with a single administration volume of 10 µL/g, a single administration dosage of 5 mg/kg (calculated based on the mass of oligonucleotide), wherein the dosage of MMAE contained was equivalent to 0.3 mg/kg.

For test group 3e, the above conjugate 20 solution with a concentration of 2.06 mg/mL was administered by tail intravenous injection with a single administration volume of 10 µL/g, a single administration dosage of 16.5 mg/kg (calculated based on the mass of oligonucleotide), wherein the dosage of MMAE contained was equivalent to 1 mg/kg.

For test group 3f, the above conjugate 20 solution with a concentration of 1.25 mg/mL was administered by subcutaneous injection with a single administration volume of 5 µL/g, a single administration dosage of 5 mg/kg (calculated based on the mass of oligonucleotide), wherein the dosage of MMAE contained was equivalent to 0.3 mg/kg.

### [3] Detection

Long diameter and short diameter of the tumors were measured by *in vitro* measurement method. The tumor volume was calculated according to the formula 1/2 (long diameter × short diameter²). Prior to the first administration on D8, the tumor volumes in each group were measured and the average tumor volumes were recorded; the tumor volumes in each group were measured and recorded from D16 twice a week.

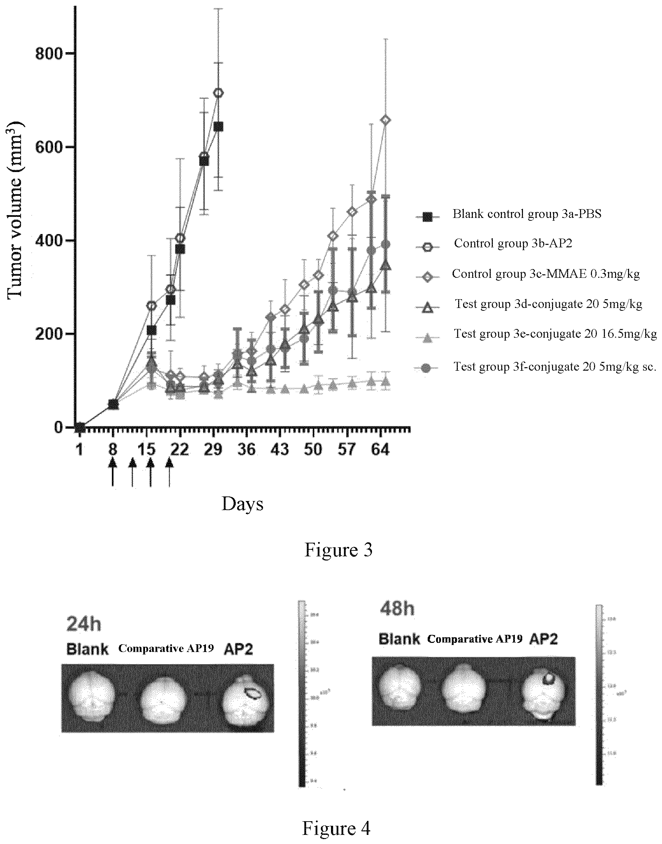

Figure 3 was a line graph showing the changes in tumor volume in each group of mice over time. As shown in Figure 3, the tumor volumes were increased rapidly in blank control group 3a administered with PBS only and control group 3b administered with AP2. The increase rate of tumor volume was decreased in control group 3c administered with MMAE only, indicating that MMAE *per se* has an inhibition effect on tumor proliferation. Furthermore, the tumor volumes of test groups 3d and 3f with MMAE content comparable to control group 3c were significantly smaller than control group 3c during the test period, showing more excellent anti-tumor activity than control group 3c administrated with MMAE alone. This indicated that the conjugates provided by the present desclosure can effectively deliver MMAE to tumor tissues, showing tumor targeting ability and reducing the risk of toxicity caused by the distribution of MMAE molecules in other tissues, and various ways of administration can effectively inhibit tumor proliferation. In addition, further increasing the administration dosage of test group 3e can result in almost no increase in tumor volume during the testing period, showing more excellent anti-tumor effects.

The above results indicated that the conjugates provided by the present disclosure can effectively deliver small molecule drug group having inhibition effect on tumors to tumor tissue, showing good anti-tumor activity and dose-dependent effect.

### Experimental Example 4 In vivo distribution of conjugates in U118MG cell orthotopic tumor model mice

U118MG human glioma cells (purchased from Guangzhou Jennio Biotech Co., Ltd) were cultured in DMEM complete medium (MACGENE company, Cat No. CM15019) supplemented with 10% fetal bovine serum (FBS, RMBIO company) at 37°C in an incubator containing 5% CO₂/95% air.

The cells were digested with 0.25% pancreatic enzyme and collected. The supernatant was aspirated, and the cells were resuspended in DMEM medium supplemented with 10% FBS to formulate into cell culture medium with a concentration of 4×10⁷ cells/mL.

Experimental animals were 6 NOD-SCID mice, male, 12 weeks old (purchased from SPF (Beijing) Biotechnology Co., LTD.). The above cell culture medium was inoculated into NOD-SCID mice. The cell culture medium was injected into the right striatum of the mice by the method of mice lateral ventricle injection, the position was AP (anteroposterior): 1 mm, ML (medial lateral): 1.5 mm, DV (dorsal ventral): 3.5 mm, the injection volume was 10µL, i.e., each mouse was inoculated with 4×10⁵ cells. The mice were kept for 14 days after injection.

AP2 and comparative AP19 were dissolved into 0.3 mg/mL of solutions (calculated according to aptamer) with 1×DMEM medium. 4 mice as described above were taken and respectively injected with AP2 and comparative AP19 solution administered by tail intravenous injection. The administration dosage of all animals was calculated based on their body weights, the administration volume was all 10 µL/g, the administration dosage was 3 mg/kg per animal (calculated based on the amount of aptamer), 2 mice were administrated in each group and designated as test group 4a and control group 4b, respectively.

Each of two mice was injected with 10 µL of DMEM medium, and designated as blank control group 4Y.

24h after administration, one mouse in each group was respectively killed and brain tissue was taken, and the remaining mice were killed at 48h after administration and brain tissues were taken. The brain tissues of mice were taken for fluorescence imaging in IVIS Lumina Series III. The results were shown in Figure 4.

Figure 4 showed the fluorescence imaging pictures of brain tissues of U118MG orthotopic tumor model mice established after administration of blank control group 4Y, test group 4a, and control group 4b at 24h and 48h after administration. Among them, Blank represented blank control group, Ith represented an intrathecal injection, and iv represented a tail intravenous injection. As can be seen from Figure 4, the blank control group and the control group 4b administered with comparative AP19 showed no fluorescence signal at all in the brain, indicating that there was no significant targeting effect on brain orthotopic glioma. In contrast, test group 4a administered with the conjugates provided by the present disclosure showed strong fluorescence signals at the inoculation tumor location at 24h and 48h, indicating that the conjugate AP2 can still reach and effectively target brain glioma when administered through tail intravenous injection. This indicated that the conjugates provided by the present disclosure can also enter into brain glioma by penetrating Blood-Brain Barrier (BBB) even in the case of systematic administration, showing the excellent targeting effect and druggability.

### Experimental Example 5 In vivo activity of conjugates in U118MG cell orthotopic tumor model mice

U118MG human glioma cells expressing Luciferase (*Photinus pyralis*) reporter gene, hereinafter referred to as U118mG-luc human glioma cells (purchased from Cobioer Biosciences Co., Ltd.), were cultured according to the method of experiment example 4. U118MG-luc human glioma cells that grow logarithmically were selected and digested with 0.25% pancreatic enzyme, the cells were collected and the supernatant was aspirated after centrifugation, then the cells were resuspended in serum-free DMEM medium to formulate into cell culture medium with a cell density of 4×10⁷ cells/mL.

Experimental animals were 24 Balb/C-nude mice, male, 12 weeks old (purchased from SPF (Beijing) Biotechnology Co., LTD.). The above cell culture medium was inoculated into the striatum of Balb/C-nude mice. The cell culture medium was injected into the right striatum of the mice by the method of mice striatum injection, the position was AP (anteroposterior): 1 mm, ML (medial lateral): 1.5 mm, DV (dorsal ventral): 3.5 mm, the injection volume was 10µL, i.e., each mouse was inoculated with 4×10⁵ cells. The mice were kept for 14 days after orthotopic tumor injection.

Conjugat 20 was dissolved into a conjugate solution with a concentration of 1 mg/mL (calculated according to aptamer) with 1×DMEM medium (purchased from M&C gene technology (BEIJING) LTD., Lot No. K1902200). Conjugate 21 and compartive conjugate 24 were dissolved into conjugate solutions with a concentration of 0.8 mg/mL, respectively (calculated according to aptamer).

Day 15 after orthotopic tumor inoculation, each mouse was taken for living imaging using small animals living optical imaging system IVIS Lumina Series III. According to the fluorescence intensity of the brain, the mice are grouped, 6 mice in each group, and the day of administration was designated as D1 (i.e., the first day of the experiment, the following D4, D8, etc., corresponding to the fourth and eighth days of the experiment, and so on).

Living imaging method: each mouse was intraperitoneally injected with 10 µL/g of D-fluorescein potassium salt working solution with body weight concentration of 15 mg/mL (purchased from Yeasen Biotechnology (Shanghai) Co., Ltd.), and living imaging (IVIS^{®} Lumina III small animals living imaging system) was performed 10 minutes after injection. After imaging, the fluorescent region (ROI) of the mouse brain was circled, and the fluorescence intensity was measured by software (Radiance). Under these conditions, the Luciferase (*Photinus pyralis*) reporter gene expressed in U118MG-luc human glioma cells can produce a fluorescent response, and thus the fluorescence intensity can reflect the number of proliferation of glioma cells. The higher the fluorescence intensity, the greater the number of glioma cells.

In the experiment, each group of mice was administered by subcutaneous injection on D1, D4, D8 and D12, respectively. The mice were weighed before administration and administered by weight.

For test group 5a, each mouse was administered with conjugate 20 with a single administration volume of 5 µL/g mouse body weight. It was calculated that a single administration dosage was 5 mg/kg, wherein the dosage of MMAE contained was equivalent to 0.3 mg/kg.

For test group 5b, each mouse was administered with conjugate 21 with a single administration volume of 5 µL/g mouse body weight. It was calculated that a single administration dosage was 4 mg/kg, wherein the dosage of MMAE contained was equivalent to 0.3 mg/kg.

For control group 5c, each mouse was administered with comparative conjugate 24 with a single administration volume of 5 µL/g mouse body weight. It was calculated that a single administration dosage was 4 mg/kg, wherein the dosage of MMAE contained was equivalent to 0.3 mg/kg.

For blank control group, each mouse was administered with DMEM medium with a single administration volume of 5 µL/g mouse body weight.

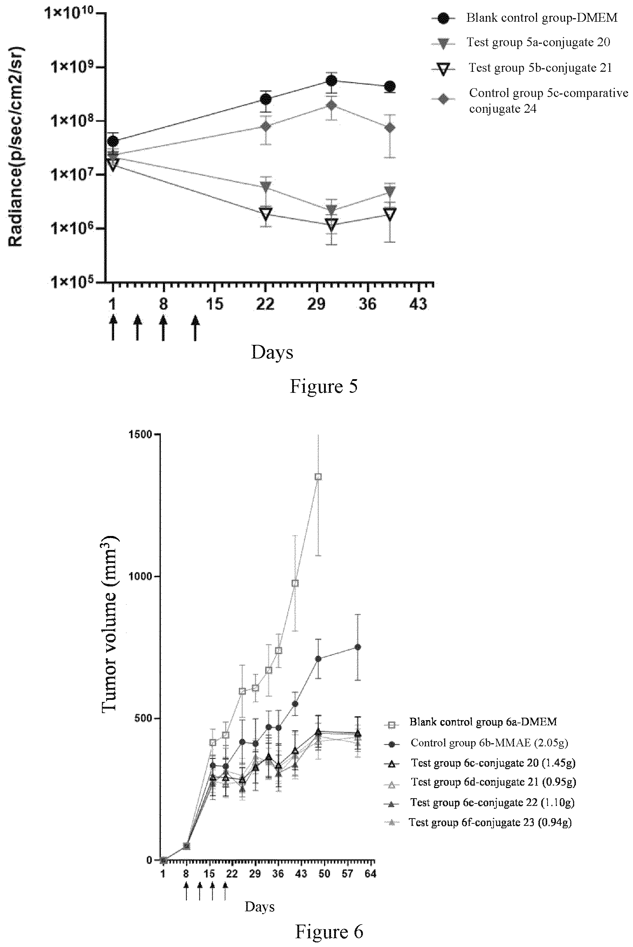

According to the above method, the mice in each group were performed for living imaging analysis on D1, D22, D31 and D39, respectively, and fluorescence intensity was determined. The results were shown in Figure 5.

Figure 5 was a line graph showing the changes of tumor fluorescence intensity in U118MG orthotopic tumor model mice over time after administration of the conjugates or control compounds provided by the present disclosure. As can be seen from Figure 5, with the exntension of observation time, the fluorescence intensity (Radiance) of tumor in the blank control group and the control group was siginicantly increased as compared with D1 after administration, indicating that the number of U118MG human glioma cells was siginicantly increased. In contrast, the fluorescence intensity of tumor in the test groups 5a and 5b administered with the conjugates provided by the present disclosure was siginicantly decreased, and the magnitude of the reduction can be up to one order, even up to more than two orders as compared with the control group, indicating that the number of U118MG human glioma cells was siginicantly decreased, decreasing to 1/10 of the initial number as compared with the beginning of the experiment, even decreasing to less than 1% of the control group. Even only by subcutaneous administration, the conjugates provided by the present disclosure can effectively penetrate the blood-brain barrier, and effectively target and enter into brain glioma, and had a good inhibitory effects on tumor growth, demonstrating good treatment compliance and high druggability of efficiently inhibiting tumors.

### Experimental Example 6 In vivo inhibitory activity of conjugates against U118MG subcutaneous tumors in mice

U118MG human glioma cells (purchased from GuangZhou Jennio Biotech Co., Ltd) were cultured according to the method of experimental example 2. U118MG human glioma cells that grow logarithmically were selected and digested with 0.25% pancreatic enzyme, the cells were collected and centrifuged to remove the supernatant, then the cells were resuspended in serum-free DMEM medium to formulate into cell culture medium with a concentration of 1×10⁸ cells/mL.

Experimental animals were 36 NOD-SCID mice, male, 12 weeks old (purchased from SPF (Beijing) Biotechnology Co., Ltd). The above cell culture medium was inoculated on subcutaneous site of the right back of NOD-SCID mice with an inoculation volume of 100 µL per mouse, i.e., each mouse was inoculated with 1×10⁷ cells. The mice were kept for 7 days after injection.

Conjugate 20 was dissolved into a conjugate solution with a concentration of 1 mg/mL (calculated according to aptamer) with serum-free DMEM medium. Conjugate 21, conjugate 22, and conjugate 23 were respectively dissolved into conjugate solutions with a concentration of 0.8 mg/mL (calculated according to aptamer). MMAE was dissolved into a solution with a concentration of 0.06 mg/mL with 10% DMSO + 90% serum-free DMEM medium (volume ratio).

The administration was initiated 7 days after inoculation of U118MG cells, and the day of administration was designated as D8. The administration method of subcutaneous administration in the abdomen was adopted in this experiment, administered once on D8, D12, D16 and D20, four times in total.
For blank control group 6a, each mouse was administered with DMEM with a single administration volume of 5 µL/g mouse body weight;
For control group 6b, each mouse was administered with MMAE with a single administration volume of 5 µL/g mouse body weight. It was calculated that a single administration dosage was 0.3 mg/kg;
For test group 6c, each mouse was administered with conjugate 20 with a single administration volume of 5 µL/g mouse body weight. It was calculated that a single administration dosage was 5 mg/kg, wherein the dosage of MMAE contained was equivalent to 0.3 mg/kg;
For three groups of mice, each mouse was administered with conjugate 21, conjugate 22 or conjugate 23 respectively with a single administration volume of 5 µL/g mouse body weight. It was calculated that the single administration dosage was 4 mg/kg, wherein the dosage of MMAE contained was equivalent to 0.3 mg/kg; they were respectively designated as the test groups 6d, 6e and 6f;
The tumor volumes were measured on D16, D20, D25, D29, D33, D36, D41, D48, and D60. The long diameter and short diameter of the tumors were measured by *in vitro* measurement method. The tumor volume was calculated according to the Formula 1/2 (long diameter × short diameter²). At the end of the experiment, tumor tissues were taken on D60 and weighed. Figure 6 was a line graph showing the changes in tumor volume in mice over time on different days after administration of different conjugates. As can be seen from Figure 6, as compared with the control group 6b or the blank control group, the tumor volume and tumor weight of the mice in the test groups 6c to 6f administered with the conjugates of the present disclosure were significantly reduced. The above results indicated that the conjugates of the present disclosure can effectively reach tumor tissues and exhibit good anti-tumor activity.

### Experimental Example 7: In vivo activity of long-interval administration of conjugates against U118MG subcutaneous tumor model mice

According to the method of experimental example 2, 42 mice inoculated with U118MG subcutaneous tumor were obtained, and the mice were kept after injection.

MMAE was dissolved into solutions with a concentration of 0.03 mg/mL and 0.01 mg/mL with 10% DMSO + 90% serum-free DMEM medium (volume ratio); conjugate 21 was dissolved into a solution with a concentration of 0.5 mg/mL and 0.165 mg/mL (calculated according to aptamer); and comparative conjugate 25 was dissolved into solutions with a concentration of 0.5 mg/mL and 0.165 mg/mL (calculated according to aptamer).

7 days after inoculation of U118MG cells, all mice were randomly divided into seven groups, with six mice in each group, and each group of mice was administered. The day of administration was designated as D8. The administration method of subcutaneous administration in the abdomen was adopted in this experiment; administered once on D8, D11, D15, D29, D32 and D36, six times in total. The mice were weighed before administration, and the administration volume was calculated by weight.
For blank control group, each mouse was administered with DMEM medium with a single administration volume of 10 µL/g mouse body weight;
For test group 7a, each mouse was administered with MMAE with a concentration of 0.01 mg/ml with a single administration volume of 10 µL/g mouse body weight. It was calculated that a single administration dosage was 0.1 mg/kg.
For test group 7b, each mouse was administered with conjugate 21 with a concentration of 0.165 mg/ml with a single administration volume of 10 µL/g mouse body weight. It was calculated that a single administration dosage was 1.65 mg/kg (the corresponding MMAE dosage was 0.1 mg/kg).
For test group 7c, each mouse was administered with comparative conjugate 25 with a concentration of 0.165 mg/ml with a single administration volume of 10 µL/g mouse body weight. It was calculated that a single administration dosage was 1.65 mg/kg (the corresponding MMAE dosage was 0.1 mg/kg).
For test group 7d, each mouse was administered with MMAE with a concentration of 0.03 mg/ml with a single administration volume of 10 µL/g mouse body weight. It was calculated that a single administration dosage was 0.3 mg/kg.
For test group 7e, each mouse was administered with conjugate 21 with a concentration of 0.5 mg/ml with a single administration volume of 10 µL/g mouse body weight. It was calculated that a single administration dosage was 5 mg/kg (the corresponding MMAE dosage was 0.3 mg/kg).
For control group 7f, each mouse was administered with comparative conjugate 25 with a concentration of 0.5 mg/ml with a single administration volume of 10 µL/g mouse body weight. It was calculated that a single administration dosage was 5 mg/kg (the corresponding MMAE dosage was 0.3 mg/kg).

The tumor volumes were measured on D1, D9, D16, D19, D21, D24, D26, D29, D32, D36, D39, D43, D47, D53, D57, D60, D64, D67, D71, D74, D78, D81, D84, D88, D92, D95 and D99. Among them, the blank control group was measured on D53, and the test groups 7a, 7b and the control group 7c was measured on D60, and then the experiment was terminated.

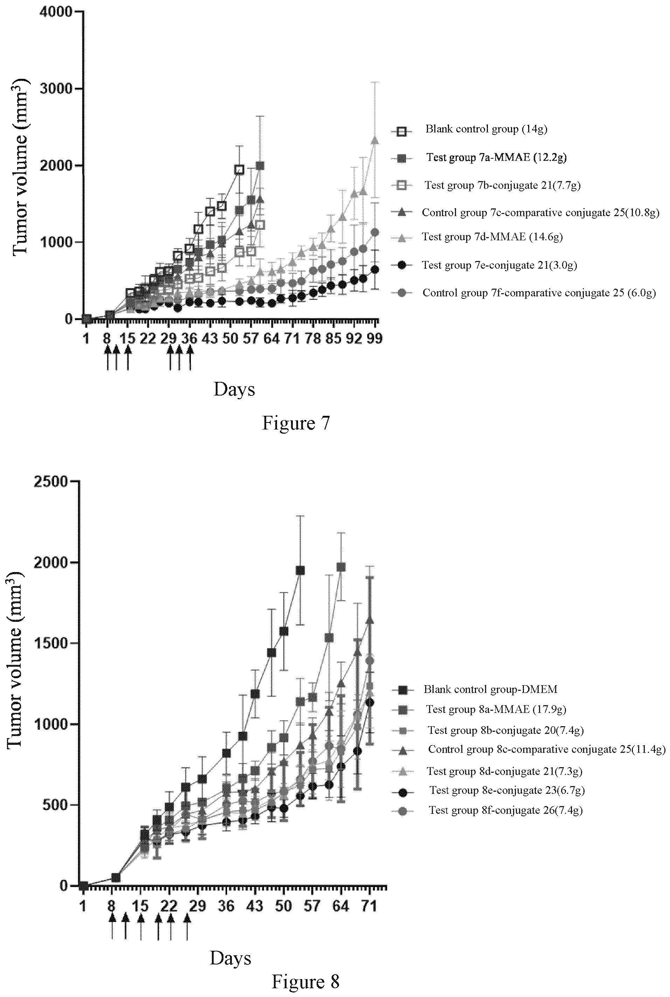

The long diameter and short diameter of the tumors were measured by *in vitro* measurement method. The tumor volume was calculated according to Formula 1/2 (long diameter × short diameter²). At the end of the experiment, tumor tissues from each group were taken, weighed and the average value was determined. The results were shown in Figure 7.

Figure 7 was a line graph showing the changes in tumor volume in U118MG subcutaneous tumor model mice over time after administration of different concentrations of the conjugates or control compounds provided by the present disclosure.

As shown in the results of Figure 7, in the blank control group, the tumor volume was increased rapidly; in the test groups administered with only MMAE, the increase rates of tumor volume were slightly decreased, indicating that MMAE *per se* showed an inhibitory effect on tumor proliferation.

Furthermore, the tumor volume of the test group 7b during the test period was significantly smaller than that of the test group 7a and the control group 7c with the equivalent content of MMAE; the tumor volume of the test group 7e during the test period was significantly smaller than that of the test group 7d and the control group 7f with the equivalent content of MMAE, showing superior anti-tumor activity than that of the test groups 7a and 7d administered with MMAE alone, and than that of the group administered with the comparative conjugate 25. After the experiment was terminated, the tumor weight in the mice administered with the conjugates of the present disclosure was also significantly lower than that in the MMAE group and the control group. The above results indicated that the conjugates provided by the present desclosure can effectively deliver MMAE to tumor tissues, showing tumor targeting ability and reducing the risk of toxicity caused by the distribution of MMAE molecules in other tissues, showing dose-dependency and excellent anti-tumor effects.

The above results indicated that the conjugates provided by the present disclosure were capable of effectively targeted delivery of small molecule drug group having inhibition effect on tumors to tumor tissue, showing good anti-tumor activity and dose-dependent effect.

### Experimental Example 8 In vivo activity of conjugates against U118MG subcutaneous tumor model mice

According to the method of experimental example 2, 42 mice inoculated with U118MG subcutaneous tumor were obtained, and the mice were kept after injection.

MMAE was dissolved into a solution with a concentration of 0.02 mg/mL with 10% DMSO + 90% serum-free DMEM medium (volume ratio); conjugate 20 and comoarative conjugate 25 were dissolved into solutions with a concentration of 0.33 mg/mL (calculated according to aptamer); conjugate 21 and conjugate 23 were dissolved into solutions with a concentration of 0.26 mg/mL (calculated according to aptamer); and conjugate 26 was dissolved into a solution with a concentration of 0.23 mg/mL (calculated according to aptamer).

7 days after inoculation of U118MG cells, the above inoculated mice were grouped, with six mice in each group, and each group of mice was administered. The day of administration was designated as D8. The mice were weighed before administration and the administration volume was calculated according to the average weight of 20g per animal.

The administration method of subcutaneous administration in the abdomen was adopted in this experiment; administered once on D8, D12, D15 and D19, four times in total.

For blank control group, each mouse was administered with DMEM medium with a single administration volume of 100 µL.

For test group 8a, each mouse was administered with MMAE with a single administration volume of 100 µL. It was calculated that a single administration dosage was 0.1 mg/kg.

For test group 8b, each mouse was administered with conjugate 20 with a single administration volume of 100 µL. It was calculated that a single administration dosage was 1.65 mg/kg.

For test group 8c, each mouse was administered with comparative conjugate 25 with a single administration volume of 100 µL. It was calculated that a single administration dosage was 1.65 mg/kg.

For test group 8d, each mouse was administered with conjugate 21 with a single administration volume of 100 µL. It was calculated that a single administration dosage was 1.32 mg/kg.

For test group 8e, each mouse was administered with conjugate 23 with a single administration volume of 100 µL. It was calculated that a single administration dosage was 1.32 mg/kg.

For test group 8f, each mouse was administered with conjugate 26 with a single administration volume of 100 µL. It was calculated that a single administration dosage was 1.17 mg/kg. The administration dosage in the above 8b-8f groups was equivalent to 0.1 mg/kg of MMAE contained.

The tumor volumes were measured on D1, D9, D16, D19, D22, D26, D30, D36, D40, D43, D47, D50, D54, D57, D61, D64, D68 and D71. Among them, the blank control group was measured on D54, and 8a group (administered with only MMAE) was measured on D64, and then the experiment was terminated.

The long diameter and short diameter of the tumors were measured by *in vitro* measurement method. The tumor volume was calculated according to Formula 1/2 (long diameter × short diameter²). At the end of the experiment, tumor tissues from each group were taken and weighed. The results were shown in Figure 8.

Figure 8 was a line graph showing the changes in tumor volume in each group of mice over time and the tumor weight on D72. As shown in the results of Figure 8, the tumor volume of the blank control group was increased rapidly, while increase rates of the tumor volume of the other groups were slightly decreased; and as compared with the test group 8a administered with only MMAE and the control group 8c, increase rates of the tumor volume of the test groups administered with the conjugates of the present disclosure at a dose equivalent to a single administration dose of 0.1mg/kg of MMAE were all significantly further decreased. Furthermore, on the experimental endpoint D72, the tumor weights of conjugate 20, conjugate 21, conjugate 23 and conjugate 26 were reduced by at least 58% as compared with that of the test group 8a, showing more excellent anti-tumor effects.

### Experimental Example 9 In vivo activity of conjugates against A549 subcutaneous tumor model mice

A549 human lung adenocarcinoma cells (purchased from GuangZhou Jennio Biotech Co., Ltd) were cultured in DMEM complete medium (MACGENE company, Cat. No. CM15019) containing 10% FBS (Gibco, Cat. No. 10099-141) at 37°C in an incubator containing 5% CO₂/95% air. The cells were digested with 0.25% pancreatic enzyme and collected. The supernatant was aspirated, and the cells were resuspended in serum-free DMEM medium to formulate into cell culture medium with a cell density of 1×10⁸ cells/mL.

According to the method of experimental example 2, 30 mice inoculated with A549 subcutaneous tumors were obtained, and the mice were kept after injection.

Conjugate 20 and conjugate 21 were dissolved into conjugate solutions with a concentration of 1 mg/mL (calculated according to aptamer) with serum-free DMEM medium. Comparative conjugate 24 were dissolved into a conjugate solution with a concentration of 0.8 mg/mL (calculated according to aptamer). MMAE was dissolved into a solution with a concentration of 0.06 mg/mL with 10% DMSO + 90% serum-free DMEM medium (volume ratio).

7 days after the inoculation of A549 human lung cancer cells, all mice were grouped, with 6 mice in each group, and each mouse was administrated. The day of administration was designated as D8. The mice were weighed before administration, and the administration volume was calculated by the mouse body weight.

Each group of mice was administrated once on D8, D12, D15 and D19, four times in total.

For blank control group 9a, each mouse was administered with DMEM with a single administration volume of 5 µL/g mouse body weight;

For control group 9b, each mouse was administered with MMAE with a single administration volume of 5 µL/g mouse body weight. It was calculated that a single administration dosage was 0.3 mg/kg;

For test group 9c, each mouse was administered with conjugate 20 with a single administration volume of 5 µL/g mouse body weight. It was calculated that a single administration dosage was 5 mg/kg, wherein the dosage of MMAE contained was equivalent to 0.3 mg/kg;

For test group 9d, each mouse was administered with conjugate 21 with a single administration volume of 5 µL/g mouse body weight. It was calculated that a single administration dosage was 5 mg/kg, wherein the dosage of MMAE contained was equivalent to 0.3 mg/kg;

For test group 9e, each mouse was administered with comparative conjugate 24 with a single administration volume of 5 µL/g mouse body weight. It was calculated that a single administration dosage was 5 mg/kg, wherein the dosage of MMAE contained was equivalent to 0.3 mg/kg;

Each group of mice was administrated once on D46, D50 and D54 with a single administration volume of 10 µL/g mouse body weight. It was calculated that the dosage of MMAE contained in a single administration dosage was equivalent to 0.6 mg/kg.

Tumor volumes were measured on D1, D9, D16, D19, D22, D26, D30, D36, D40, D43, D47, D50, D54, and D57. The blank control group was measured on D50, and then the experiment was terminated.

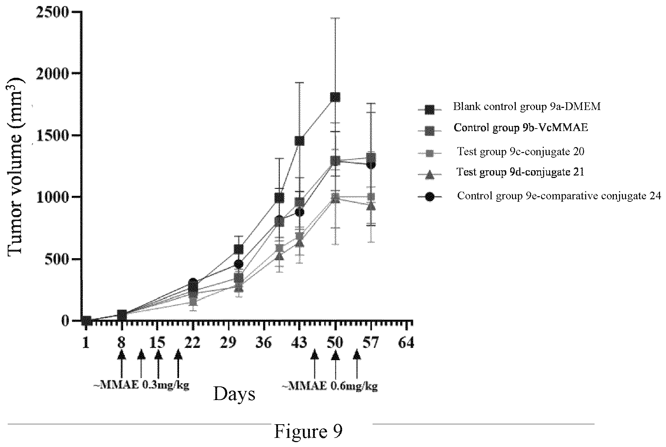

The long diameter and short diameter of the tumors were measured by *in vitro* measurement method. The tumor volume was calculated according to the Formula 1/2 (long diameter × short diameter²). The results were shown in Figure 9.

Figure 9 was a line graph showing the changes in tumor volume in A549 subcutaneous tumor model mice over time after administration of different concentrations of the conjugates or control compounds provided by the present disclosure. As shown in the results of Figure 9, the tumor volume of mice in the blank control group was increased rapidly, while the increase rates of tumor volume in the other groups were all slightly decreased; at each time period, the tumor volumes of mice administrated the conjugates 20 and 21 were smaller than those of the control groups 9b and 9e. The above results indicated that the conjugates of the present disclosure can effectively target and reach tumor tissues of A549 lung cancer and exhibit good anti-tumor activity.

The preferred embodiments of the present disclosure are described above in detail, but the present disclosure is not limited to the specific details of the above embodiments. Various simple variations of the technical solutions of the present disclosure can be made within the scope of the technical concept of the present disclosure, and these simple variations are within the scope of the present disclosure.

It is to be noted that each of the specific technical features described in the above embodiments can be combined in any suitable manner as long as no contradiction is caused. In order to avoid unnecessary repetition, various possible combinations are no longer described in the present disclosure.

In addition, various different embodiments of the present disclosure may also be arbitrarily combined as long as it does not deviate from the idea of the present disclosure, which should also be regarded as the disclosure of the present disclosure.

## Claims

1. A conjugate comprising one or more delivery groups and one or more functional groups; wherein the delivery group is formed by removing one or more hydrogen atoms or one or more functional radicals from an aptamer, the aptamer comprises a segment of continuous nucleotide sequence, the group connecting two adjacent nucleotides is independently a phosphate group or a phosphate group with a modified group, each nucleotide is selected from one of modified or unmodified A, U, C or G, and the continuous nucleotide sequence has a sequence as shown by Formula (1):
5'-T₁-S₁-Nₐ-S₂-N_{d}-S₃-N_{c}-S₄-T₂-3' Formula (1)
wherein T₁ is a motif consisting of 1-3 nucleotides, T₂ is a motif consisting of 0-15 nucleotides, and T₂ does not contain a motif that is completely reverse complementary to T₁;
S₁ and S₄ are a motif consisting of 3-7 nucleotides, respectively, and S₁ and S₄ have the same length and are completely reverse complementary;
Nₐ and N_{c} are a motif consisting of 1-4 nucleotides, respectively, each nucleotide in Nₐ is not complementary to each nucleotide in N_{c}, and the total number of U in Nₐ and N_{c} accounts for more than 50% of the total number of all nucleotides in Nₐ and N_{c};
S₂ and S₃ are a motif consisting of 1-4 nucleotides, respectively, S₂ and S₃ have the same length and are completely reverse complementary;
N_{b} is a motif consisting of 3-6 nucleotides, and the nucleotides at both ends of N_{b} do not form AU or GC complementation;
each of the delivery groups is independently connected to the functional group via a covalent bond or via a linking group; and each of the functional groups is selected from a small molecule therapeutic agent group having a therapeutic effect on tumors.

2. The conjugate according to claim 1, wherein the continuous nucleotide sequence has a length of 18-50 nucleotides, or 20-40 nucleotides, or 21-36 nucleotides, or 24-32 nucleotides.

3. The conjugate according to claim 1 or 2, wherein T₁ is consisted of 2 nucleotides; or, T₁ is consisted of 2 nucleotides and contains at least one C; or, in the 5'-3' direction, T₁ is CU, UC or AC.

4. The conjugate according to claim 1 or 2, wherein T₂ is consisted of 0-10 nucleotides; or, in the 5'-3' direction, T₂ is consisted of 1-9 nucleotides starting with U.

5. The conjugate according to claim 1, wherein S₁ and S₄ are consisted of 3-5 nucleotides, respectively, and have the same length; or, in the reverse complementation formed by S₁ and S₄, GC complementation accounts for at least 40% of the total number of all complementations; or, in the 5'-3' direction, S₁ is GCU and S₄ is AGC, or S₁ is GAGU and S₄ is GCUC, or S₁ is GGAGU and S₄ is GCUCU, or S₁ is UAUGG and S₄ is CCAUG.

6. The conjugate according to claim 1, wherein the sum of the number of nucleotides in Nₐ and N_{c} is an integer of 2-4; or, the sum of the number of nucleotides in Nₐ and N_{c} is 3 or 4, and the sum of the number of U in Nₐ and N_{c} is 2 or 3; or, in the 5'-3' direction, Nₐ or N_{c} is independently U, UU, UC or CU.

7. The conjugate according to claim 1, wherein S₂ and S₃ are consisted of 2-3 nucleotides, respectively, and have the same length; or, the reverse complementation formed by S₂ and S₃ contains at least one GC complementation; or, in the 5'-3' direction, S₂ is CA and S₃ is UG, or S₂ is AC and S₃ is GU, or S₂ is GCC and S₃ is GGU.

8. The conjugate according to claim 1, wherein N_{b} is consisted of 4 or 5 nucleotides; or, in the 5'-3' direction, N_{b} is GACG, GACGU, GACCG, UACU, GUUG or GAUCU.

9. The conjugate according to claim 1, wherein the contiguous nucleotide sequence has the sequence as shown in SEQ ID NO: 1, SEQ ID NO: 2 or SEQ ID NO: 3.

10. The conjugate according to claim 1, wherein the contiguous nucleotide sequence has the nucleotide sequence as shown in SEQ ID NO: 4:
5'-N₆GGAGUUCAN₁N₂N₃N₄UGN₅GCUCN₇-3' (SEQ ID NO: 4), wherein N₁, N₂, N₃ are each independently one of A, U, C and G; N₄ is U, C or G or a motif consisting of two of U, C or G; N₅ is U, CU or UU; N₆ is CU, UC or AC; N₇ is U, UU or UUN₈, and N₈ is a motif consisting of 1-15 nucleotides.

11. The conjugate according to claim 10, wherein the motif N₁N₂N₃N₄ consisting of N₁, N₂, N₃ and N₄ is one of GACG, GACGU, GACCG, UACU, GUUG or GAUCU.

12. The conjugate according to claim 10 or 11, wherein the contiguous nucleotide sequence has the nucleotide sequence as shown in any one of SEQ ID NOs: 5-11.

13. The conjugate according to claim 10, wherein N₈ is a motif consisting of 1-8 nucleotides; or, in the 5'-3' direction, the nucleotide sequence of N₈ is CCGAUCUC; or, the continuous nucleotide sequence has a sequence as shown in one of SEQ ID NOs: 12-14.

14. The conjugate according to claim 1, wherein each cytosine nucleotide in the continuous nucleotide sequence is a fluoro modified cytosine nucleotide, and/or each uracil nucleotide in the continuous nucleotide sequence is a fluoro modified uracil nucleotide; or, each nucleotide in the continuous nucleotide sequence is a 2'-methoxy modified nucleotide; or, one or more uracil nucleotides in the motifs of N_{b} and S₃ in the continuous nucleotide sequence have a modified base.

15. The conjugate according to claim 14, wherein the contiguous nucleotide sequence has the nucleotide sequence as shown in one of SEQ ID NOs: 15-33.

16. The conjugate according to claim 1, wherein at least one group connecting two adjacent nucleotides in the continuous nucleotide sequence is a phosphorothioate group, or each group connecting two adjacent nucleotides is a phosphorothioate group.

17. The conjugate according to claim 16, wherein the contiguous nucleotide sequence has the nucleotide sequence as shown in one of SEQ ID NOs: 34-39.

18. The conjugate according to claim 17, having a structure as shown by Formula (101): 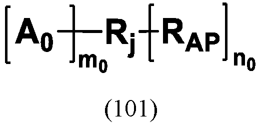
wherein each R_{AP} group is independently a group having a structure as shown by Formula (102): 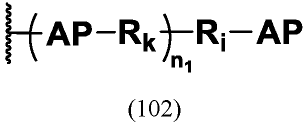
wherein each AP group is the same or different and independently represents one of the delivery groups; Rⱼ, each Rₖ or each Rᵢ is the same or different and independently represent a covalent bond or a linking group, and Rᵢ and Rₖ are not both a covalent bond at the same time; and each n₁ independently represents an integer of 0-4;
each A₀ group is the same or different and independently represents one of the functional groups; m₀ is an integer of 1-6; n₀ is an integer of 1-6, 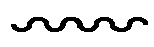 represents the site where the group is covalently linked.

19. The conjugate according to claim 18, wherein m₀ is an integer of 1-4, and/or n₀ is an integer of 1-3, and/or each n₁ is independently an integer of 0-1;
or, m₀ is 1, and/or n₀ is 1, and/or at least one or each n₁ is 0.

20. The conjugate according to claim 18 or 19, wherein each of the Rₖ or each of the Rᵢ is independently a covalent bond or a linear alkylene group of 1 to 70 carbon atoms in length, or one or more carbon atoms in the linear alkylene group are replaced with one or more groups selected from the group consisting of: C(O), NH, O, S, CH=N, S(O)₂, OP(O)₂, OP(O)(S), C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl).

21. The conjugate according to claim 20, wherein each n₁ is 0, and each Rⱼ is independently a covalent bond, or any one linking group or a connection combination of more linking groups selected from the group consisting of: C₁-C₂₀ alkylene, phosphate bond, phosphorothioate bond, amide bond, ester bond, ether bond, thioether bond, disulfide bond, 1,2,3-triazolylidene group, a subunit of polyethylene glycol, pyrrolidinylidene group, 2-oxopyrrolidinylidene group, phenylene, cyclohexylene, 2-succinimidoylidene group, 2-thiosuccinimidoylidene group, a subunit of amino acid, a subunit of nucleotide.

22. The conjugate according to claim 21, wherein each Rᵢ is independently a covalent bond, or any one linking group or a connection combination of two linking groups selected from the group consisting of: a disulfide bond, propylene phosphate group, 2-thiosuccinimidoylidene group, a subunit of amino acid, or a subunit of GAU trinucleotide.

23. The conjugate according to any one of claims 18 to 22, wherein Rⱼ is a covalent bond and m₀ is 1.

24. The conjugate according to any one of claims 18 to 22, wherein Rⱼ is a linking group, the linking group Rⱼ comprises a main chain moiety, a side chain moiety and a conjugated linking moiety, the main chain moiety is respectively connected to the conjugated linking moiety and the side chain moiety, each of the side chain moieties is respectively connected to the main chain moiety and the R_{AP} group, each of the conjugated linking moieties is respectively connected to the main chain moiety and the functional group A₀,
wherein the main chain moiety is a linear alkylene group of 1 to 70 carbon atoms in length, or one or more carbon atoms in the linear alkylene group are replaced with one or more groups selected from the group consisting of: C(O), NH, O, S, CH=N, S(O)₂, OP(O)₂, C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
wherein the side chain moiety is independently a covalent bond or a linear alkylene group of 1 to 70 carbon atoms in length, or one or more carbon atoms in the linear alkylene group are replaced with one or more groups selected from the group consisting of: C(O), NH, O, S, CH=N, S(O)₂, OP(O)₂, C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and wherein the linear alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O)(phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
each of the conjugated linking moieties is independently a covalent bond, or any one connection structure or a connection combination of more connection structures selected from the group consisting of: C₁-C₁₀ linear alkylene, phosphate bond, phosphorothioate bond, amide bond, ester bond, ether bond, disulfide bond, 1,2,3-triazolylidene group, a subunit of polyethylene glycol, pyrrolidinylidene group, 2-oxopyrrolidinylidene group, phenylene, cyclohexylene, 2-succinimidoylidene group, 2-thiosuccinimidoylidene group, a subunit of amino acid, a subunit of nucleotide.

25. The conjugate according to claim 24, wherein each of the conjugated linking moieties in the linking group Rⱼ is respectively connected to the main chain moiety and one of the functional groups A₀; the number of the side chain moieties is n₀, and each of the side chain moieties is respectively connected to the main chain moiety and one of the R_{AP} groups.

26. The conjugate according to claim 24 or 25, wherein all the side chain moieties are connected to a same atom on the main chain moiety; or, each of the side chain moieties is connected to different atoms on the main chain moiety.

27. The conjugate according to claim 26, wherein m₀ is 1, and the linking group Rⱼ comprises a structure as shown by Formula (301): 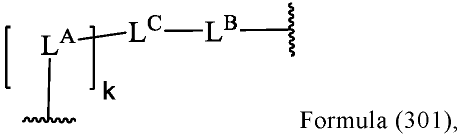
wherein k is an integer of 1-3; L^{C} is the main chain moiety, L^{A} is the side chain moiety, L^{B} is the conjugated linking moiety, and 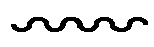 represents the site where the group is covalently linked;
the main chain moiety L^{C} is a covalent bond or a 2- to 4-valent, linear or branched C₁-C₂₅ saturated hydrocarbyl group, or one or more carbon atoms in the saturated hydrocarbyl group are replaced with one or more groups selected from the group consisting of: C(O), NH, O, S, CH=N, S(O)₂, OP(O)₂, C₅-C₈ glycosylene, C₂-C₅ alkenylene, C₂-C₅ alkynylene, C₆-C₁₀ arylene, C₃-C₈ heterocyclylene, and C₅-C₁₀ heteroarylene; wherein the saturated hydrocarbyl group may have any one or more substituents selected from the group consisting of: C₁-C₅ alkyl, C₆-C₁₀ aryl, C₅-C₁₀ heteroaryl, -OC₁-C₅ alkyl, -OC₁-C₅ alkylphenyl, -C₁-C₅ alkyl-OH, -SC₁-C₅ alkyl, nitro, -C(O)O(C₁-C₅ alkyl), -CON(C₁-C₅ alkyl)(C₁-C₅ alkyl), -CONH(C₁-C₅ alkyl), -CONH₂, -NHC(O)(C₁-C₅ alkyl), -NHC(O)(phenyl), -N(C₁-C₅ alkyl)C(O)(C₁-C₅ alkyl), -N(C₁-C₅ alkyl)C(O) (phenyl), -C(O)C₁-C₅ alkyl, -C(O)C₁-C₅ alkylphenyl, -OC(O)C₁-C₅ alkyl, -SO₂(C₁-C₅ alkyl), -SO₂(phenyl), -SO₂NH₂, -SO₂NH(C₁-C₅ alkyl), -SO₂NH(phenyl), -NHSO₂(C₁-C₅ alkyl) and -NHSO₂(phenyl);
each of the side chain moieties is independently a covalent bond, or a linear alkylene group of 1 to 70 carbon atoms in length, or one or more carbon atoms in the linear chain alkylene group are replaced with one or more groups selected from the group consisting of: C(O), NH, O, S, CH=N, S(O)₂, OP(O)₂, C₅-C₈ glycosylene, C₂-C₁₀ alkenylene, C₂-C₁₀ alkynylene, C₆-C₁₀ arylene, C₃-C₁₈ heterocyclylene, and C₅-C₁₀ heteroarylene; and the linear alkylene group may have any one or more substituents selected from the group consisting of: C₁-C₁₀ alkyl, C₆-C₁₀ aryl, C₅-C₁₀ heteroaryl, C₁-C₁₀ haloalkyl, -OC₁-C₁₀ alkyl, -OC₁-C₁₀ alkylphenyl, -C₁-C₁₀ alkyl-OH, -OC₁-C₁₀ haloalkyl, -SC₁-C₁₀ alkyl, -SC₁-C₁₀ alkylphenyl, -C₁-C₁₀ alkyl-SH, -SC₁-C₁₀ haloalkyl, halogen substituent, -OH, -SH, -NH₂, -C₁-C₁₀ alkyl-NH₂, -N(C₁-C₁₀ alkyl)(C₁-C₁₀ alkyl ), -NH(C₁-C₁₀ alkyl), -N(C₁-C₁₀ alkyl)(C₁-C₁₀ alkylphenyl), -NH(C₁-C₁₀ alkylphenyl), cyano, nitro, -CO₂H, -C(O)O(C₁-C₁₀ alkyl), -CON(C₁-C₁₀ alkyl)(C₁-C₁₀ alkyl), -CONH(C₁-C₁₀ alkyl), -CONH₂, -NHC(O)(C₁-C₁₀ alkyl), -NHC(O)(phenyl), -N(C₁-C₁₀ alkyl)C(O)(C₁-C₁₀ alkyl), -N(C₁-C₁₀ alkyl)C(O) (phenyl), -C(O)C₁-C₁₀ alkyl, -C(O)C₁-C₁₀ alkylphenyl, -C(O)C₁-C₁₀ haloalkyl, -OC(O)C₁-C₁₀ alkyl, -SO₂(C₁-C₁₀ alkyl), -SO₂(phenyl), -SO₂(C₁-C₁₀ haloalkyl), -SO₂NH₂, -SO₂NH(C₁-C₁₀ alkyl), -SO₂NH(phenyl), -NHSO₂(C₁-C₁₀ alkyl), -NHSO₂(phenyl) and -NHSO₂(C₁-C₁₀ haloalkyl);
each of the conjugated linking moieties is independently a covalent bond, or any one connection structure or a connection combination of more connection structures selected from the group consisting of: C₁-C₁₀ linear alkylene, phosphate bond, phosphorothioate bond, amide bond, ester bond, ether bond, disulfide bond, 1,2,3-triazolylidene group, a subunit of polyethylene glycol, pyrrolidinylidene group, 2-oxopyrrolidinylidene group, phenylene, cyclohexylene, 2-succinimidoylidene group, 2-thiosuccinimidoylidene group, a subunit of amino acid, a subunit of nucleotide.

28. The conjugate according to claim 27, wherein the conjugate has a structure as shown by Formula (305):

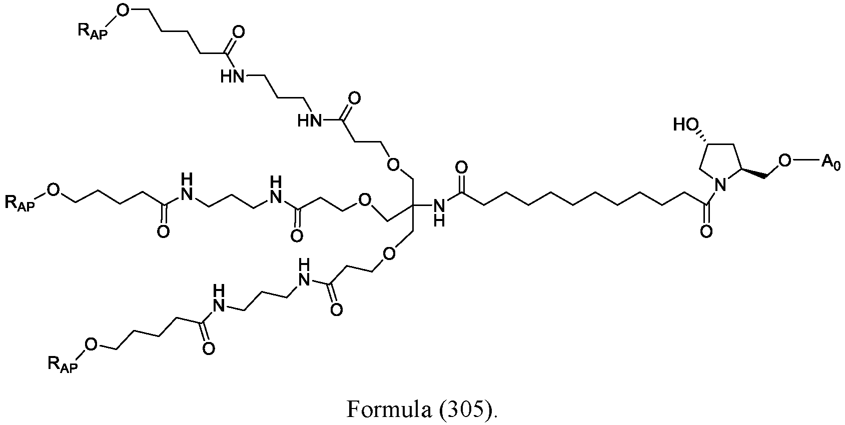

29. The conjugate according to claim 27, wherein the linking group Rⱼ has a structure as shown by Formula (306): 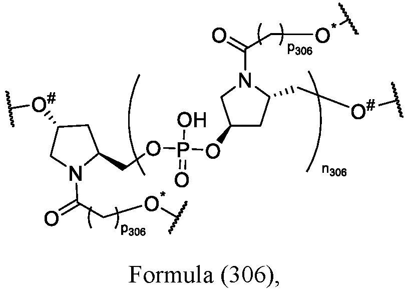 wherein, n₃₀₆ is an integer of 0-3, each p306 is independently an integer of 1-6, 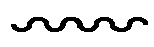 represents the site where the group is covalently linked; the oxygen atom marked by * forms a phosphate bond, an ether bond or an ester bond with the R_{AP} group; at least one of the oxygen atoms marked by # forms an ether bond, an ester bond or a phosphate bond with the functional group A₀, and the remaining oxygen atoms marked by # are connected to hydrogen atoms to form hydroxyl groups, or are connected to C₁-C₃ alkyl groups to form C₁-C₃ alkoxy groups.

30. The conjugate according to claim 29, wherein the conjugate has a structure as shown by Formula (307a), (307b) or (307c):

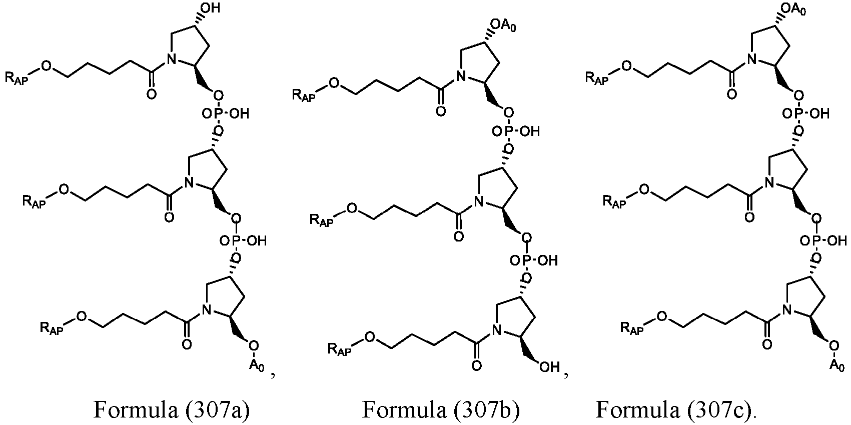

31. The conjugate according to claim 27, wherein the conjugate has a structure as shown by Formula (308): 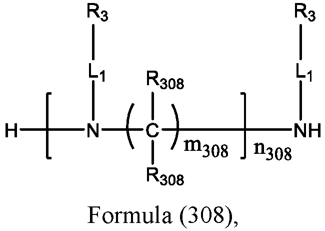 wherein,
n₃₀₈ is an integer selected from 1-10;
each m₃₀₈ is independently an integer selected from 2-10;
each R₃₀₈ is independently H, C₁-C₁₀ alkyl, C₁-C₁₀ haloalkyl or C₁-C₁₀ alkoxy;
each R₃ is independently the functional group A₀, or is the R_{AP} group, and at least one of R₃ is the functional group A₀, and at least one of R₃ is the R_{AP} group; or, one of R₃ is the functional group A₀, and the remaining R₃ are the R_{AP} groups;
each L₁ connected to the functional group A₀ represents the conjugated linking moiety, and each L₁ connected to the R_{AP} represents the side chain moiety.

32. The conjugate according to claim 31, wherein each L₁ is independently selected from the group consisting of groups L4-L23 and any connection combination thereof.

33. The conjugate according to claim 32, wherein each L₁ is independently selected from the group consisting of a connection combination of at least two of the groups L4-L9, L13, L14, and L18; or, each L₁ is independently a connection combination of at least two of the groups L4, L5, L7, L9, L13, L14, and L18.

34. The conjugate according to any one of claims 31 to 33, wherein the length of each L₁ is independently 3-25 atoms; or, the length of each L₁ is independently 4-15 atoms.

35. The conjugate according to any one of claims 31 to 34, wherein n₃₀₈ is an integer of 2-6, 2-4 of R₃ are the R_{AP} groups, and the remaining R₃ are the functional groups.

36. The conjugate according to any one of claims 31 to 35, wherein each m₃₀₈ is independently an integer of 2-5, and/or each m₃₀₈ is equal.

37. The conjugate according to any one of claims 31 to 36, wherein n₃₀₈ is an integer selected from 2-4; each m₃₀₈ is independently an integer selected from 2-4; and each R₃₀₈ is H.

38. The conjugate according to claim 37, wherein one of R₃ is the functional group A₀ and the remaining R₃ are the R_{AP} groups.

39. The conjugate according to claim 37 or 38, wherein each L₁ contains both an attachment site attached to the N atom of the nitrogen-containing skeleton and an attachment site attached to the functional group A₀ or the R_{AP} group, and the site attached to the N atom of the nitrogen-containing skeleton forms an amide bond with the N atom; or, one or more L₁ is selected from B5, B6, B5' or B6': 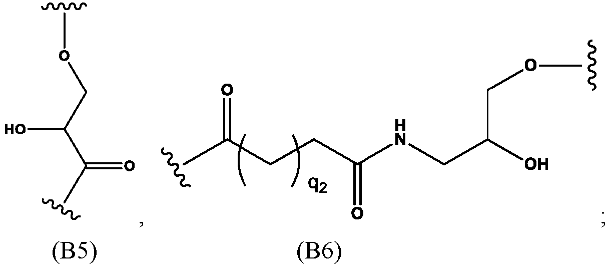 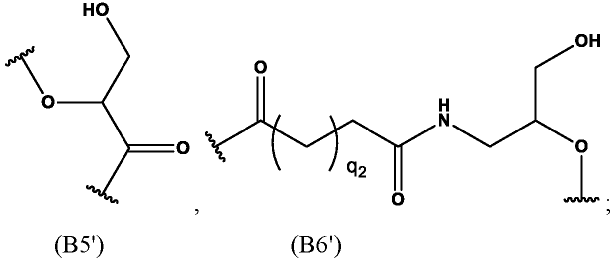 wherein, 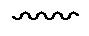 represents the site where the group is covalently linked, q₂ is an integer of 1-10; or, q₂ is an integer of 1-5.

40. The conjugate according to any one of claims 31-39, having a structure as shown by Formula (403), (404), (405), (406), (407), (408), (409), (410), (411), (412), (413), (414), (415), (416), (417), (418), (419), (420), (421), (422), (423), (424), (425), (426) or (427):

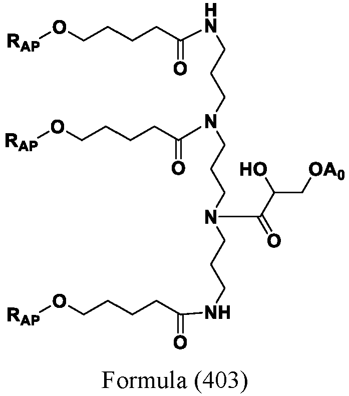

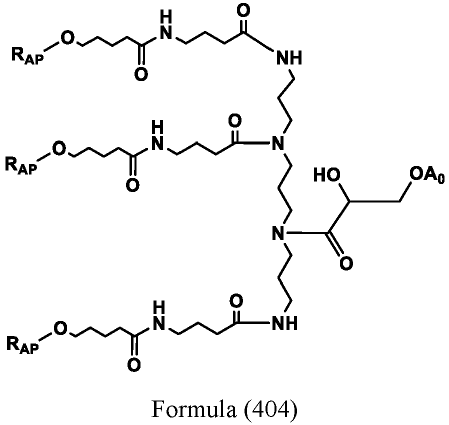

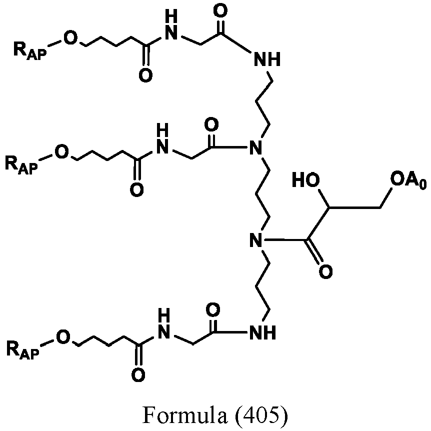

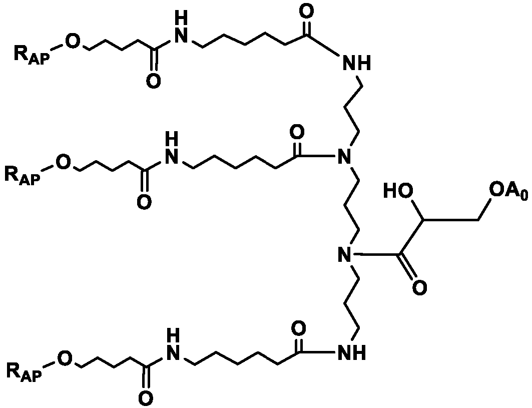

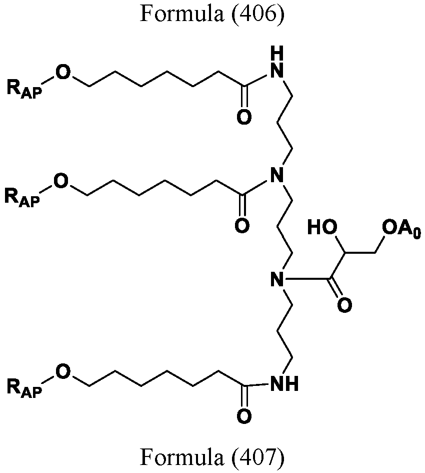

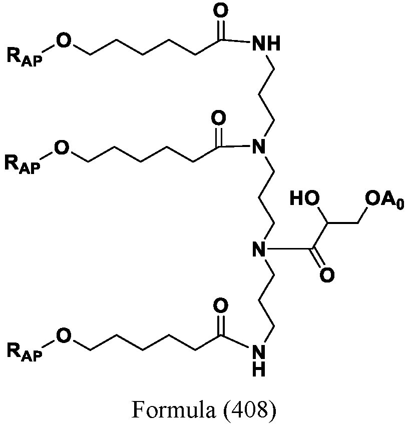

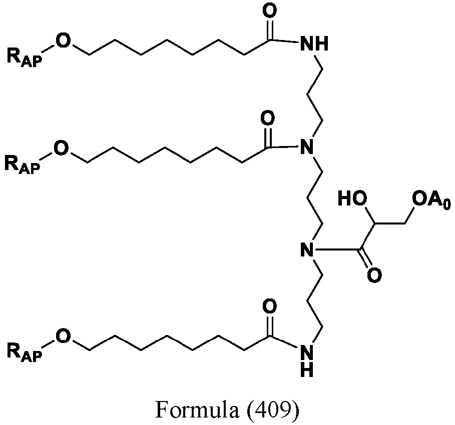

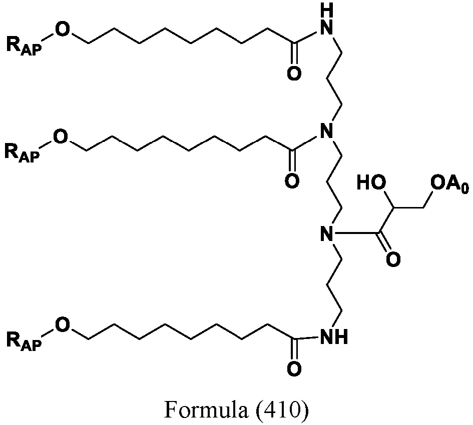

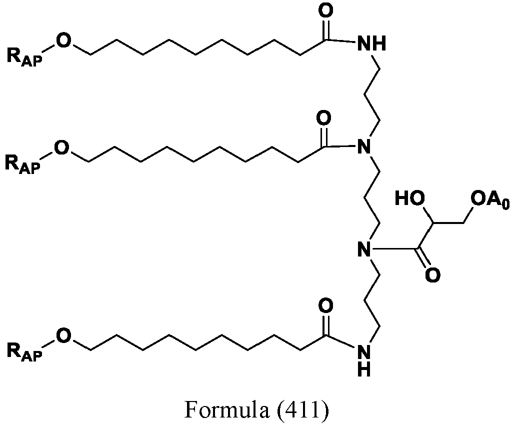

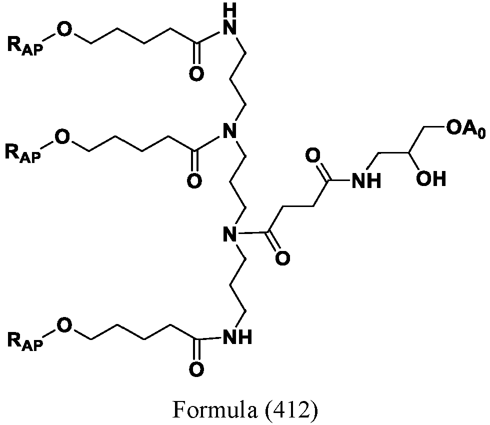

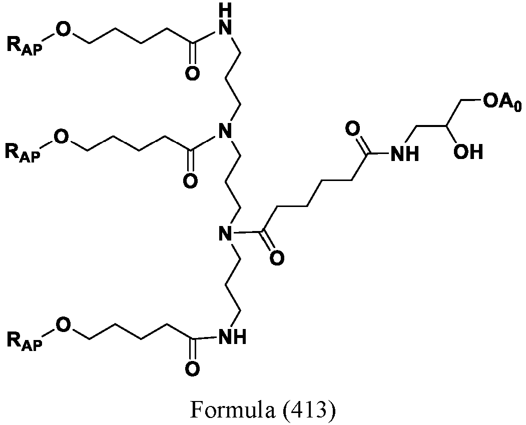

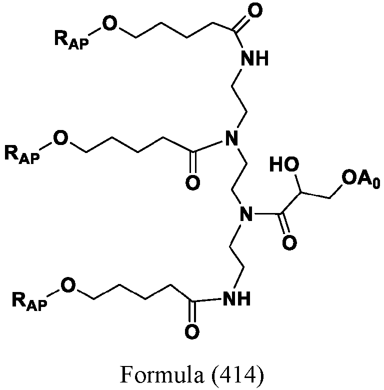

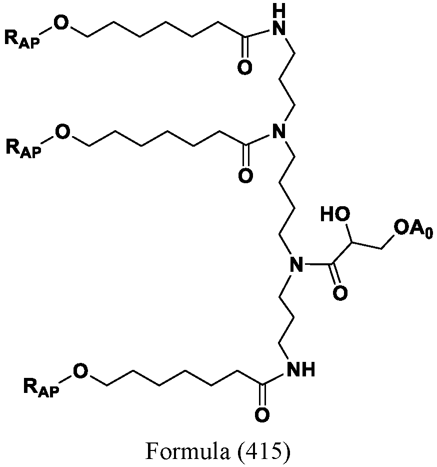

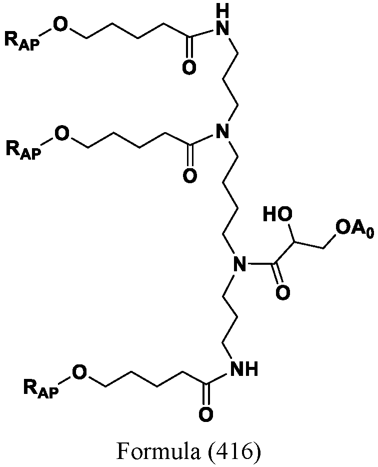

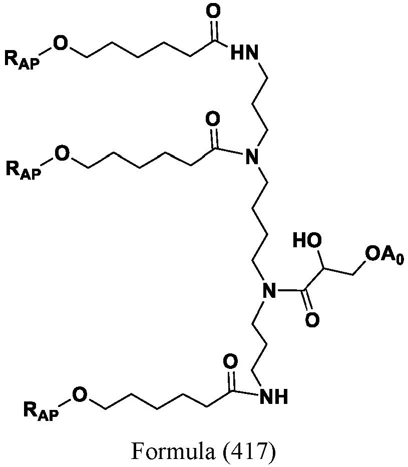

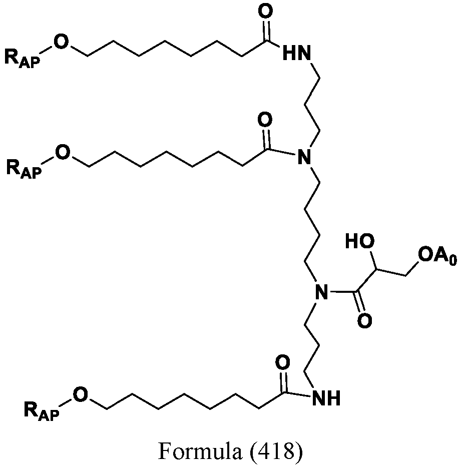

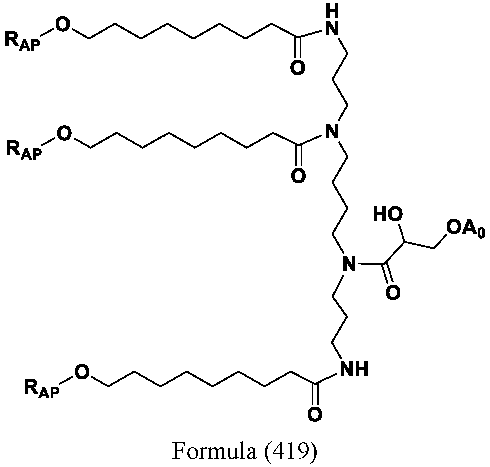

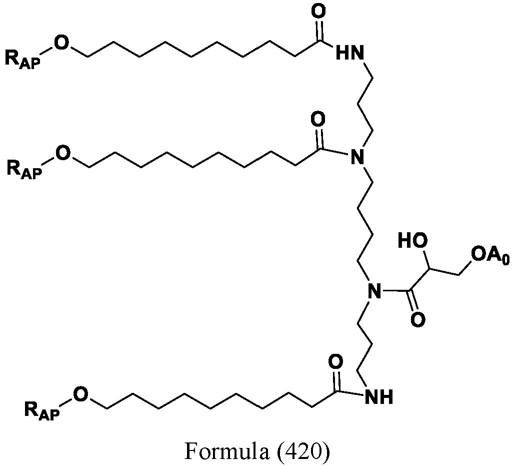

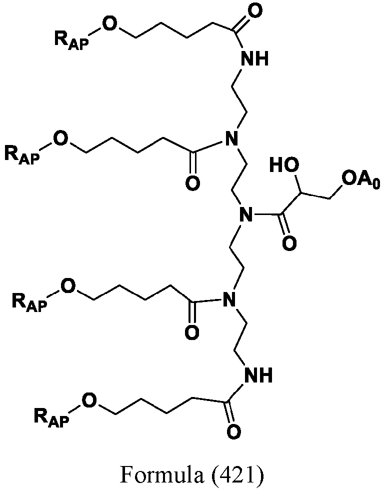

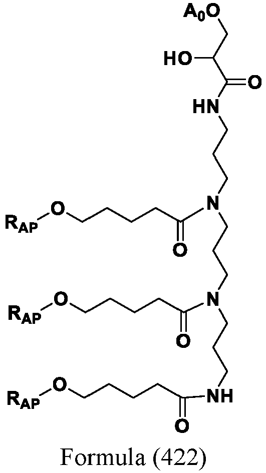

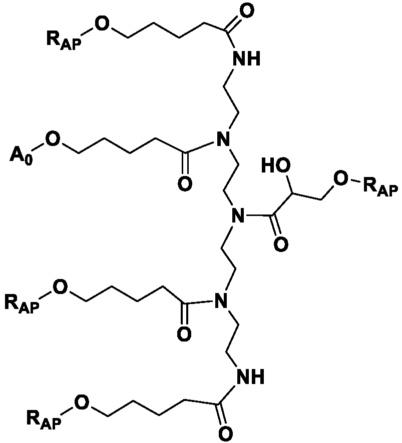

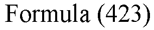

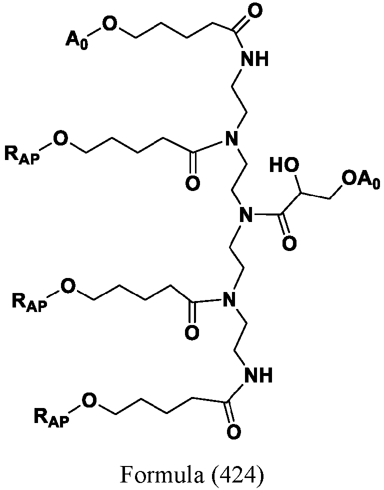

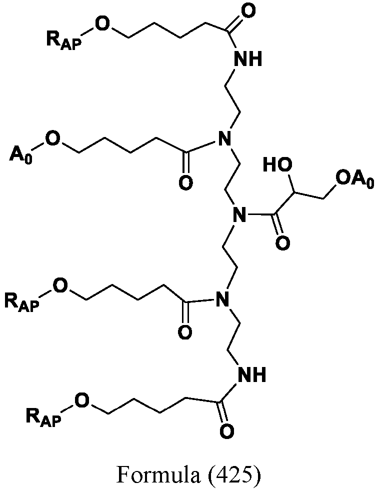

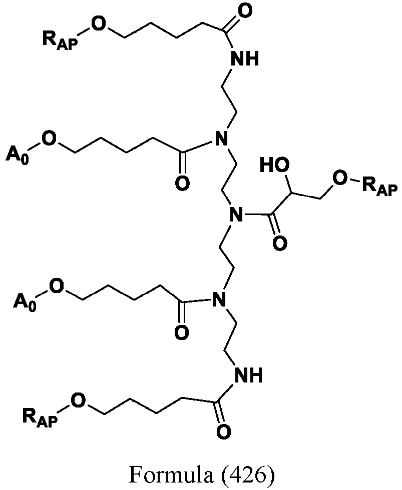

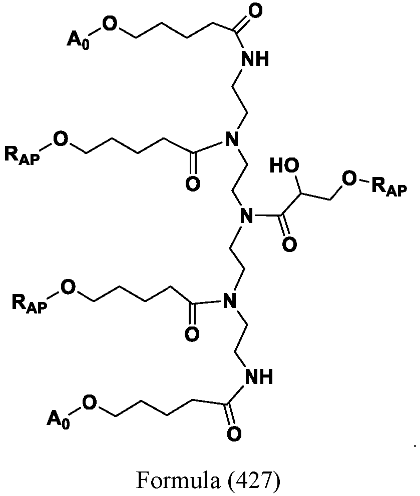

41. The conjugate according to any one of claims 18 to 22, wherein Rⱼ comprises a nucleotide sequence I and a nucleotide sequence II, each of the nucleotide sequence I and the nucleotide sequence II comprises 5 to 25 modified or unmodified nucleotides, the nucleotide sequence I and the nucleotide sequence II are at least partially reverse complementary, the delivery group is connected to the nucleotide sequence I, the functional group is connected to the nucleotide sequence II, and the nucleotide sequence I and the nucleotide sequence II do not elicit an immune response or a toxic response in a subject.

42. The conjugate according to claim 41, wherein the 3' terminal of the delivery group is connected to the 5' position of the ribose of the 5' terminal nucleotide of the nucleotide sequence I via a phosphate bond, and the functional group is connected to the 5' position of the ribose of the 5' terminal nucleotide of the nucleotide sequence II; or, the functional group comprises a segment of nucleotide sequence, and the 3' terminal of the nucleotide sequence is connected to the 5' position of the ribose of the 5' terminal nucleotide of the nucleotide sequence II via a phosphate bond.

43. The conjugate according to claim 42, wherein the nucleotide sequence I and the nucleotide sequence II are substantially reverse complementary or completely reverse complementary; or, the nucleotide sequence I and the nucleotide sequence II are equal in length and both are 10-20 modified or unmodified nucleotides; or, the nucleotide sequence I and the nucleotide sequence II are both consisted of 17 nucleotides and are completely reverse complementary; or, the nucleotide sequence I and the nucleotide sequence II have the sequences as shown in SEQ ID NO: 40 and SEQ ID NO: 41, respectively:
5'-GUACAUUCUAGAUAGCC-3' (SEQ ID NO: 40)
5'-GGCUAUCUAGAAUGUAC-3' (SEQ ID NO: 41),
or, the nucleotide sequence I and the nucleotide sequence II have the sequences as shown in SEQ ID NO: 42 and SEQ ID NO: 43, respectively:
5'- GmUfAmCfAmUfUfCfUfAmGmAmUfAmGmCfCf -3' (SEQ ID NO: 42)
5'- GmGmCfUfAmUfCfUfAmGmAmAmUfGmUfAmCf -3' (SEQ ID NO: 43).

44. The conjugate according to any one of claims 18 to 43, wherein Rⱼ is cleavable.

45. The conjugate according to any one of claims 1 to 44, wherein each of the small molecule therapeutic agent groups is independently selected from a cytotoxin group, an antibiotic group or an angiogenesis inhibitor.

46. The conjugate according to claim 45, wherein the small molecule therapeutic agent group is formed by removing one or more hydrogen atoms or one or more functional radicals from one of the following small molecule therapeutic agents: methotrexate, doxorubicin, vinca alkaloids, auristatin, calicimycin, maytansine, camptothecin, and calicheamicin.

47. The conjugate according to claim 46, wherein the small molecule therapeutic agent group is a group formed by removing one or more hydrogen atoms or one or more functional radicals from monomethyl auristatin E (MMAE).

48. A pharmaceutical composition comprising the conjugate according to any one of claims 1 to 47 and a pharmaceutically acceptable carrier.

49. Use of the conjugate according to any one of claims 1 to 47 and/or the pharmaceutical composition according to claim 48 in the manufacture of a medicament for treating tumors and tumor-related diseases or symptoms.

50. Use according to claim 49, wherein the tumor is one or more of glioma, renal cancer, and lung cancer.

51. A method for treating tumors and tumor-related diseases or symptoms, comprising administering an effective amount of the conjugate according to any one of claims 1 to 47 and/or the pharmaceutical composition according to claim 48 to a subject in need thereof.

52. The method according to claim 51, wherein the tumor is one or more of glioma, renal cancer, and lung cancer.

53. A kit comprising the conjugate according to any one of claims 1 to 47 and/or the pharmaceutical composition according to claim 48.
